# EUROPEAN PATENT SPECIFICATION

(11) **EP 1 332 132 B1**
(45) Date of publication and mention of the grant of the patent: **10.10.2007**
(21) Application number: 01921580.5
(22) Date of filing: 12.04.2001
(51) Int. Cl.: C07D 213/81, C07D 217/22, C07C 225/20, C07D 471/04, A61K 31/44, A61P 19/08, A61P 19/10, A61P 29/00

(54) **ENAMINE DERIVATIVES AS CELL ADHESION MOLECULES**
ENAMIN-DERIVATE ALS ZELL-ADHÄSIONSMOLEKÜLE
DERIVES D'ENAMINE COMME MOLECULES D'ADHESION CELLULAIRE

(30) Priority: 17.04.2000 GB 0009493; 26.07.2000 GB 0018353; 02.08.2000 GB 0018966
(43) Date of publication of application: 06.08.2003
(73) Proprietor: UCB Pharma, S.A., 1070 Brussels (BE)
(72) Inventor: NORMAN, Timothy, John, c/o Celltech R & D Limited, Slough, Berkshire SL1 3WE (GB); PORTER, John, Robert, c/o Celltech R & D Limited, Slough, Berkshire SL1 3WE (GB); HUTCHINSON, Brian,Woodside, c/o Celltech R & D Limited, Slough, Berkshire SL1 3WE (GB); RATCLIFFE, Andrew,James, c/o Celltech R & D Limited, Slough, Berkshire SL1 3WE (GB); HEAD, John, Clifford, c/o Celltech R & D Limited, Slough, Berkshire SL1 3WE (GB); ALEXANDER, Rikki,Peter, c/o Celltech R & D Limited, Slough, Berkshire SL1 3WE (GB); LANGHAM, Barry, John, c/o Celltech R & D Limited, Slough, Berkshire SL1 3WE (GB); WARRELLOW, Graham, John, Northwood, Middlesex HA6 3QU (GB); ARCHIBALD, Sarah,Catherine, c/o Celltech R & D Limited, Slough, Berkshire SL1 3WE (GB); LINSLEY, Janeen, Marsha, High Wycombe, Buckinghamshire HP13 5PE (GB)
(74) Representative: Thompson, John
(86) International application number: PCT/GB2001/001682
(87) International publication number: WO 2001/079173

(56) References cited:
- WO-A-00/18759
- WO-A-02/20522
- WO-A-97/36859
- WO-A-99/10313
- DATABASE CAOLD [Online] CHEMICAL ABSTRACTS SERVICE, COLUMBUS, OHIO, US; JT PENDERS: "Mass Spectral Identification of the methyl esters of 2,4-dinitrophenylamino acids" retrieved from STN Database accession no. CA65:1554b XP002206382
- DATABASE CAOLD [Online] CHEMICAL ABSTRACTS SERVICE, COLUMBUS, OHIO, US; S AONUMA: "Effect of Thyroid-stimulating hormone, thyroxine, diiodotyrosine, 2,4-dinitrophenol, and related compounds on thiamine metabolism" retrieved from STN Database accession no. CA57:15725b XP002206383
- DATABASE CAOLD [Online] CHEMICAL ABSTRACTS SERVICE, COLUMBUS, OHIO, US; T OKUYAMA: "Preparation and Properties of 2,4,6-trinitrophenylamino acids and peptides" retrieved from STN Database accession no. CA54:24470i XP002206384
- CRABBE, PIERRE ET AL: "Cotton effect of dimedone and dihydroresorcinol condensation compounds of amino acids and peptides" TETRAHEDRON (1968), 24(11), 4315-26 , XP002206381
- HENKE B R ET AL: "N-(2-BENZOYLPHENYL)-L-TYROSINE PPARGAMMA AGONISTS. 1. DISCOVERY OF A NOVEL SERIES OF POTENT ANTIHYPERGLYCEMIC AND ANTIHYPERLIPIDEMIC AGENTS" JOURNAL OF MEDICINAL CHEMISTRY, AMERICAN CHEMICAL SOCIETY. WASHINGTON, US, vol. 41, no. 25, 1998, pages 5020-5036, XP000864731 ISSN: 0022-2623
- BROWN K K ET AL: "A NOVEL N-ARYL TYROSINE ACTIVATOR OF PEROXISOME PROLIFERATOR-ACTIVATED RECEPTOR-GAMMA REVERSES THE DIABETIC PHENOTYPE OF THE ZUCKER DIABETIC FATTY RAT" DIABETES, NEW YORK, NY, US, vol. 48, July 1999 (1999-07), pages 1415-1424, XP000985787 ISSN: 0012-1797

## Description

This invention relates to a series of enamine derivatives, to compositions containing them, to processes for their preparation, and to their use in medicine.

Over the last few years it has become increasingly clear that the physical interaction of inflammatory leukocytes with each other and other cells of the body plays an important role in regulating immune and inflammatory responses [Springer, T. A., Nature, 346, 425, (1990); Springer, T. A, Cell, 76, 301, (1994)]. Specific cell surface molecules collectively referred to as cell adhesion molecules mediate many of these interactions.

The adhesion molecules have been sub-divided into different groups on the basis of their structure. One family of adhesion molecules which is believed to play a particularly important role in regulating immune and inflammatory responses is the integrin family. This family of cell surface glycoproteins has a typical non-covalently linked heterodimer structure. At least 16 different integrin alpha chains and 8 different integrin beta chains have been identified [Newman, P. *et al,* Molecular Medicine Today, 304, (1996)]. The members of the family are typically named according to their heterodimer composition although trivial nomenclature is widespread in the field. Thus the integrin α4β1 consists of the integrin alpha 4 chain associated with the integrin beta 1 chain, but is also widely referred to as Very Late Antigen 4 or VLA-4. Not all of the potential pairings of integrin alpha and beta chains have yet been observed in nature and the integrin family has been subdivided into a number of subgroups based on the pairings that have been recognised to date [Sonnenberg, A., Current Topics in Microbiology and Immunology, 184, 7, (1993)].

The importance of integrin function in normal physiological responses is highlighted by two human deficiency diseases in which integrin function is defective. Thus in the disease termed Leukocyte Adhesion Deficiency (LAD) there is a defect in one of the families of integrins expressed on leukocytes [Marlin, S. D. et al, J. Exp. Med. 164, 855, (1986)]. Patients suffering from this disease have a reduced ability to recruit leukocytes to inflammatory sites and suffer recurrent infections, which in extreme cases may be fatal. In the case of patients suffering from the disease termed Glanzman's thrombasthenia (a defect in a member of the beta 3 integrin family) there is a defect in blood clotting (Hodivala-Dilke, K. M., J. Clin. Invest. 103, 229, (1999)].

The potential to modify integrin function in such a way as to beneficially modulate cell adhesion has been extensively investigated in animal models using specific antibodies and peptides that block various functions of these molecules [e.g. Issekutz, T. B., J. Immunol. 149, 3394, (1992); Li, Z. et al, Am. J. Physiol. 263. L723, (1992); Mitjans, F. et al, J. Cell Sci. 108, 2825, (1995); Brooks, P. C. et al, J. Clin. Invest. 96, 1815, (1995); Binns, R. M. et al, J. Immunol. 157. 4094, (1996); Hammes, H.-P. et al, Nature Medicine 2, 529, (1996); Srivata, S. et al , Cardiovascular Res. 36, 408 (1997)]. A number of monoclonal antibodies which block integrin function are currently being investigated for their therapeutic potential in human disease, and one, ReoPro, a chimeric antibody against the platelet integrin αIIbβ3 is in use as a potent anti-thrombotic agent for use in patients with cardiovascular complications following coronary angioplasty.

Integrins recognize both cell surface and extracellular matrix ligands, and ligand specificity is determined by the particular alpha-beta subunit combination of the molecule [Newman, P., *ibid*]*.* One particular integrin subgroup of interest involves the α4 chain which can pair with two different beta chains β1 and β7 [Sonnenberg, A, *ibid*]. The α4β1 pairing occurs on many circulating leukocytes (for example lymphocytes, monocytes, eosinophils and basophils) although it is absent or only present at low levels on circulating neutrophils. α4β1 binds to an adhesion molecule (Vascular Cell Adhesion Molecule-1 also known as VCAM-1) frequently up-regulated on endothelial cells at sites of inflammation [Osborne, L., Cell, 62, 3, (1990)]. The molecule has also been shown to bind to at least three sites in the matrix molecule fibronectin [Humphries, M. J. et al, Ciba Foundation Symposium, 189, 177, (1995)]. Based on data obtained with monoclonal antibodies in animal models it is believed that the interaction between α4β1 and ligands on other cells and the extracellular matrix plays an important role in leukocyte migration and activation [Yednock, T. A et al, Nature, 356, 63, (1992); Podolsky, D. K. et al, J. Clin. Invest. 92, 372, (1993); Abraham, W. M. et al, J. Clin. Invest. 93, 776, (1994)].

The integrin generated by the pairing of α4 and β7 has been termed LPAM-1 [Holzmann, B. and Weissman, I. L., EMBO J. 8, 1735, (1989)]. The α4β7 pairing is expressed on certain sub-populations of T and B lymphocytes and on eosinophils [Erie, D. J. et al, J. Immunol. 153, 517 (1994)]. Like α4β1, α4β7 binds to VCAM-1 and fibronectin. In addition, α4β7 binds to an adhesion molecule believed to be involved in the homing of leukocytes to mucosal tissue termed MAdCAM-1 [Berlin, C. et al, Cell, 74, 185, (1993)]. The interaction between α4β7 and MAdCAM-1 may also be important sites of inflammation outside of mucosal tissue [Yang, X.-D. et al, PNAS, 91, 12604, (1994)].

Regions of the peptide sequence recognizeded by α4β1 and α4β7 when they bind to their ligands have been identified. α4β1seems to recognise LDV, IDA or REDV peptide sequences in fibronectin and a QIDSP sequence in VCAM-1 [Humphries, M. J. *et al, ibid*] whilst α4β7 recognises a LDT sequence in MAdCAM-1 [Birskin, M. J. et al, J. Immunol. 156, 719, (1996)]. There have been several reports of inhibitors of these interactions being designed from modifications of these short peptide sequences [Cardarelli, P. M. et al, J. Biol. Chem., 269, 18668, (1994); Shorff, H. N. et al, Biorganic Med. Chem. Lett., 6, 2495, (1996); Vanderslice, P. et al, J. Immunol., 158, 1710, (1997)]. It has also been reported that a short peptide sequence derived from the α4β1 binding site in fibronectin can inhibit a contact hypersensitivity reaction in a trinitrochlorobenzene sensitised mouse [Ferguson; T. A, et al, PNAS, 88, 8072, (1991)].

Since the alpha 4 subgroup of integrins are predominantly expressed on leukocytes their inhibition can be expected to be beneficial in a number of immune or inflammatory disease states. However, because of the ubiquitous distribution and wide range of functions performed by other members of the integrin family it is important to be able to identify selective inhibitors of the alpha 4 subgroup.

WO00/18759 discloses phenylalanine derivatives which are useful as a4 integrin inhibitors.

Crabbe, P. et al., Tetrahedron (1968), 24(11), 4315-26 discloses dimedonyl-(S)-tyrosine-O-benzyl methyl ester and forms the basis of the disclaimer in claim 1.

We have now found a group of compounds which are potent and selective inhibitors of α4 integrins. Members of the group are able to inhibit α4 integrins such as α4β1 and/or α4β7 at concentrations at which they generally have no or minimal inhibitory action on α integrins of other subgroups. These compounds possess the additional advantage of good pharmacokinetic properties, especially low plasma clearance.

Thus according to one aspect of the invention we provide a compound of formula (1) 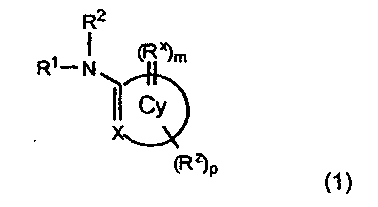 wherein
R¹ is a group Ar¹L²Ar²Alk- in which Ar¹ is an optionally substituted aromatic or heteroaromatic group, L² is a covalent bond or a linker atom or group L^{2a} or a linker -(Alk^{a})L^{2a}-, where Alk^{a} is an aliphatic or heteroaliphatic chain, and L^{2a} is a linker atom or group selected from -O- or -S- atoms or -C(O)-, -C(O)O-, -OC(O)-, -C(S)-, -S(O)-, -S(O)₂-, -N(R⁸)- [where R⁸ is a hydrogen atom or a straight or branched C₁₋₆alkyl group], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)-, or -N(R⁸)SO₂N(R⁸)-, Ar² is an optionally substituted arylene or heteroarylene group and Alk is a chain -CH₂-CH(R)-, in which R is a carboxylic acid (-CO₂H) or a -CO₂Alk⁷ group;
Alk⁷ is a straight or branched C₁₋₈alkyl group, or a C₂₋₈alkenyl, C₂₋₈alkynyl, C₃₋₈cycloalkyl, C₃₋₈cycloalkylC₁₋₈alkyl, C₃₋₈heterocycloalkylC₁₋₆alkyl, C₁₋₆alkyloxyC₁₋₆alkyl, C₁₋₆alkylthioC ₁₋₆alkyl, C₁₋₆alkylsulfinylC₁₋₆alkyl, C₁₋₆alkylsulfonylC₁₋₆alkyl, C₃₋₈cycloalkyloxyC₁₋₆alkyl, C₃₋₈cycloalkylthioC1-6alkyl, C₃₋₈cycloalkylsulfinylC₁₋₆alkyl, C₃₋₈cycloalkylsulfonylC₁₋₆alkyl, C₁₋₆alkyloxycarbonylC₁₋₆alkyl, C₁₋₆alkyloxycarbonylC₁₋₆alkenyl, C₁₋₆alkyloxycarbonyloxyC₁₋₆alkyl, C₁₋₆alkyloxycarbonyloxyC₁₋₆alkenyl, C₃₋₈cycloalkyloxycarbonyloxyC₁₋₆alkyl, N-di-C₁₋₈alkylaminoC₁₋₈alkyl, N-C₆₋₁₂aryl-N-C₁₋₆alkylaminoC₁₋₆alkyl, N-di-C₁₋₈alkylcarbamoylC₁₋₈alkyl, C₆₋₁₀arylC₁₋₆alkyl, C₆₋₁₀aryl, C₆₋₁₀aryloxyC₁₋₈alkyl, C₆₋₁₂arylthioC₁₋₈alkyl, C₆₋₁₂arylsulfinylC₁₋₈alkyl, C₆₋₁₂arylsulfonylC₁₋₈alkyl, C₁₋₈alkanoyloxyC₁₋₈alkyl, C₄₋₈imidoC₁₋₈alkyl, C₆₋₁₂aroyloxyC₁₋₈alkyl, or a triglyceride group;
R² is a hydrogen atom;
the ring Cy is an unsaturated cycloaliphatic or heterocycloaliphatic ring containing X, in which X is a N atom or a C(R^{w}) group;
R^{w} is a group R^{z};
R^{x} which may be present on any available carbon atom of the ring Cy is a oxo (=O) group;
m is the integer 1;
R^{z} which may be present on any available carbon or nitrogen atom of the ring Cy is selected from a halogen atom or -(Alk⁴)ᵥL¹(Alk¹₎ₙR³ in which Alk⁴ is a straight or branched C₁₋₃alkylene chain, v is zero or the integer 1, L¹ is a covalent bond or a linker atom or group as defined for L^{2a}, n is zero or the integer 1, Alk¹ is an optionally substituted aliphatic chain and R³ is a hydrogen atom or a -CN, -NO₂ or an optionally substituted heteroaliphatic, cycloaliphatic, heterocycloaliphatic, polycycloaliphatic, heteropolycycloaliphatic, aromatic or hetero-aromatic group;
p is zero or the integer 1, 2, 3 or 4;
provided that Cy is not a cyclobutenedione group; and the compound of formula (1) is not dimedonyl-(S)-tyrosine-O-benzyl methyl ester;
and the salts, solvates, hydrates and N-oxides thereof.

It will be appreciated that compounds of formula (1) may have one or more chiral centres, and exist as enantiomers or diastereomers. The invention is to be understood to extend to all such enantiomers, diastereomers and mixtures thereof, including racemates. Formula (1) and the formulae hereinafter are intended to represent all individual isomers and mixtures thereof, unless stated or shown otherwise.

Optionally substituted aromatic groups represented by Ar¹ when present in the group R¹ include for example optionally substituted monocyclic or bicyclic fused ring C₆₋₁₂ aromatic groups, such as phenyl, 1- or 2-naphthyl, 1- or 2-tetrahydronaphthyl, indanyl or indenyl groups.

Optionally substituted heteroaromatic groups represented by the group Ar¹ when present in the group R¹ include for example optionally substituted C₁₋₉ heteroaromatic groups containing for example one, two, three or four heteroatoms selected from oxygen, sulphur or nitrogen atoms. In general, the heteroaromatic groups may be for example monocyclic or bicyclic fused ring heteroaromatic groups. Monocyclic heteroaromatic groups include for example five- or six-membered heteroaromatic groups containing one, two, three or four heteroatoms selected from oxygen, sulphur or nitrogen atoms. Bicyclic heteroaromatic groups include for example eight- to thirteen-membered fused-ring heteroaromatic groups containing one, two or more heteroatoms selected from oxygen, sulphur or nitrogen atoms.

Particular examples of heteroaromatic groups of these types include pyrrolyl, furyl, thienyl, imidazolyl, N-C₁₋₆alkylimidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-thiadiazole, pyridyl, pyrimidinyl, pyridazinyl, pyrazinyl, 1,3,5-triazinyl, 1,2,4-triazinyl, 1,2,3-triazinyl, benzofuryl, [2,3-dihydro]benzofuryl, [2,3-dihydro]benzothienyl, benzothienyl, benzotriazolyl, indolyl, isoindolyl, benzimidazolyl, imidazo[1,2-a]pyridyl, benzothiazolyl, benzoxazolyl, benzopyranyl, [3,4-dihydro]benzopyranyl, quinazolinyl, quinoxalinyl, naphthyridinyl, e.g. 2,6-naphthyridinyl, pyrido[3,4-b]pyridyl, pyrido[3,2-b]pyridyl, pyrido[4,3-b]-pyridyl, quinolinyl, isoquinolinyl, tetrazolyl, 5,6,7,8-tetrahydroquinolinyl, 5,6,7,8-tetrahydroisoquinolinyl, and imidyl, e.g. succinimidyl, phthalimidyl, or naphthalimidyl such as 1,8-naphthalimidyl.

Each aromatic or heteroaromatic group represented by the group Ar¹ may be optionally substituted on any available carbon or, when present, nitrogen atom. One, two, three or more of the same or different substituents may be present and each substituent may be selected for example from an atom or group -L³(Alk²)ₜL⁴(R⁴)ᵤ in which L³ and L⁴, which may be the same or different, is each a covalent bond or a linker atom or group, t is zero or the integer 1, u is an integer 1, 2 or 3, Alk² is an aliphatic or heteroaliphatic chain and R⁴ is a hydrogen or halogen atom or a group selected from optionally substituted C₁₋₆alkyl, C₃₋₈ cycloalkyl, C₆₋₁₂aromatic or C₁₋₉heteroaromatic, -OR⁵ [where R⁵ is a hydrogen atom, an optionally substitued C₁₋₆alkyl or C₃₋₈ cycloalkyl group], -SR⁵, -NR⁵R⁶ [where R⁶ is as just defined for R⁵ and may be the same or different], -NO₂, -CN, -CO₂R⁵, -SO₃H, -SOR⁵, -SO₂R⁵, -SO₃R⁵, -OCO₂R⁵, -CONR⁵R⁶, -OCONR⁵R⁶, -CSNR⁵R⁶, -COR⁵, -OCOR⁵, -N(R⁵)COR⁶, -N(R⁵)CSR⁶, -SO₂N(R⁵)(R⁶), -N(R⁵)SO₂R⁶, N(R⁵)CON(R⁶)(R⁷) [where R⁷ is a hydrogen atom, an optionally substituted C₁₋₆alkyl or C₃₋₈cycloalkyl group], -N(R⁵)CSN(R⁶)(R⁷) or -N(R⁵)SO₂N(R⁶)(R⁷), provided that when t is zero and each of L³ and L⁴ is a covalent bond then u is the integer 1 and R⁴ is other than a hydrogen atom

Optionally substituted C₆₋₁₂aromatic and C₁₋₉heteroaromatic groups represented by R⁴ include those aromatic and heteroaromatic groups as described hereinbefore for the group Ar¹. Optional substituents which may be present on these groups include those R¹³ optional substituents as described hereinafter.

When L³ and/or L⁴ is present in these substituents as a linker atom or group it may be any divalent linking atom or group. Particular examples include -O- or -S- atoms or -C(O)-, -C(O)O-, -OC(O)-, -C(S)-, -S(O)-, -S(O)₂-, -N(R⁸)- [where R⁸ is a hydrogen atom or an optionally substituted straight or branched C₁₋₆alkyl group], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-,' -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)-, or -N(R⁸)SO₂N(R⁸)- groups. Where the linker group contains two R⁸ substituents, these may be the same or different.

When R⁴, R⁵, R⁶, R⁷ and/or R⁸ is present as a C₁₋₆alkyl group it may be a straight or branched C₁₋₆alkyl group, e.g. a C₁₋₃alkyl group such as a methyl or ethyl group. C₃₋₈cycloalkyl groups represented by R⁴, R⁵, R⁶, R⁷ and/or R⁸ include C₃₋₆cycloalkyl groups e.g. cyclopropyl, cyclobutyl, cyclopentyl and cyclohexyl groups. Optional substituents which may be present on such groups include for example one, two or three substituents which may be the same or different selected from halogen atoms, for example fluorine, chlorine, bromine or iodine atoms, or hydroxy or C₁₋₆alkoxy e.g. methoxy or ethoxy groups.

When the groups R⁵ and R⁶ or R⁶ and R⁷ are both C₁₋₆alkyl groups these groups may be joined, together with the N atom to which they are attached, to form a heterocyclic ring. Such heterocyclic rings may be optionally interrupted by a further heteroatom selected from -O-, -S- or -N(R⁵)-. Particular examples of such heterocyclic rings include piperidinyl, morpholinyl, thiomorpholinyl, pyrrolidinyl, imidazolidinyl and piperazinyl rings.

When Alk² is present as an aliphatic or heteroaliphatic chain it may be for example any divalent chain corresponding to the below-mentioned aliphatic or heteroaliphatic group described for Alk¹ or R³ respectively.

Halogen atoms represented by R⁴ in the optional Ar¹ substituents include fluorine, chlorine, bromine, or iodine atoms.

Examples of the substituents represented by -L³(Alk¹)ₜL⁴(R⁴)ᵤ when present in Ar¹ groups in compounds of the invention include atoms or groups -L³Alk²L⁴R⁴, -L³Alk²R⁴, -L³R⁴, -R⁴ and -Alk²R⁴ wherein L³, Alk², L⁴ and R⁴ are as defined above. Particular examples of such substituents include -L³CH₂L⁴R⁴, -L³CH(CH₃)L⁴R⁴, -L³CH(CH₂)₂L⁴R⁴, -L³CH₂R⁴, -L³CH(CH₃)R⁴, -L³(CH₂)₂R⁴, -CH₂R⁴, -CH(CH₃)R⁴ , -(CH₂)₂R⁴ and -R⁴ groups.

Thus Ar¹ in compounds of the invention may be optionally substituted for example by one, two, three or more halogen atoms, e.g. fluorine, chlorine, bromine or iodine atoms, and/or C₁₋₆alkyl, e.g. methyl, ethyl, n-propyl, i-propyl, n-butyl or t-butyl, C₃₋₈cycloalkyl, e.g. cyclopropyl, cyclobutyl, cyclopentyl or cyclohexyl, C₁₋₆hydroxyalkyl, e.g. hydroxymethyl, hydroxyethyl or -C(OH)(CF₃)₂, carboxyC₁₋₆alkyl, e.g. carboxyethyl, C₁₋₆alkylthio e.g. methylthio or ethylthio, carboxyC₁₋₆alkylthio, e.g. carboxymethylthio, 2-carboxyethylthio or 3-carboxy-propylthio, C₁₋₆alkoxy, e.g. methoxy or ethoxy, hydroxyC₁₋₆alkoxy, e.g. 2-hydroxyethoxy, haloC₁₋₆alkyl, e.g. -CF₃, -CHF₂, CH₂F, haloC₁₋₆alkoxy, e.g. -OCF₃, -OCHF₂, -OCH₂F, C₁₋₆alkylamino, e.g. methylamino or ethylamino, amino (-NH₂), aminoC₁₋₆alkyl, e.g. aminomethyl or aminoethyl, C₁₋₆dialkylamino, e.g. dimethylamino or diethylamino, C₁₋₆alkylaminoC₁₋₆alkyl, e.g. ethylaminoethyl, C₁₋₆ dialkylaminoC₁₋₆alkyl, e.g. diethylaminoethyl, aminoC₁₋₆alkoxy, e.g. aminoethoxy, C₁₋₆alkylaminoC₁₋₆alkoxy, e.g. methylaminoethoxy, C₁₋₆dialkylaminoC₁₋₆alkoxy, e.g. dimethylaminoethoxy, diethylaminoethoxy, diisopropylaminoethoxy, or dimethylaminopropoxy, nitro, cyano, amidino, hydroxyl (-OH), formyl [HC(O)-], carboxyl (-CO₂H), -CO₂Alk³ [where Alk³ is as defined below for Alk⁷], C₁₋₆ alkanoyl e.g. acetyl, thiol (-SH), thioC₁₋₆alkyl, e.g. thiomethyl or thioethyl, sulphonyl (-SO₃H) -SO₃Alk³, C₁₋₆alkylsulphinyl, e.g. methylsulphinyl, C₁₋₆alkylsulphonyl, e.g. methylsulphonyl, aminosulphonyl (-SO₂NH₂), C₁₋₆ alkylaminosulphonyl, e.g. methylaminosulphonyl or ethylaminosulphonyl, C₁₋₆dialkylaminosulphonyl, e.g. dimethylaminosulphonyl or diethylaminosulphonyl, phenylaminosulphonyl, carboxamido (-CONH₂), C₁₋₆alkylaminocarbonyl, e.g. methylaminocarbonyl or ethylaminocarbonyl, C₁₋₆dialkylaminocarbonyl, e.g. dimethylaminocarbonyl or diethylaminocarbonyl, aminoC₁₋₆alkylaminocarbonyl, e.g. aminoethylaminocarbonyl, C₁₋₆dialkylaminoC₁₋₆alkylaminocarbonyl, e.g. diethylaminoethylaminocarbonyl, aminocarbonylamino, C₁₋₆alkylaminocarbonylamino, e.g. methylaminocarbonylamino or ethylaminocarbonylamino, C₁₋₆dialkylaminocarbonylamino, e.g. dimethylaminocarbonylamino or diethylaminocarbonylamino, C₁₋₆alkylaminocabonylC₁₋₆alkylamino, e.g. methylaminocarbonylmethylamino, aminothiocarbonylamino, C₁₋₆alkylaminothiocarbonylamino, e.g. methylaminothiocarbonylamino or ethylaminothiocarbonylamino, C₁₋₆dialkylaminothiocarbonylamino, e.g. dimethylaminothiocarbonylamino or diethylaminothiocarbonylamino, C₁₋₆alkylaminothiocarbonylC₁₋₆alkylamino, e.g. ethylaminothiocarbonylmethylamino, C₁₋₆alkylsulphonylamino, e.g. methylsulphonylamino or ethylsulphonylamino, C₁₋₆dialkylsulphonylamino, e.g. dimethylsulphonylamino or diethylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, C₁₋₆alkanoylamino, e.g. acetylamino, amino C₁₋₆alkanoylamino e.g. aminoacetylamino, C₁₋₆dialkylaminoC₁₋₆alkanoylamino, e.g. dimethylaminoacetylamino, C₁₋₆alkanoylaminoC₁₋₆alkyl, e.g. acetylaminomethyl, C₁₋₆alkanoylaminoC₁₋₆alkylamino, e.g. acetamidoethylamino, C₁₋₆alkoxycarbonylamino, e.g. methoxycarbonylamino, ethoxycarbonylamino or t-butoxycarbonylamino groups.

L² when present as part of the group R¹ in compounds of the invention is a linker atom or group L^{2a} or a linker -(Alk^{a})L^{2a}-, where Alk^{a} is an optionally substituted aliphatic or heteroaliphatic chain as previously defined for Alk², and L^{2a} is a linker atom or group as described above for L³ and L⁴.

Optionally substituted arylene groups represented by Ar² when present as part of the group R¹ include those aromatic groups as previously described for Ar¹.

Optionally substituted heteroarylene groups represented by Ar² when present as part of the group R¹ include those heteroaromatic groups as previously described for Ar¹.

Each arylene or heteroarylene group represented by Ar² may be attached to the remainder of the molecule through any available ring carbon or nitrogen atoms.

The arylene and heteroarylene groups represented by Ar² may be optionally substituted by one, two or more substituents selected from the atoms or groups -L³(Alk²)ₜL⁴(R⁴)ᵤ described herein. Where two of these atoms or groups are present they may be the same or different.

When the group R is present in R¹ in compounds of the invention as a derivative of a carboxylic acid it may be a carboxylic acid ester -CO₂Alk⁷ group as defined herein.

Ester (-CO₂Alk⁷) derivatives of the carboxylic acid group (-CO₂H) in compounds of formula (1) may advantageously be used as prodrugs of the active compound. Such prodrugs are compounds which undergo biotransformation to the corresponding carboxylic acid prior to exhibiting their pharmacological effects and the invention particularly extends to produgs of the acids of formula (1). Such prodrugs are well known in the art, see for example International Patent Application No. WO00/23419, Bodor, N. (Alfred Benzon Symposium, 1982, 17, 156-177), Singh, G. et al (J. Sci. Ind. Res., 1996, 55, 497-510) and Bundgaard, H., (Design of Prodrugs, 1985, Elsevier, Amsterdam).

Esterified carboxyl groups represented by the group -CO₂Alk⁷ wherein Alk⁷ include groups is a straight or branched optionally substituted C₁₋₈alkyl group such as a methyl, ethyl, n-propyl, i-propyl, n-butyl, i-butyl, s-butyl or t-butyl group, an optionally substituted C₂₋₈alkenyl group such as a propenyl e.g. 2-propenyl or butenyl e.g. 2-butenyl or 3-butenyl group, an optionally substituted C₂₋₈alkynyl group such as a ethynyl, propynyl e.g. 2-propynyl or butynyl e.g. 2-butynyl or 3-butynyl group, an optionally substituted C₃₋₈cycloalkyl group such as a cyclopropyl, cyclobutyl, cyclopentyl, cyclohexyl or cycloheptyl group; an optionally substituted C₃₋₈cycloalkylC₁₋₈alkyl group such as a cyclopentylmethyl, cyclohexylmethyl or cyclohexylethyl group; an optionally substituted C₃₋₈heterocycloalkylC₁₋₆alkyl group such as a morpholinyl-N-ethyl, thiomorpholinyl-N-methyl, pyrrolidinyl-N-ethyl, pyrrolidinyl-N-propyl, piperidinyl-N-ethyl, pyrazolidinyl-N-methyl or piperazinyl-N-ethyl group; an optionally substituted C₁₋₆alkyloxyC₁₋₆alkyl group such as a methyloxyethyl or propyloxyethyl group; an optionally substituted C₁₋₆alkylthioC₁₋₆alkyl group such as an ethylthioethyl group; an optionally substituted C₁₋₆alkylsulfinylC₁₋₆alkyl group such as an methylsulfinylethyl group; an optionally substituted C₁₋₆alkylsulfonylC₁₋₆alkyl group such as an methylsulfonylmethyl group; an optionally substituted C₃₋₈cycloalkyloxyC₁₋₆alkyl group such as a cyclohexyloxy-methyl group; an optionally substituted C₃. ₈cycloalkylthioC₁₋₆alkyl group such as a cyclopentylthiomethyl group; an optionally substituted C₃₋₈cycloalkylsulfinylC₁₋₆alkyl group such as a cyclopentylsulfinylmethyl group; an optionally substituted C₃₋₈cycloalkylsulfonylC₁₋₆alkyl group such as a cyclopentylsulfonylmethyl group; an optionally substituted C₁₋₆alkyloxycarbonylC₁₋₆alkyl group such as isobutoxycarbonylpropyl group; an optionally substituted C₁₋₆alkyloxycarbonylC₁₋₆alkenyl group such as isobutoxycarbonylpentenyl group; an optionally substituted C₁₋₆alkyloxycarbonyloxyC₁₋₆alkyl group such as an isopropoxycarbonyloxyethyl e.g a 1-(isopropoxycarbonyloxy) ethyl, 2-(isopropoxycarbonyloxy)ethyl or ethyloxycarbonyloxymethyl group; an optionally substituted C₁₋₆alkyloxycarbonyloxyC₁₋₆alkenyl group such as a isopropoxycarbonyloxybutenyl group, an optionally substituted C₃₋₈cycloalkyloxycarbonyloxyC₁₋₆alkyl group such as a cyclohexyloxycarbonyloxyethyl, e.g. a 2-(cyclohexyloxycarbonyloxy)ethyl group, an optionally substituted N-di-C₁₋₈alkylaminoC₁₋₈alkyl group such as a N-dimethylaminoethyl or N-diethylaminoethyl group; an optionally substituted N-C₆₋₁₂aryl-N-C₁₋₆-alkylaminoC₁₋₆alkyl group such as a N-phenyl-N-methylaminomethyl group; an optionally substituted N-di-C₁₋₈alkylcarbamoylC₁₋₈alkyl group such as a N-diethylcarbamoylmethyl group; an optionally substituted C₆₋₁₀aryl C₁₋₆alkyl group such as an optionally substituted benzyl, phenylethyl, phenylpropyl, 1-naphthylmethyl or 2-naphthylmethyl group; a C₆₋₁₀ aryl group such as an optionally substituted phenyl, 1-naphthyl or 2-naphthyl group; a C₆₋₁₀aryloxyC₁₋₈alkyl group such as an optionally substituted phenyloxymethyl, phenyloxyethyl, 1-naphthyloxymethyl, or 2-naphthyloxymethyl group; a C₆₋₁₂arylthioC₁₋₈alkyl group such as an optionally substituted phenylthioethyl group; a C₆₋₁₂arylsulfinylC₁₋₈alkyl group such as an optionally substituted phenylsulfinylmethyl group; a C₆₋₁₂arylsulfonylC₁₋₈alkyl group such as an optionally substituted phenylsulfonylmethyl group; an optionally substituted C₁₋₈alkanoyloxyC₁₋₈alkyl group, such as a acetoxymethyl, ethoxycarbonyloxyethyl, pivaloyloxymethyl, propionyloxyethyl or propionyloxypropyl group; an optionally substituted C₄₋₈imidoC₁₋₈alkyl group such as a succinimidomethyl or phthalamidoethyl group; a C₆₋₁₂aroyloxyC₁₋₈alkyl group such as an optionally substituted benzoyloxyethyl or benzoyloxypropyl group or a triglyceride such as a 2-substituted triglyceride e.g. a 1,3-di-C₁₋₈alkylglycerol-2-yl group such as a 1,3-diheptylglycerol-2-yl group.

Optional substituents which may be present on the Alk⁷ group include R^{13a} substituents as defined hereinafter.

Unsaturated cycloaliphatic groups represented by the ring Cy include unsaturated C₄₋₁₀ cycloaliphatic groups. Particular examples of unsaturated cycloaliphatic groups represented by Cy include C₃₋₇cycloalkenyl groups.

Particular examples of unsaturated cycloaliphatic groups represented by Cy include cyclobutenyl, cyclopentenyl, cyclohexenyl and cycloheptenyl groups.

Unsaturated heterocycloaliphatic groups represented by the ring Cy include unsaturated C₃₋₁₀ heterocycloaliphatic groups. Particular examples include optionally substituted C₃₋₁₀ heterocycloalkenyl e.g. C₃₋₇ heterocycloalkenyl groups, each of said groups containing one, two, three or four heteroatoms or heteroatom containing groups L⁷ where L⁷ is as defined above for L³ where L³ is a linker atom or group. It will be appreciated that when X in the ring Cy is a nitrogen atom then a further heteroatom or heteroatom containing group L⁷ need not be present in the ring Cy.

Particular examples of unsaturated heterocycloaliphatic groups represented by Cy include dihydrofuranyl, dihydrothiophenyl, 2,3-dihydrothiophene-1,1-dioxide, 3-amino-4,5-dihydro-1,1-dioxothiophenyl [where amino refers to the group NR¹ R²], 3,4-dihydrol-1,1-dioxo-2H-thiopyranyl, 2,3,4,5-tetrahydro-1,1-dioxo-thiopinyl, pyrrolinyl, e.g. 2- or 3-pyrrolinyl, oxazolinyl, thia-zolinyl, imidazolinyl, pyrazolinyl, isoxazolinyl, isothiazolinyl, pyranyl, dihydropyridyl, tetrahydropyridyl, dihydropyridazinyl, tetrahydropyridazinyl, dihydropyrimidinyl, tetrahydropyrimidinyl, dihydropyrazinyl, tetrahydro-pyrazinyl, dihydrotriazinyl, tetrahydrotriazinyl, oxazinyl or dihydrothiadia-zinyl group.

The unsaturated cycloaliphatic or heterocycloaliphatic ring represented by Cy is substituted by a group R^{x}, where R^{x} is a oxo (=O), group. It will be understood that when the ring Cy includes such a group R^{x}, then the corresponding hydroxy (-OH), tautomers in which the double bond migrates to become part of the ring Cy are also included.

When the group R³⁰ is present in compounds of formula (1) as an aliphatic group it may be any aliphatic chain as described hereinafter for Alk¹ but with each containing a terminal hydrogen atom. When the group R³⁰ is present in compounds of formula (1) as a heteroaliphatic, cycloaliphatic, heterocycloaliphatic, aromatic or heteroaromatic group it may be any heteroaliphatic, cycloaliphatic, heterocycloaliphatic, aromatic or heteroaromatic group as described hereinafter for the group R³. Optional substituents which may be present on these groups include any optional substituents described herein in relation to the corresponding Alk¹ aliphatic chains or R³ heteroaliphatic, cycloaliphatic, heterocycloaliphatic, aromatic or heteroaromatic groups.

The group R^{z} when present may be attached to any suitable carbon or nitrogen atom of the unsaturated cycloaliphatic or heterocycloaliphatic ring represented by Cy.

Halogen atoms represented by the group R^{z} include fluorine, chlorine, bromine and iodine atoms.

C₁₋₃ alkylene chains represented by Alk⁴ in the group R^{z} in compounds of formula (1) include for example a -CH₂-, -CH₂CH₂-, -CH₂CH₂CH₂-, -CH(CH₃)CH₂- or -CH₂CH(CH₃)- chain.

When present the linker atom or group represented by L¹ in the group R^{z} in compounds of formula (1) may be any linker atom or group as described above for the linker atom or group L³.

When Alk¹ is present in the group R^{z} in compounds of formula (1) as an optionally substituted aliphatic chain it may be an optionally substituted C₁₋₁₀ aliphatic chain. Particular examples include optionally substituted straight or branched chain C₁₋₆ alkylene, C₂₋₆ alkenylene, or C₂₋₆ alkynylene chains.

Particular examples of aliphatic chains represented by Alk¹ include optionally substituted -CH₂-, -(CH₂)₂-, -CH(CH₃)CH₂-, -(CH₂)₂CH₂-, -(CH₂)₃CH₂-, -CH(CH₃)(CH₂)₂-, -CH₂CH(CH₃)CH₂-, -C(CH₃)₂CH₂-, -CH₂C(CH₃)₂CH₂-, -(CH₂)₂C(CH₃)₂CH₂-, -(CH₂)₄CH₂-, -(CH₂)₅CH₂-, -CHCH-, -CHCHCH₂-, -CH₂CHCH-, -CHCHCH₂CH₂-, -CH₂CHCHCH₂-, -(CH₂)₂CHCH-, -CC-, -CCCH₂-, -CH₂CC-, -CCCH₂CH₂-, -CH₂CCCH₂- or -(CH₂)₂CCH- groups.

Heteroaliphatic groups represented by the group R³ when present in the group R^{z} in compounds of formula (1) include the aliphatic chains just described for Alk¹ but with each containing a terminal hydrogen atom and additionally containing one, two, three or four heteroatoms or heteroatom-containing groups. Particular heteroatoms or groups include atoms or groups L⁵ where L⁵ is as defined above for L³ when L³ is a linker atom or group. Each L⁵ atom or group may interrupt the aliphatic group, or may be positioned at its terminal carbon atom to connect the group to an adjoining atom or group. Particular examples include optionally substituted -L⁵CH₃, -CH₂L⁵CH₃, -L⁵CH₂CH₃, -CH₂L⁵CH₂CH₃, -(CH₂)₂L⁵CH₃, -(CH₂)₃L⁵CH₃, -L⁵(CH₂)₃, and -(CH₂)₂L⁵CH₂CH₃ groups.

The optional substituents which may be present on aliphatic chains or heteroaliphatic groups represented by Alk¹ and R³ respectively or aliphatic groups represented by -Alk¹R³ include one, two, three or more substituents where each substituent may be the same or different and is selected from halogen atoms, e.g. fluorine, chlorine, bromine or iodine atoms, or -OH, -CO₂H, -CO₂R⁹, where R⁹ is an optionally substituted straight or branched C₁₋₆alkyl group as defined above for R⁴, -CONHR⁹, -CON(R⁹)₂, -COR⁹, -COCH₃, C₁₋₆alkoxy, e.g. methoxy or ethoxy, haloC₁₋₆alkoxy e.g. -OCF₃, thiol, -S(O)R⁹, -S(O)₂R⁹, C₁₋₆alkylthio e.g. methylthio or ethylthio, amino or substituted amino groups. Substituted amino groups include -NHR⁹ and -N(R⁹)₂ groups . Where two R⁹ groups are present in any of the above substituents these may be the same or different.

Optionally substituted cycloaliphatic groups represented by the group R³ when present in the group R^{z} in compounds of the invention include optionally substituted C₃₋₁₀ cycloaliphatic groups. Particular examples include optionally substituted C₃₋₁₀ cycloalkyl, e.g. C₃₋₇ cycloalkyl or C₃₋₁₀ cycloalkenyl, e.g C₃₋₇ cycloalkenyl groups.

Optionally substituted heterocycloaliphatic groups represented by the group R³ when present in the group R^{z} include optionally substituted C₃₋₁₀heterocycloaliphatic groups. Particular examples include optionally substituted C₃₋₁₀heterocycloalkyl, e.g. C₃₋₇ heterocycloalkyl, or C₃₋₁₀heterocycloalkenyl, e.g. C₃₋₇ hetercycloalkenyl groups, each of said groups containing one, two, three or four heteroatoms or heteroatom-containing groups L⁵ as defined above.

Optionally substituted polycycloaliphatic groups represented by the group R³ when present in the group R^{z} include optionally substitued C₇₋₁₀ bi- or tricycloalkyl or C₇₋₁₀bi- or tricycloalkenyl groups. Optionally substituted heteropolycycloaliphatic groups represented by the group R³ include the optionally substituted polycycloalkyl groups just described, but with each group additionally containing one, two, three or four L⁵ atoms or groups.

Particular examples of cycloaliphatic, polycycloaliphatic, heterocycloaliphatic and heteropolycycloaliphatic groups represented by the group R³ include optionally substituted cyclopropyl, cyclobutyl, cyclopentyl, cyclohexyl, cycloheptyl, 2-cyclobuten-1-yl, 2-cyclopenten-1-yl, 3-cyclopenten-1-yl, adamantyl, norbornyl, norbornenyl, tetrahydrofuranyl, tetrahydropyranyl, tetrahydrothiopyranyl, pyrroline, e.g. 2- or 3-pyrrolinyl, pyrrolidinyl, pyrrolidinone, oxazolidinyl, oxazolidinone, dioxolanyl, e.g. 1,3-dioxolanyl, imidazolinyl, e.g. 2-imidazolinyl, imidazolidinyl, pyrazolinyl, e.g. 2-pyrazolinyl, pyrazolidinyl, pyranyl, e.g. 2- or 4-pyranyl, piperidinyl, piperidinone, 1,4-dioxanyl, morpholinyl, morpholinone, 1,4-dithianyl, thiomorpholinyl, piperazinyl, 1,3,5-trithianyl, oxazinyl, e.g. 2H-1,3-, 6H-1,3-, 6H-1,2-, 2H-1,2- or 4H-1,4- oxazinyl, 1,2,5-oxathiazinyl, isoxazinyl, e.g. o- or p-isoxazinyl, oxathiazinyl, e.g. 1,2,5 or 1,2,6-oxathiazinyl, or 1,3,5,-oxadiazinyl groups.

The optional substituents which may be present on the cycloaliphatic, polycycloaliphatic, heterocycloaliphatic or heteropolycycloaliphatic groups represented by the group R³ include one, two, three or more substituents each selected from halogen atoms, e.g. fluorine, chlorine, bromine or iodine atoms, or C₁₋₆alkyl, e.g. methyl, ethyl or propyl, haloC₁₋₆alkyl, e.g. halomethyl or haloethyl such as difluoromethyl or trifluoromethyl, optionally substituted by hydroxyl, e.g. -C(OH)(CF₃)₂, C₁₋₆alkoxy, e.g. methoxy, ethoxy or propoxy, haloC₁₋₆alkoxy, e.g. halomethoxy or haloethoxy such as difluoromethoxy or trifluoromethoxy, thiol, C₁₋₆alkylthio e.g. methylthio, ethylthio or propylthio, or -(Alk^{4a})_{g}R¹⁰ groups in which Alk^{4a} is a straight or branched C₁₋₃alkylene chain, g is zero or an integer 1 and R¹⁰ is a -OH, -SH, -N(R¹¹)₂, (in which R¹¹ is an atom or group as defined herein for R⁸) -CN, -CO₂R¹¹, -NO₂, -CON(R¹¹)₂, -CSN(R¹¹)₂, -COR¹¹, -CSN(R¹¹)₂, -N(R¹¹)COR¹¹, -N(R¹¹)CSR¹¹, -SO₂N(R¹¹)₂, -N(R¹¹)SO₂R¹¹, -N(R¹¹)CON(R¹¹)₂, -N(R¹¹)CSN(R¹¹), N(R¹¹)SO₂N(R¹¹)₂ or optionally substituted phenyl group. Where two R¹¹ atoms or groups are present in these substituents these may be the same or different. Optionally substituted phenyl groups include phenyl substituted by one, two or three of the R¹³ groups described below

Additionally, when the group R³ is a heterocycloaliphatic group containing one or more nitrogen atoms each nitrogen atom may be optionally substituted by a group -(L⁶)ₚ(Alk⁵)_{q}R¹² in which L⁶ is -C(O)-, -C(O)O-, -C(S)-, -S(O)₂-, -CON(R¹¹)-, -CSN(R¹¹)- or SO₂ N(R¹¹)-; p is zero or an integer 1; Alk⁵ is an optionally substituted aliphatic or heteroaliphatic chain; q is zero or an integer 1; and R¹² is a hydrogen atom or an optionally substituted cycloaliphatic, heterocycloaliphatic, polycyclo-aliphatic, polyheterocycloaliphatic, aromatic or heteroaromatic group.

C₁₋₃alkylene chains represented by Alk^{4a} include those groups as previously described for Alk⁴.

Optionally substituted aliphatic or heteroaliphatic chains represented by Alk⁵ include those optionally substituted chains described above for Alk¹ and R³ respectively. Optional substituents which may be present on these groups include those described above in relation to Alk¹ and R³ aliphatic and heteroaliphatic chains.

Cycloaliphatic, heterocycloaliphatic, polycycloaliphatic or polyheterocycloaliphatic groups represented by R¹² include those groups just described for the group R³. Optional substituents which may be present on those groups include those described above in relation to R³ cycloaliphatic groups.

Aromatic or heteroaromatic groups represented by R¹² include those groups described herein for the group Ar¹. Optional substituents which may be present on these groups include those R¹³ optional substituents described hereinafter.

When the group R³ is an optionally substituted aromatic or heteroaromatic group it may be for example an aromatic or heteroaromatic group as described herein for the group Ar¹.

Optional substituents which may be present on the aromatic or heteroaromatic groups represented by the group R³ include one, two, three or more substituents, each selected from an atom or group R¹³ in which R¹³ is -R^{13a} or -Alk⁶(R^{13a})ₘ, where R^{13a} is a halogen atom, or an amino (-NH₂), substituted amino, nitro, cyano, amidino, hydroxyl (-OH), substituted hydroxyl, formyl, carboxyl (-CO₂H), esterified carboxyl, thiol (-SH), substituted thiol, -COR¹⁴ [where R¹⁴ is an -Alk⁶(R^{13a})ₘ, aryl or heteroaryl group], -CSR¹⁴, -SO₃H, -SOR¹⁴, -SO₂R¹⁴, -SO₃R¹⁴, -SO₂NH₂, -SO₂NHR¹⁴ SO₂N(R¹⁴)₂, -CONH₂, -CSNH₂, -CONHR¹⁴, -CSNHR¹⁴, -CON[R¹⁴]₂, -CSN(R¹⁴)₂, -N(R¹¹)SO₂R¹⁴, -N(SO₂R¹⁴)₂, -NH(R¹¹)SO₂NH₂, -N(R¹¹)SO₂NHR¹⁴, -N(R¹¹)SO₂N(R¹⁴)₂, -N(R¹¹)COR¹⁴, -N(R¹¹)CONH₂, -N(R¹¹)CONHR¹⁴, -N(R¹¹)CON(R¹⁴)₂, -N(R¹¹)CSNH₂, -N(R¹¹)CSNHR¹⁴, -N(R¹¹)CSN(R¹⁴)₂, -N(R¹¹)CSR¹⁴, -N(R¹¹)C(O)OR¹⁴, -SO₂NHet¹ [where -NHet¹ is an optionally substituted C₅₋₇cyclicamino group optionally containing one or more other -O- or -S-atoms or -N(R¹¹)-, -C(O)-, -C(S)-, S(O) or -S(O)₂ groups], -CONHet¹, -CSNHet¹, -N(R¹¹)SO₂NHet¹, -N(R¹¹)CONHet¹, -N(R¹¹)CSNHet¹, -SO₂N(R¹¹)Het² [where Het² is an optionally substituted monocyclic C₅₋₇carbocyclic group optionally containing one or more -O- or -S- atoms or -N(R¹¹)-, -C(O)- or -C(S)- groups], -Het², -CON(R¹¹)Het², -CSN(R¹¹)Het², -N(R¹¹)CON(R¹¹)Het², -N(R¹¹)CSN(R¹¹)Het², cycloaliphatic, heterocycloaliphatic, aryl or heteroaryl group; Alk⁶ is a straight or branched C₁₋₆alkylene, C₂₋₆alkenylene or C₂₋₆alkynylene chain, optionally interrupted by one, two or three -O- or -S- atoms or -S(O)ₙ [where n is an integer 1 or 2] or -N(R¹⁵)- groups [where R¹⁵ is a hydrogen atom or C₁₋₆alkyl, e.g. methyl or ethyl group]; and m is zero or an integer 1, 2 or 3. It will be appreciated that when two R¹¹ or R¹⁴ groups are present in one of the above substituents, the R¹¹ or R¹⁴ groups may be the same or different.

When in the group -Alk⁶(R^{13a})ₘ m is an integer 1, 2 or 3, it is to be understood that the substituent or substituents R^{13a} may be present on any suitable carbon atom in -Alk⁶. Where more than one R^{13a} substituent is present these may be the same or different and may be present on the same or different atom in -Alk⁶. Clearly, when m is zero and no substituent R^{13a} is present the alkylene, alkenylene or alkynylene chain represented by Alk⁶ becomes an alkyl, alkenyl or alkynyl group.

When R^{13a} is a substituted amino group it may be for example a group -NHR¹⁴ [where R¹⁴ is as defined above] or a group -N(R¹⁴)₂ wherein each R¹⁴ group is the same or different.

When R^{13a} is a halogen atom it may be for example a fluorine, chlorine, bromine, or iodine atom.

When R^{13a} is a substituted hydroxyl or substituted thiol group it may be for example a group -OR¹⁴ or a -SR¹⁴ or -SC(=NH)NH₂ group respectively.

Esterified carboxyl groups represented by the group R^{13a} include groups of formula -CO₂Alk⁷ wherein Alk⁷ is a group as defined hereinbefore.

When Alk⁶ is present in or as a substituent it may be for example a methylene, ethylene, n-propylene, i-propylene, n-butylene, i-butylene, s-butylene, t-butylene, ethenylene, 2-propenylene, 2-butenylene, 3-butenylene, ethynylene, 2-propynylene, 2-butynylene or 3-butynylene chain, optionally interrupted by one, two, or three -O- or -S-, atoms or -S(O)-, -S(O)₂- or -N(R⁹)- groups.

Cycloaliphatic or heterocycloaliphatic groups represented by the groups R^{13a} or R¹⁴ include those optionally substituted C₃₋₁₀cycloaliphatic or C₃₋₁₀ heterocycloaliphatic groups described above for R³.

Aryl or heteroaryl groups represented by the groups R^{13a} or R¹⁴ include mono- or bicyclic optionally substituted C₆₋₁₂ aromatic or C₁₋₉ heteroaromatic groups as described above for the group Ar¹. The aromatic and heteroaromatic groups may be attached to the remainder of the compound of formula (1) by any carbon or hetero e.g. nitrogen atom as appropriate.

When -NHet¹ or -Het² forms part of a substituent R¹³ each may be for example an optionally substituted pyrrolidinyl, pyrazolidinyl, piperazinyl, morpholinyl, thiomorpholinyl, piperidinyl or thiazolidinyl group. Additionally Het² may represent for example, an optionally substituted cyclopentyl or cyclohexyl group. Optional substituents which may be present on -NHet¹ or -Het² include those R⁷ substituents described above.

Particularly useful atoms or groups represented by R¹³ include fluorine, chlorine, bromine or iodine atoms, or C₁₋₆alkyl, e.g. methyl, ethyl, n-propyl, i-propyl, n-butyl or t-butyl, optionally substituted phenyl, pyridyl, pyrimidinyl, pyrrolyl, furyl, thiazolyl, thienyl, morpholinyl, thiomorpholinyl, piperazinyl, e.g. t-butyloxycarbonylpiperazinyl, pyrrolidinyl, dioxolanyl, dioxanyl, oxazolidinyl, thiazolidinyl, imidazolidinyl or piperidinyl, C₁₋₆hydroxyalkyl, e.g. hydroxymethyl or hydroxyethyl, carboxyC₁₋₆alkyl, e.g. carboxyethyl, C₁₋₆alkylthio e.g. methylthio or ethylthio, carboxyC₁₋₆alkylthio, e.g. carboxymethylthio, 2-carboxyethylthio or 3-carboxypropylthio, C₁₋₆alkoxy, e.g. methoxy or ethoxy, hydroxyC₁₋₆alkoxy, e.g. 2-hydroxyethoxy, optionally substituted phenoxy, pyridyloxy, thiazolyoxy, phenylthio or pyridylthio, C₄₋₇cycloalkyl, e.g. cyclobutyl, cyclopentyl, C₅₋₇cycloalkoxy, e.g. cyclopentyloxy, haloC₁₋₆alkyl, e.g. trifluoromethyl, haloC₁₋₆alkoxy, e.g. trifluoromethoxy, C₁₋₆alkylamino, e.g. methylamino, ethylamino or propylamino, C6-12arylCl-6alkylamino, e.g. benzylamino, 4-fluorobenzylamino or 4-hydroxyphenylethylamino, amino (-NH₂), aminoC₁₋₆alkyl, e.g. aminomethyl or aminoethyl, C₁₋₆dialkylamino, e.g. dimethylamino or diethylamino, aminoC₁₋₆alkylamino, e.g. aminoethylamino or aminopropylamino, optionally substituted Het¹NC₁₋₆alkylamino, e.g. 3-morpholinopropylamino, C₁₋₆alkylaminoC₁₋₆alkyl, e.g. ethylaminoethyl, C₁₋₆dialkylaminoC₁₋₆alkyl, e.g. diethylaminoethyl, aminoC₁₋₆alkoxy, e.g. aminoethoxy, C₁₋₆alkylaminoC₁₋₆alkoxy, e.g. methylaminoethoxy, C₁₋₆dialkylaminoC₁₋₆alkoxy, e.g. dimethylaminoethoxy, diethylaminoethoxy, diisopropylaminoethoxy, or dimethylaminopropoxy, hydroxyC₁₋₆alkylamino, e.g. 2-hydroxyethylamino, 3-hydroxypropylamino or 3-hydroxybutylamino, imido, such as phthalimido or naphthalimido, e.g. 1,8-naphthalimido, nitro, cyano, amidino, hydroxyl (-OH), formyl [HC(O)-], carboxyl (-CO₂H), -CO₂Alk⁷ [where Alk⁷ is as defined above], C₁₋₆ alkanoyl e.g. acetyl, propyryl or butyryl, optionally substituted benzoyl, thiol (-SH), thioC₁₋₆alkyl, e.g. thiomethyl or thioethyl, -SC(=NH)NH₂, sulphonyl (-SO₃H), -SO₃Alk⁷, C₁₋₆alkylsulphinyl, e.g. methylsulphinyl, ethylsulphinyl or propylsulphinyl, C₁₋₆alkylsulphonyl, e.g. methylsulphonyl, ethylsulphonyl or propylsulphonyl, aminosulphonyl (-SO₂NH₂), C₁₋₆alkylaminosulphonyl, e.g. methylaminosulphonyl, ethylaminosulphonyl or propylaminosulphonyl C₁₋₆dialkylaminosulphonyl, e.g. dimethylaminosulphonyl or diethylaminosulphonyl, phenylaminosulphonyl, carboxamido (-CONH₂), C₁₋₆alkylaminocarbonyl, e.g. methylaminocarbonyl, ethylaminocarbonyl or propylaminocarbonyl, C₁₋₆dialkylaminocarbonyl, e.g. dimethylaminocarbonyl or diethylaminocarbonyl, aminoC₁₋₆alkylaminocarbonyl, e.g. aminoethylaminocarbonyl, C₁₋₆alkylaminoC₁₋₆alkylaminocarbonyl, e.g. methylaminoethylaminocarbonyl, C₁₋₆dialkylaminoC₁₋₆alkylaminocarbonyl, e.g. diethylaminoethylaminocarbonyl, aminocarbonylamino, C₁₋₆alkylaminocarbonylamino, e.g. methylaminocarbonylamino or ethylaminocarbonylamino, C₁₋₆dialkylaminocarbonylamino, e.g. dimethylaminocarbonylamino or diethylaminocarbonylamino, C₁₋₆alkylaminocabonylC₁₋₆alkylamino, e.g. methylaminocarbonylmethylamino, aminothiocarbonylamino, C₁₋₆alkylaminothiocarbonylamino, e.g. methylaminothiocarbonylamino or ethylaminothiocarbonylamino, C₁₋₆dialkylaminothiocarbonylamino, e.g. dimethylaminothiocarbonylamino or diethylaminothiocarbonylamino, C₁₋₆alkylaminothiocarbonylC₁₋₆alkylamino, e.g. ethylaminothiocarbonylmethylamino, -CONHC(=NH)NH₂, C₁₋₆alkylsulphonylamino, e.g. methylsulphonylamino or ethylsulphonylamino, haloC₁₋₆alkylsulphonylamino, e.g. trifluoromethylsulphonylamino, C₁₋₆dialkylsulphonylamino, e.g. dimethylsulphonylamino or diethylsulphonylamino, optionally substituted phenylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, optionally substituted morpholinesulphonylamino or morpholinesulphonylC₁₋₆alkylamino, optionally substituted phenylaminosulphonylamino, C₁₋₆alkanoylamino, e.g. acetylamino, aminoC₁₋₆alkanoylamino e.g. aminoacetylamino, C₁₋₆dialkylaminoC₁₋₆salkanoylamino, e.g. dimethylaminoacetylamino, C₁₋₆alkanoylaminoC₁₋₆alkyl, e.g. acetylaminomethyl, C₁₋₆alkanoylaminoC₁₋₆alkylamino, e.g. acetamidoethylamino, C₁₋₆alkoxycarbonylamino, e.g. methoxycarbonylamino, ethoxycarbonylamino or t-butoxycarbonylamino or optionally substituted benzyloxy, pyridylmethoxy, thiazolylmethoxy, benzyloxycarbonylamino, benzyloxycarbonylaminoC₁₋₆alkyl e.g. benzyloxycarbonylaminoethyl, thiobenzyl, pyridylmethylthio or thiazolylmethylthio groups.

Where desired, two R¹³ substituents may be linked together to form a cyclic group such as a cyclic ether, e.g. a C₁₋₆alkylenedioxy group such as methylenedioxy or ethylenedioxy.

It will be appreciated that where two or more R¹³ substituents are present, these need not necessarily be the same atoms and/or groups. In general, the substituent(s) may be present at any available ring position in the aromatic or heteroaromatic group represented by R³.

Where desired two R^{z} substituents attached to a single carbon atom of the ring Cy may be linked together to form an optionally substituted spiro-linked monocyclic C₃₋₇cycloaliphatic or C₂₋₇heterocycloaliphatic group. C₃₋₇cycloaliphatic groups of this type include cyclopropyl, cyclobutyl, cyclopentyl, cyclohexyl, cycloheptyl, cyclopentenyl and cyclohexenyl groups. C₂₋₇heterocycloaliphatic groups include pyrrolidinyl, tetrahydrofuranyl, tetrahydropyranyl, piperidinyl, and tetrahydrothiophenyl groups. Optional substituents which may be present on such groups include those optional substituents described hereinbefore in relation to R³ cycloaliphatic and heterocycloaliphatic groups.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isothionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

In the compounds according to the invention the group R¹ is an Ar¹ L²Ar²Alk- group. In compounds of this type Ar¹ is preferably an optionally substituted phenyl, monocyclic heteroaromatic or bicyclic heteroaromatic group. Particularly useful monocyclic heteroaromatic groups are optionally substituted five- or six-membered heteroaromatic groups as described previously, especially five- or six-membered heteroaromatic groups containing one or two heteroatoms selected from oxygen, sulphur or nitrogen atoms. Nitrogen-containing groups are especially useful, particularly pyridyl or pyrimidinyl groups. Particularly useful substituents present on these Ar¹ groups include halogen atoms or alkyl, haloalkyl, -OR⁵, -SR⁵, -NR⁵R⁶, -CO₂H, -CO₂CH₃, -NO₂, -N(R⁵)COR⁶ or -CN groups as described above in relation to the compounds of formula (1). Particularly useful bicyclic heteroaromatic groups represented by Ar¹ include optionally substituted ten-membered fused-ring heteroaromatic groups containing one or two heteroatoms, especially nitrogen atoms. Particular examples include optionally substituted naphthyridinyl, especially 2,6-naphthyridinyl and 2,7-naphthyridinyl, quinolinyl and isoquinolinyl, especially isoquinolin-1-yl groups. Particular optional substituents include those just described for monocyclic heteroaromatic groups. In compounds according to the invention the ring Cy is preferably an optionally substituted unsaturated cycloaliphatic or heterocycloaliphatic ring. One particularly useful class of Cy rings is that in which m is the integer 1 and R^{x} represents an oxo substituent (=O) on a carbon atom adjoined to the group X. A further particularly useful class of Cy rings is that in which m is zero, X is a C(R^{w}) group and the ring Cy contains an L⁷ sulfoxide or sulfone heteroatom containing group within its ring structure.

A particularly useful group of compounds according to the invention has the formula (2a): 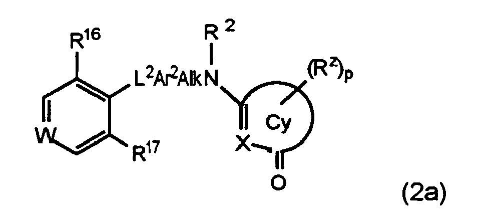 wherein -W= is -CH= or -N=;
R¹⁶ and R¹⁷, which may be the same or different is each a hydrogen atom or an atom or group -L³(Alk²)ₜL⁴(R⁴)ᵤ in which L³, Alk², t, L⁴ R⁴ and u are as defined previously;
L², Ar², Alk, R², Cy, X, R^{z} and p are as defined for formula (1);
provided that Cy is other than a cyclobutenedione group;
and the salts, solvates, hydrates and N-oxides thereof.

In one particularly useful class of compounds of formula (2a) -W= is a -N= atom.

In anoher particularly useful class of compounds of formula (2a) -W= is a -CH= group.

R¹⁶ and R¹⁷ in compounds of formula (2a) is each preferably as particularly described above for compounds of formula (1), other than a hydrogen atom. Particularly useful R¹⁶ and R¹⁷ substituents include halogen atoms, especially fluorine or chlorine atoms, or methyl, halomethyl, especially -CF₃, -CHF₂ or -CH₂F, methoxy or halomethoxy, especially -OCF₃, -OCHF₂ or -OCH₂F groups.

A further particularly useful group of compounds according to the invention has the formula (2b): 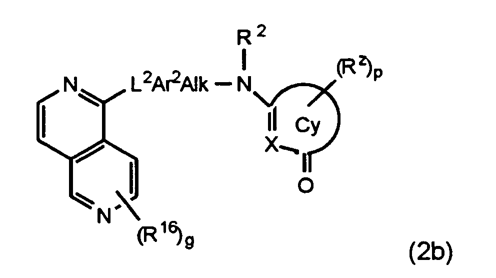 wherein R¹⁶, L², Ar², Alk, Cy, R², X, R^{z} and p are as defined for formula (2a);
g is the integer 1, 2, 3 or 4.
provided that Cy is other than a cyclobutenedione group;
and the salts, solvates, hydrates and N-oxides thereof.

Each R¹⁶ atom or group in compounds of formula (2b) may be independently selected from an atom or group -L³(Alk²)ₜL⁴(R⁴)ᵤ in which L2, Alk², t, L³, R⁴ and u are as previously defined. Particularly useful R¹⁶ substituents when present in compounds of formula (2b) include halogen atoms, especially fluorine or chlorine atoms, or straight or branched C₁₋₆alkyl, especially methyl, ethyl or isopropyl, C₃₋₈cycloalkyl especially cyclopropyl, haloC₁₋₆alkyl, especially halomethyl, most especially -CF₃ or -CHF₂, straight of branched C₁₋₆alkoxy, especially methoxy or ethoxy, haloC₁₋₆alkoxy, especially halomethoxy, most especially -OCF₃ or -OCHF₂, -SR⁵ especially methylthio or ethylthio, -CN, -CO₂Alk³, especially -CO₂CH₃, -NO₂, amino (-NH₂), substituted amino (-NR⁵R⁶), -N(R⁵)COR⁶, especially -NHCOCH₃, -COR⁵, especially -COCH₃, optionally substituted C₆₋₁₂aromatic, especially optionally substituted phenyl and C₁₋₉heteroaromatic groups, especially optionally substituted thienyl, pyridyl and pyrimidinyl groups.

A further particularly useful group of compounds according to the invention has the formula (2c): 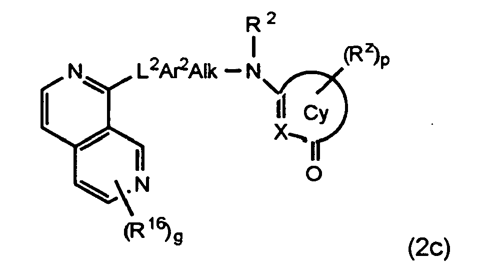 wherein R¹⁶, g, L², Ar², Alk, Cy, R², X, R^{z} and p are as defined for formula (2b);
provided that Cy is other than a cyclobutenedione group;
and the salts, solvates, hydrates and N-oxides thereof.

Each R¹⁶ atom or group in compounds of formula (2d) may be independently selected from an atom or group -L³(Alk²)ₜL⁴(R4)ᵤ as previously defined for compounds of formula (2b).

In one particularly preferred class of compounds of formula (2c) g is zero.

In another particularly preferred class of compounds of formula (2c) g is the integer 1 or 2.

An especially preferred class of compounds of formula (2c) is that where g is the integer 1 and R¹⁶ is a substituent at the 3-position of the 2,7-naphthyridine ring. In this class of compounds R¹⁶ is most preferably a methyl or halomethyl, especially -CF₃ group, or an optionally substituted phenyl group.

Particularly useful optional substituents which may be present on R¹⁶ aromatic and heteroaromatic groups when present in compounds of formula (2b) or (2c) include halogen atoms, especially fluorine and chlorine atoms, C₁₋₆alkyl groups, especially fluorine and chlorine atoms, C₁₋₆alkyl groups, especially methyl, ethyl and i-propyl groups and -CF₃, -OCH₃, -OCH₂CH₃, -OCH(CH₃)₂, -OCF₃, -SCH₃, -NHCH₃, -N(CH₃)₂, -CN, -CO₂CH₃, -COCH₃, and -N(CH₃)COCH₃ groups.

A further particularly useful group of compounds according to the invention has the formula (2d): 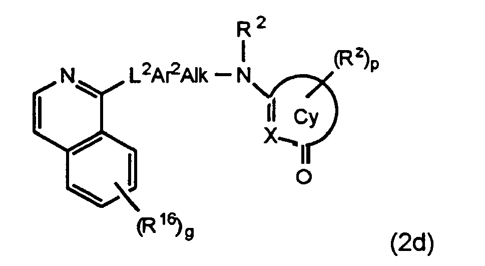 wherein R¹⁶, g, L², Ar², Alk, Cy, R², X, R^{z} and p are as defined for formula (2b);
provided that Cy is other than a cyclobutenedione group;
and the salts, solvates, hydrates and N-oxides thereof.

Each R¹⁶ atom or group in compounds of formula (2d) may be independently selected from an atom or group -L³(Alk²)ₜL⁴(R4)ᵤ as previously defined for compounds of formula (2b).

In one preferred class of compounds of formula (2d) g is zero.

In another preferred class of compounds of formula (2d) g is the integer 1. In this class of compounds R¹⁶ is preferably a substituent at the 3-positions of the isoquinoline ring as just defined. Most especially useful R¹⁶ substituents of this type include halogen atoms, especially fluorine and chlorine atoms and straight or branched C₁₋₆alkyl groups, especially methyl, ethyl or isopropyl, most especially methyl groups.

In another preferred class of compounds of formula (2d) g is the integer 2 or 3 where one R¹⁶ group is as just generally and particularly defined, and is located at the 3-position of the isoquinoline ring. In this class of compounds the second and when present third R¹⁶ optional substituents may be selected from an R¹⁶ optional substituent as described for compounds of formula (2b) or when g is the integer 3 a C₁₋₆alkylenedioxy group, especially a methylenedioxy or ethylenedioxy group. In one particularly useful group of compounds of this class g is the integer 2 where one R¹⁶ group is at the 3-position of the isoquinoline ring as previously generally and particularly described and the other R¹⁶ group is at the 6-, 7- or-8-position of the isoquinoline ring, most especially the 7-position. Most especially preferred substituents at the 7-position include a halogen atom, especially a fluorine or chlorine atom, or a C₁₋₆alkoxy group, especially a methoxy group.

It will be understood that compounds according to formulae (2a), (2b), (2c) and (2d) include, where applicable, the corresponding hydroxy tautomers.

Alk in compounds of the invention is -CH₂CH(R)-.

In another preferred class of the compounds of formulae (1), (2a), (2b), (2c) and (2d) R is preferably a -CO₂H group.

In a further preferred class of compounds of formulae (1) and (2) R is an esterified carboxyl group of formula -CO₂Alk⁷. In this class of compound Pdk⁷ is preferably a C₁₋₈alkyl group, especially a methyl, ethyl, propyl or i-propyl group, an optionally substituted C₆₋₁₀aryl group, especially a phenyl group, an optionally substituted C₆₋₁₀arylC₁₋₆alkyl group, especially a benzyl group, a C₃₋₈heterocycloalkylC₁₋₆alkyl group, especially a morpholinyl-N-ethyl group or a C₁₋₆alkyloxyC₁₋₆alkyl group, especially a methyloxyethyl group. Especially preferred esterified carboxyl groups include -CO₂CH₃, -CO₂CH₂CH₃, -CO₂CH₂CH₂CH₃ and -CO₂CH(CH₃)₂ groups.

In general in compounds of formula (2a) when W is a -N= atom, L² is preferably L^{2a} where L^{2a} is a -CON(R⁸)- group, especially -CONH- or -(Alka)L2a- where -(Alk^{a})L^{2a}- is especially a -CH₂O- group. Most preferred is a -CONH- group.

In general in compounds of formula (2a) when W is a -CH= group L² is preferably a covalent bond or L^{2a} where L^{2a} is a -CON(R)⁸- group, especially -CONH- or -(Alk^{a})L^{2a}- where -(Alk^{a})L^{2a}- is especially a -CH₂O-group. When W in compounds of formula (2a) is a -CH= group L² is most preferably a covalent bond.

In general in compounds of formula (2b), (2c) and (2d) L² is preferably L^{2a} where L^{2a} is an -O- atom or -N(R⁸)- group. An especially useful -N(R⁸)-group is -NH-.

The group Ar² in compounds of formulae (1), (2a), (2b), (2c) and (2d) is preferably an optionally substituted phenylene group. Particularly useful groups include optionally substituted 1,4-phenylene groups.

The ring Cy in compounds of formulae (1), (2a), (2b), (2c) and (2d) is preferably an optionally substituted cyclopentenyl, cyclohexenyl, cyclohepenyl, dihydropyrimidinyl, dihydropyridinyl or imidazolinyl group, each containing a oxo substituent (=O) within their ring structure on the carbon atom adjoined to the group X.

It will be understood that the corresponding hydroxy (-OH) tautomers of those oxo (=O) containing Cy rings, where the double bond migrates to become part of the Cy ring structure are also included in the definition or preferred Cy rings.

In one preferred class of compounds of formulae (1), (2a), (2b), (2c) and (2d) the group X in the unsaturated heteroaliphatic ring represented by Cy is preferably a N atom. Preferred Cy rings containing this X group include optionally substituted 2-aminopyrimidin-4-ones [where amino refers to the group -N(R²)-], 4-aminopyrimidin-2-ones, 2-aminopyridin-4-ones, 6-aminopyridin-2-ones and 2-aminoimidazolin-4-ones.

In another preferred class of compounds of formulae (1), (2a), (2b), (2c) and (2d) the group X in the unsaturated cycloaliphatic or heteroaliphatic ring represented by Cy is preferably a C(R^{w}) group. Particularly useful R^{w} atoms and groups that may be present in the group X include hydrogen and halogen atoms, especially fluorine, chlorine or bromine atoms or nitro, C₁₋₆alkyl, especially -CH₃, -CH₂CH₃, -CH₂CH₂CH₃, -(CH₂)₃CH₃, -CH(CH₃)₂, -C(CH₃)₃, -CH(CH₂CH₃)₂, -CH₂CH(CH₃)₂, and -CH₂C(CH₃)₃ C₂₋₆alkynylene, especially -CH₂CHCH₂ and -CH₂CH₂CHCH₂, C₂₋₆alkylnylene especially -CH₂CCH and -CH₂CH₂CCH, optionally substituted C₁₋₆alkylC₆₋₁₂aromatic, especially optionally substituted benzyl or phenylethyl, optionally substituted C₆₋₁₂aromatic, especially optionally substituted phenyl, optionally substituted C₃₋₁₀cycloaliphatic, especially optionally substituted C₃₋₇cycloalkyl, most especially optionally substituted cyclopentyl, cyclohexyl or cycloheptyl or optionally substituted C₃₋₁₀heterocycloaliphatic especially C₃₋₇heterocycloalkyl, most especially tetrahydropyranyl and tetrahydrothiopyranyl groups. Particularly preferred Cy rings in compounds of formulae (1), (2a), (2b), (2c) and (2d) containing such X groups include optionally substituted 3-amino-2-cyclopenten-1-one [where amino refers to the group -N(R²)-] and 3-amino-2-cyclohexen-1-one rings.

In general in compounds of formula (1), (2a), (2b), (2c) and (2d) p is preferably zero or the integer 1 or 2.

In one preferred class of compounds of formulae (1), (2a), (2b), (2c) and (2d) p is zero or the integer 1 or 2. In this class of compounds Cy is preferably an optionally substituted 3-amino-2-cyclopenten-1-one ring. In one most preferred group of compounds of this class p is zero.

In another preferred class of compounds of formulae (1), (2a), (2b), (2c) and (2d) p is zero or the integer 1 or 2. In this class of compounds Cy is preferably an optionally substituted 3-amino-2-cyclohexen-1-one ring.

In general in compounds of formulae (1), (2a), (2b), (2c) and (2d) when v and n in the group R^{z} are zero or the integer 1 the group R³ may especially be an optionally substituted heteroaliphatic, cycloaliphatic, heterocycloaliphatic, aromatic or heteroaromatic group as defined herein. Particularly useful groups of this type include optionally substituted C₂₋₆heteroalkyl, particularly C₁₋₃alkoxyC₁₋₃alkyl, especially methoxypropyl, optionally substituted C₃₋₇cycloalkyl, especially optionally substituted cyclopropyl, cyclobutyl cyclopropyl or cyclohexyl, optionally substituted C₅₋₇heterocycloaliphatic, optionally substituted pyrrolidinyl or thiazolidinyl, optionally substituted phenyl and optionally substituted C₅₋₇heteroaromatic, especially optionally substituted pyridyl groups. Optional substituents on these groups include in particular R¹³ atoms or groups where the group is an aromatic or heteroaromatic group and -(L⁶)ₚ(Alk⁵)_{q}R¹² groups as described earlier where the group is a nitrogen-containing heterocycloaliphatic group such as a pyrrolidinyl or thiazolidinyl group. Particularly useful -(L⁶)ₚ(Alk⁵)_{q}R¹² groups include those in which L⁶ is a -CO- group. Alk⁵ in these groups is preferably present (i.e. q is preferably an integer 1) and in particular is a -CH₂- chain. Compounds of this type in which R¹² is a hydrogen atom or an optionally substituted aromatic or heteroaromatic group, especially an optionally substituted phenyl, pyridyl or imidazolyl group are particularly preferred.

In another preferred class of compounds of formulae (1), (2a), (2b), (2c) and (2d) v is zero, Alk¹ in the group R^{z} is present as an optionally substituted aliphatic chain as defined herein (i.e. n is the integer 1) and R³ is a hydrogen atom. Compounds of this type where -Alk¹R³ is an optionally substituted C₁₋₆alkyl group, particularly a methyl, ethyl, n-propyl i-propyl, i-butyl, t-butyl or n-butyl group or an allyl (-CH₂CHCH₂) or (propargyl) -CH₂CCH group are especially useful. In one preferred group of compounds of this type L¹ is a covalent bond. In another preferred group of compounds of this type L¹ is a group -N(R⁸)- as previously generally and particularly defined.

Particularly preferred optional substituents which may be present on aliphatic groups of formula -Alk¹ R³ include one, two, three or more halogen atoms, esepcially fluorine, chlorine or bromine atoms or C₁₋₆alkoxy groups e.g. methoxy or ethoxy, haloC₁₋₆alkoxy groups e.g. -OCF₃, substituted amino groups e.g. -NHCH₃ or -N(CH₃)₂ or -COR⁹ groups e.g. -COCH₃ or carboxyl (-CO₂H) or esterified carboxyl e.g. -CO₂CH₃ or -CO₂C(CH₃)₂ groups.

In one most preferred class of compounds of formulae (1), (2a), (2b), (2c) and (2d) p is the integer 1 and R^{z} is an optionally substituted aliphatic group -Alk¹ R³ as just defined.

In another preferred class of compounds of formulae (1), (2a), (2b), (2c) and (2d) p is the integer 2 and R^{z} is an optionally substituted aliphatic group -Alk¹R³ as just defined. Most preferably the R^{z} groups are identical and especially preferred is the case where the R^{z} groups are each attached to the same carbon atom of the Cy ring.

in another preferred class of compounds of formulae (1), (2a), (2b), (2c) and (2d) p is the integer 2 and two R^{z} groups are joined to form a spiro-linked optionally substituted cycloaliphatic group (both R^{z} groups joined to a single carbon atom of Cy). Especially preferred optionally substituted cycloaliphatic groups of this type include spiro-fused cyclopentyl and cyclohexyl groups. Particularly preferred optional substituents which may be present on such groups include those just described in relation to -Alk¹R³.

Particularly useful compounds of the invention include:
(*S*)-3-[4-[(2,6-Naphthyridinyl)amino]phenyl]-2-[(2-(1-ethylpropyl)-3-oxo-1-cyclopentenyl)amino]propionic acid;
(*S*)-3-[4-[(3-Methyl-2,7-naphthyridinyl)oxy]phenyl]-2-[(2-propyl-3-oxo-1-cyclopentenyl)amino]propionic acid;
(*S*)-3-[(4-[3-Methyl-2,7-naphthyridinyl]oxy)phenyl]-2-[(2-isobutyl-3-oxo-1-cyclopentenyl)amino]propionic acid:
(*S*)-3-[(4-2,7-Naphthyridin-1-yl)oxy)phenyl]-2-[(2-isobutyl-3-oxo-1-cyclopentenyl)amino]propionic acid;
(*S*)-3-[4-[(3,5-Dichloropyrid-4-yl)carboxamido)phenyl]-2-[(2-isobutyl-3-oxo-1-cyclopentenyl)amino]propionic acid;
(*S*)-3-[4-[(3-Methyl-2,7-naphthyridin-1-yl)amino]phenyl]-2-(2-isobutyl-3-oxo-1-cyclopentenyl)amino]propionic acid;
(*S*)-3-[4-(-2',6'-Dimethoxy)biphenyl]-2-[(2-isobutyl-3-oxo-1-cyclopentenyl)amino]propionic acid;
(2*S*)-3-[4-(2,6-Naphthyridin-1-yl)amino)phenyl]-2-[(2-isobutyl-3-oxo-1-cyclopentenyl)amino]propionate;
(2*S*)-3-[4-(2,6-Naphthyridin-1-yl)amino]phenyl]-2-[(2-isopropyl-3-oxo-1-cyclopentenyl)aminolproplonic acid
(2*S*)-3-[4-(2,6-Naphthyridin-1-yl)amino)phenyl]-2-[(2-allyl-3-oxo-1-cyclopentenyl)amino]propionate;
(2*S*)-3-[4-(2,6-Naphthyridin-1-yl)amino)phenyl]-2-[(2-propyl-3-oxo-1-cyclopentenyl)amino]propionate,
and the salts, solvates, hydrates and N-oxides and carboxylic acid ester, particularly methyl, ethyl, propyl and i-propyl esters thereof.

Compounds according to the invention are potent and selective inhibitors of α4 integrins. The ability of the compounds to act in this way may be simply determined by employing tests such as those described in the Examples hereinafter.

The compounds are of use in modulating cell adhesion and in particular are of use in the prophylaxis and treatment of diseases or disorders including inflammation in which the extravasculation of leukocytes plays a role and the invention extends to such a use and to the use of the compounds for the manufacture of a medicament for treating such diseases or disorders,

Diseases or disorders of this type include inflammatory arthritis such as rheumatoid arthritis vasculitis or polydermatomyositis, multiple sclerosis, allograft rejection, diabetes, inflammatory dermatoses such as psoriasis or dermatitis, asthma and inflammatory bowel disease.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection e.g. by bolus injection or infusion. Formulations for injection may be presented in unit dosage form, e.g. in glass ampoule or multi dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100ng/kg to 100mg/kg e.g. around 0.01 mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds of the invention may be prepared by a number of processes as generally described below and more specifically in the Examples hereinafter. In the following process description, the symbols Ar¹, Ar², Alk, R¹, R², R³, L¹, L², Alk¹, Cy, X, R^{x}, m, R^{z}, p, R^{w} and n when used in the formulae depicted are to be understood to represent those groups described above in relation to formula (1) unless otherwise indicated. In the reactions described below, it may be necessary to protect reactive functional groups, for example hydroxy, amino, thio or carboxy groups, where these are desired in the final product, to avoid their unwanted participation in the reactions. Conventional protecting groups may be used in accordance with standard practice [see, for example, Green, T. W. in "Protective Groups in Organic Synthesis", John Wiley and Sons, 1999]. In some instances, deprotection may be the final step in the synthesis of a compound of formula (1) and the processes according to the invention described hereinafter are to be understood to extend to such removal of protecting groups. For convenience the processes described below all refer to a preparation of a compound of formula (1) but clearly the description applies equally to the preparation of compounds of formula (2).

Thus according to a further aspect of the invention, a compound of formula (1) in which R is a -CO₂H group may be obtained by hydrolysis of an ester of formula (3): 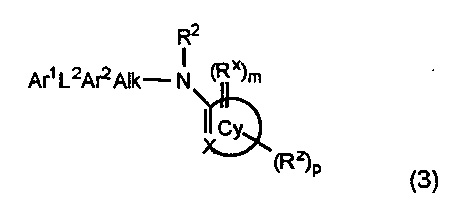 where Alk represents a group 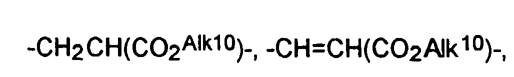 or 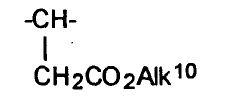 [where Alk¹⁰ is an alkyl group, for example a C₁₋₆alkyl group]

The hydrolysis may be performed using either an acid or a base depending on the nature of Alk¹⁰, for example an organic acid such as trifluoroacetic acid or an inorganic base such as lithium, sodium or potassium hydroxide optionally in an aqueous organic solvent such as an amide e.g. a substituted amide such as dimethylformamide, an ether e.g. a cyclic ether such as tetrahydrofuran or dioxane or an alcohol e.g. methanol at a temperature from ambient to the reflux temperature. Where desired, mixtures of such solvents may be used.

According to a further aspect of the invention a compound of formula (1) may be prepared by condensation of a compound of formula (4): 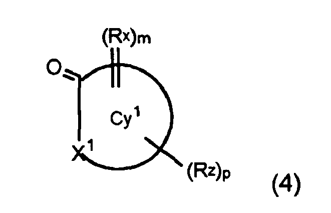 where Cy¹ is a cycloaliphatic or heterocycloaliphatic ring in which the double bond of the Cy ring of compounds of formula (1) is replaced by a single bond and an oxo (=O) substituent is attached to the carbon atom to which R¹ R²N- will subsequently be joined and X¹ is a CH(R^{w}) or NH group, with an amine R¹ R²NH or a salt thereof.

The reaction may be performed in an inert solvent or mixture of solvents, for example a hydrocarbon such as an aromatic hydrocarbon e.g. benzene or toluene and/or a halogenated hydrocarbon such as 1,2-dichloroethane, at a temperature from 0°C to the reflux temperature. Where necessary, for example when a salt of an amine R¹R²NH is used, an organic base such as diisopropylethylamine can be added.

Any carboxylic acid group present in the intermediate of formula (4) or the amine R¹R²NH may need to be protected during the displacement reaction, for example as an ethyl ester. The desired acid may then be obtained through subsequent hydrolysis, for example as particularly described above and generally described below.

A compound of formula (1) may also be prepared by displacement of a leaving group from a compound of formula (5): 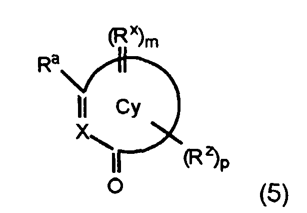 where R^{a} is a leaving group, with an amine R¹ R²NH or a salt thereof. Suitable leaving groups represented by R^{a} include a halogen atom especially a chlorine, bromine or iodine atom, or alkoxy e.g. methoxy or ethoxy or isopropyloxy, alkylthio e.g. methylthio or ethylthio, alkylsulphoxide e.g. methylsulphoxide, aryloxy e.g. dinitrophenyloxy or araalkoxy e.g. benzyloxy group.

The reaction may be performed in an inert solvent or mixture of solvents for example a substituted amide such as dimethylformamide, or alcohol such as methanol or ethanol and/or a halogenated hydrocarbon such as dichloromethane, at a temperature from 0°C to the reflux temperature. Where necessary, for example when the salt of an amine R¹ R²NH is used an organic base such as diisopropylethylamine can be added.

Where desired the displacement reaction may also be performed on an intermediate of formulae (4) or (5) or R¹R²NH which is linked, for example via its R¹ or R³ group, to a solid support, such as a polystyrene resin. After the reaction the desired compound of formula (1) may be displaced from the support by any convenient method, depending on the original linkage chosen.

Intermediates of formulae (4), (5) and R¹ R²NH may be obtained from simpler, known compounds by one or more standard synthetic methods employing substitution, oxidation, reduction or cleavage reactions. Particular substitution approaches include conventional alkylation, arylation, heteroarylation, acylation, thioacylation, halogenation, sulphonylation, nitration, formylation and coupling procedures. It will be appreciated that these methods may also be used to obtain or modify other compounds of formulae (1) and (2) where appropriate functional groups exist in these compounds.

Thus compounds of the invention and intermediates thereto may be prepared by alkylation, arylation or heteroarylation. For example, compounds containing a -L¹H or -L²H group (where L¹ and L² is each a linker atom or group) may be treated with a coupling agent R³(Alk¹)ₙX¹ or Ar¹X¹ respectively in which X¹ is a leaving atom or group such as a halogen atom, e.g. a fluorine, bromine, iodine or chlorine atom or a sulphonyloxy group such as an alkylsulphonyloxy, e.g. trifluoromethylsulphonyloxy or arylsulphonyloxy, e.g. p-toluenesulphonyloxy group.

The reaction may be carried out in the presence of a base such as a carbonate, e.g. caesium or potassium carbonate, an alkoxide, e.g. potassium t-butoxide, or a hydride, e.g. sodium hydride, or an organic amine e.g. triethylamine or N,N-diisopropylethylamine or a cyclic amine, such as N-methylmorpholine or pyridine, in a dipolar aprotic solvent such as an amide, e.g. a substituted amide such as dimethylformamide or an ether, e.g. a cyclic ether such as tetrahydrofuran.

Compounds of formula Ar¹ X¹ may be prepared from alcohols of formula Ar¹OH by reaction with a halogenating agent, for example a phosphorous oxyhalide such as phosphorous oxychloride at an elevated temperature e. g. 110°C.

Intermediate alcohols of formula Ar¹OH in which, for example, Ar¹ represents a 2,6-naphthyridine may be prepared by methods well known to a person skilled in the art, e.g. by the method of Sakamoto,T. et al [Chem. Pharm. Bull. 33, 626-633, (1985)].

Alternatively alkylating agents of formula Ar¹X¹ in which, for example, Ar¹ represents a 2,6-naphthyridine may be prepared by reaction of a 2,6-naphthyridine N-oxide or N, N'-dioxide with a halogenating agent, e.g. a phosphorous oxyhalide such as phosphorous oxychloride to give a 1-halo or 1,5-dihalo-2,6-napthyridine respectively. In the case of 1,5-dihalo-2,6-napthyridines each halogen atom may be substituted separately by a reagent such as HL²Ar²AlkN(R²)H or HL³(Alk²)ₜL⁴(R⁴)ᵤ by the particular methods just described above.

2,6-Napthyridine N-oxides and N,N'-dioxides may be generated from the corresponding 2,6-napthyridines by the general methods of synthesis of N-oxides described below or they may be synthesised by the methods of Numata, A et al (Synthesis, 1999, 306-311).

Further alkylating agents of formula Ar¹X¹ in which, for example, Ar¹ represents a 2,6-naphthyridine, may be prepared by the methods of Giacomello G. et al [Tetrahedron Letters, 1117-1121 (1965)], Tan, R. and Taurins, A. [Tetrahedron Lett., 2737-2744, (1965)], Ames, D. E. and Dodds, W. D. [J. Chem. Soc. Perkin 1, 705-710 (1972)] and Alhaique, F. et al [Tetrahedron Lett., 173-174 (1975)].

Intermediate alcohols of formula Ar¹ OH in which Ar¹ represents an optionally substituted 2,7-naphthyridin-1-yl group may be prepared by methods well known to a person skilled in the art, e.g. by the method of Sakamoto,T. et al [Chem. Pharm. Bull. 33, 626-633, (1985)] or Baldwin, J, J. et al [J. Org. Chem, 43, 4878-4880, (1978)]. Thus for example the method of Baldwin may be modified to allow the synthesis of intermediate 3-substituted 2,7-naphthyridin-1-yl groups of formula Ar¹ OH as depicted in Scheme 1:

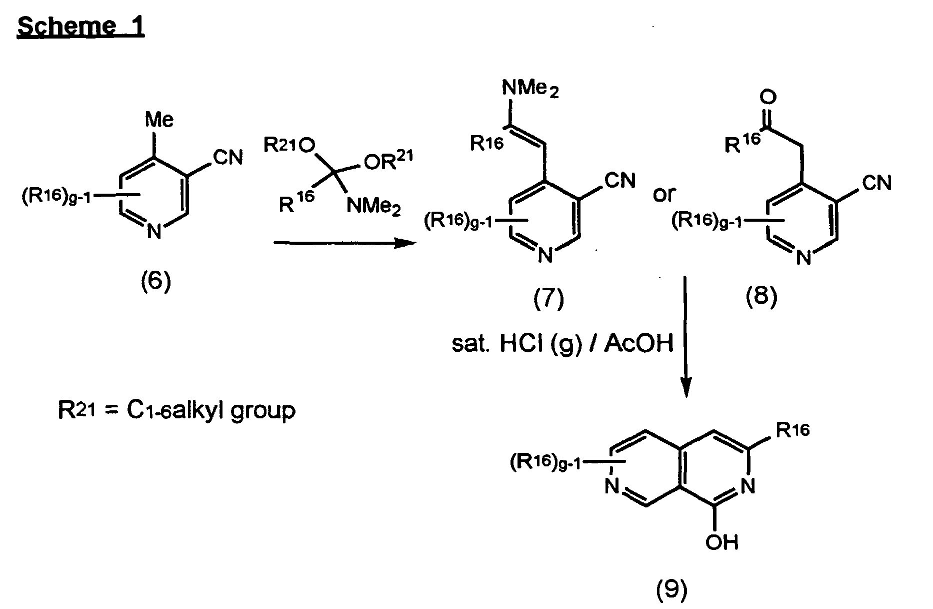

Reaction of an optionally substituted 4-methyl-3-cyano pyridine of formula (6) with a N,N-dimethylformamide di-C₁₋₆alkyl acetal, e.g. N,N-dimethylformamide diethyl acetal, in a dipolar solvent such as an amide e.g. a substituted amide such as dimethylformamide at an elevated temperature e.g. 140-150° gives a compound of formula (7) or (8) or a mixture thereof depending on the nature of the group R¹⁶.

Compounds of formula (7) or (8) may be cyclised to 3-substituted 2,7-naphthyridin-1-yl alcohol of formula (9) by treatment with an acid e.g. an inorganic acid such as hydrochloric acid or hydrobromic acid or an acidic gas such as hydrogen chloride gas in an organic solvent e.g. an organic acid such as acetic acid optionally in the presence of water at a temperature from about ambient to 50°C.

Alternatively alkylating agents of formula Ar¹X¹ in which Ar¹ represents an optionally substituted 2,7-naphthyridin-yl group may be prepared by reaction of a 2,7-naphthyridine N-oxide or N, N'-dioxide with a halogenating agent, e.g. a phosphorous oxyhalide such as phosphorous oxychloride to give a 1-halo or 1,6-dihalo- and/or-1,8-dihalo-2,7-napthyridine respectively. In the case of 1,6-dihalo- and/or 1,8-dialo-2,6-napthyridines each halogen atom may be substituted separately by a reagent such as HL²Ar²AlkN(R²)H or HL³(Alk²)ₜL⁴(R⁴)ᵤ by the particular methods just described above.

2,7-Napthyridine N-oxides and N,N'-dioxides may be generated from the corresponding 2,7-napthyridines by the general methods of synthesis of N-oxides described below or they may be synthesised by the methods of Numata, A et al (Synthesis, 1999, 306-311).

Further alkylating agents of formula Ar¹X¹ in which, for example, Ar¹ represents a 2,7-naphthyridin-1-yl, may be prepared by the methods of Wenkert E. et al J. Am. Chem. Soc. 89, 6741-5 (1967), and Aust. J. Chem. 433 (1972), and Sheffield D.J. J. Chem. Soc. Perkin. Trans I, 2506 (1972).

Intermediate alcohols of formula Ar¹OH in which Ar¹ represents a 3-substituted isoquinolin-1-yl group may be prepared by methods well known to a person skilled in the art, e.g. by the methods of Wu M.-J. et al Tetrahedron, 55, 13193-200 (1999), Hiebl J. et al Tetrahedron Lett. 40, 7935-8 (1999), Nagarajan A. et al Indian J. Chem., Sect. B, 28B, 67-78 (1989), Brun E. M. et al Synlett, 7, 1088-90 (1999) and Brun, E. M. et al Synthesis, 273-280 (2000).

Further alkylating agents of formula Ar¹X¹ in which, for example, Ar¹ represents a isoquinolin-1-yl group, may be prepared by the methods of Falk H. et al Monatsch. Chem. 25, 325-33 (1994), and Deady, L. W. et al Aust. J. Chem 42, 1029-34 (1989).

In a further example intermediates of formula R¹ R²NH may be obtained by reaction of a compound of formula Ar¹L²H with a compound of formula X¹Ar²AlkN(R²)H under the reaction conditions just described

Compounds of formula Ar¹ L²H in which, for example Ar¹ represents a 2,6-naphthyridine and L² is a -N(R⁸)- group, may be prepared from substituted 4-cyano-3-cyanomethylpyridines by the methods of Alhaique, F. *et al (ibid* and Gazz. Chim. Ital. 1975, 105, 1001-1009) or from 3-fomylpyridines by the methods of Molina, P. at al (Tetrahedron 1992, 48, 4601-4616).

Compounds of formula Ar¹ L²H in which, for example Ar¹ represents a 2,7-naphthyridin-1-yl group and L² is a -N(R⁸)- group, may be prepared from substituted 4-formylpyridines by the methods of Molina, P. et al Tetrahedron, 48, 4601-4616, (1992), or by the methods described in US 3,938,367.

Compounds of formula Ar¹L²H in which, for example Ar¹ represents a 3-substituted isoquinolin-1-yl group and L² is a -N(R⁸)- group, may be prepared by the methods of Bordner, J. et al J. Med. Chem. 31, 1036-9 (1988), Tovar J. D. et al J. Org. Chem., 64, 6499-6504 (1999), Karser E. M. et al Synthesis, 11, 805-6 (1974), and Molino, P et al J. Chem. Soc. Perkin Trans. 1 1727-31 (1990).

In another example, compounds containing a -L¹ H or -L²H or group as defined above may be functionalised by acylation or thioacylation, for example by reaction with one of the alkylating agents just described but in which X¹ is replaced by a -C(O)X², -C(S)X², -N(R⁸)COX² or -N(R⁸)C(S)X² group in which X² is a leaving atom or group as described for X¹. The reaction may be performed in the presence of a base, such as a hydride, e.g. sodium hydride or an amine, e.g. triethylamine or N-methylmorpholine, in a solvent such as a halogenated hydrocarbon, e.g. dichloromethane or carbon tetrachloride or an amide, e.g. dimethylformamide, at for example ambient temperature. Alternatively, the acylation may be carried out under the same conditions with an acid (for example one of the alkylating agents described above in which X¹ is replaced by a -CO₂H group) in the presence of a condensing agent, for example a diimide such as 1-(3-dimethylaminopropyl)-3-ethylcarbodiimide or N,N'-dicyclohexylcarbodiimide, advantageously in the presence of a catalyst such as a N-hydroxy compound e.g. a N-hydroxytriazole such as 1-hydroxybenzotriazole. Alternatively the acid may be reacted with a chloroformate, for example ethylchloroformate, prior to the desired acylation reaction

In a further example compounds may be obtained by sulphonylation of a compound containing an -OH group by reaction with one of the above alkylating agents but in which X¹ is replaced by a -S(O)Hal or -SO₂ Hal group [in which Hal is a halogen atom such as chlorine atom] in the presence of a base, for example an inorganic base such as sodium hydride in a solvent such as an amide, e.g. a substituted amide such as dimethylformamide at for example ambient temperature.

In another example, compounds containing a -L¹ H or -L²H group as defined above may be coupled with one of the alkylation agents just described but in which X¹ is replaced by an -OH group in a solvent such as tetrahydrofuran in the presence of a phosphine, e.g. triphenylphosphine and an activator such as diethyl, diisopropyl- or dimethylazodicarboxylate.

In a further example, ester groups -CO₂R⁵, -CO₂R¹¹ or -CO₂Alk⁷ in the compounds may be converted to the corresponding acid [-CO₂H] by acid- or base-catalysed hydrolysis depending on the nature of the groups R⁵, R¹ or Alk⁷. Acid- or base-catalysed hydrolysis may be achieved for example by treatment with an organic or inorganic acid, e.g. trifluoroacetic acid in an aqueous solvent or a mineral acid such as hydrochloric acid in a solvent such as dioxan or an alkali metal hydroxide, e.g. lithium hydroxide in an aqueous alcohol, e.g. aqueous methanol.

In a further example, -OR⁵ or -OR¹⁴ groups [where R⁵ or R¹⁴ each represents an alkyl group such as methyl group] in compounds of formula (1) may be cleaved to the corresponding alcohol -OH by reaction with boron tribromide in a solvent such as a halogenated hydrocarbon, e.g. dichloromethane at a low temperature, e.g. around -78°C.

Alcohol [-OH] groups may also be obtained by hydrogenation of a corresponding -OCH₂R¹⁴ group (where R¹⁴ is an aryl group) using a metal catalyst, for example palladium on a support such as carbon in a solvent such as ethanol in the presence of ammonium formate, cyclohexadiene or hydrogen, from around ambient to the reflux temperature. In another example, -OH groups may be generated from the corresponding ester [CO₂Alk⁷ or CO₂R⁵] or aldehyde [-CHO] by reduction, using for example a complex metal hydride such as lithium aluminium hydride or sodium borohydride in a solvent such as methanol.

In another example, alcohol -OH groups in the compounds may be converted to a corresponding -OR⁵ or -OR¹⁴ group by coupling with a reagent R⁵OH or R¹⁴OH in a solvent such as tetrahydrofuran in the presence of a phosphine, e.g. triphenylphosphine and an activator such as diethyl-, diisopropyl-, or dimethylazodicarboxylate.

Aminosulphonylamino [-NHSO₂NHR³ or -NHSO₂NHAr¹] groups in the compounds may be obtained, in another example, by reaction of a corresponding amine [-NH_{2]} with a sulphamide R³NHSO₂NH₂ or Ar¹ NHSO₂NH₂ in the presence of an organic base such as pyridine at an elevated temperature, e.g. the reflux temperature.

In another example compounds containing a -NHCSAr¹, -CSNHAr¹, -NHCSR³ or -CSNHR³ may be prepared by treating a corresponding compound containing a -NHCOAr¹, -CONHAr¹, -NHCOR³ or -CONHR³ group with a thiation reagent, such as Lawesson's Reagent, in an anhydrous solvent, for example a cyclic ether such as tetrahydrofuran, at an elevated temperature such as the reflux temperature.

In a further example amine (-NH₂) groups may be alkylated using a reductive alkylation process employing an aldehyde and a borohydride, for example sodium triacetoxyborohyride or sodium cyanoborohydride, in a solvent such as a halogenated hydrocarbon, e.g. dichloromethane, a ketone such as acetone, or an alcohol, e.g. ethanol, where necessary in the presence of an acid such as acetic acid at around ambient temperature.

In a further example, amine [-NH₂] groups in compounds of formula (1) may be obtained by hydrolysis from a corresponding imide by reaction with hydrazine in a solvent such as an alcohol, e.g. ethanol at ambient temperature.

In another example, a nitro [-NO₂] group may be reduced to an amine [-NH₂], for example by catalytic hydrogenation using for example hydrogen in the presence of a metal catalyst, for example palladium on a support such as carbon in a solvent such as an ether, e.g. tetrahydrofuran or an alcohol e.g. methanol, or by chemical reduction using for example a metal, e.g. tin or iron, in the presence of an acid such as hydrochloric acid.

Aromatic halogen substituents in the compounds may be subjected to halogen-metal exchange with a base, for example a lithium base such as n-butyl or t-butyl lithium, optionally at a low temperature, e.g. around -78°C, in a solvent such as tetrahydrofuran and then quenched with an electrophile to introduce a desired substituent. Thus, for example, a formyl group may be introduced by using dimethylformamide as the electrophile; a thiomethyl group may be introduced by using dimethyldisulphide as the electrophile.

In another example, sulphur atoms in the compounds, for example when present in a linker group L¹ or L² may be oxidised to the corresponding sulphoxide or sulphone using an oxidising agent such as a peroxy acid, e.g. 3-chloroperoxybenzoic acid, in an inert solvent such as a halogenated hydrocarbon, e.g. dichloromethane, at around ambient temperature.

In another example compounds of formula Ar¹X¹(where X¹ is a halogen atom such as a chlorine, bromine or iodine atom) may be converted to such compounds as Ar¹ CO₂R²⁰ (in which R²⁰ is an optionally substituted alkyl, aryl or heteroaryl group), Ar¹CHO, Ar¹CHCHR²⁰, Ar¹CCR²⁰, Ar¹N(R²⁰)H, Ar¹N(R²⁰)₂, for use in the synthesis of for example compounds of formula Ar¹ L²Ar²AlkN(R²)H, using such well know and commonly used palladium mediated reaction conditions as are to be found in the general reference texts Rodd's Chemistry of Carbon Compounds, Volumes 1-15 and Supplementals (Elsevier Science Publishers, 1989), Fieser and Fieser's Reagents for Organic Synthesis, Volumes 1-19 (John Wiley and Sons, 1999), Comprehensive Heterocyclic Chemistry, Ed. Katritzky et al, Volumes 1-8, 1984 and Volumes 1-11, 1994 (Pergamon), Comprehensive Organic Functional Group Transformations, Ed. Katritzky et al, Volumes 1-7, 1995 (Pergamon), Comprehensive Organic Synthesis, Ed. Trost and Flemming, Volumes 1-9, (Pergamon, 1991), Encyclopedia of Reagents for Organic Synthesis, Ed. Paquette, Volumes 1-8 (John Wiley and Sons, 1995), Larock's Comprehensive Organic Transformations (VCH Publishers Inc., 1989) and March's Advanced Organic Chemistry (John Wiley and Sons, 1992).

N-oxides of compounds of formula (1) may be prepared for example by oxidation of the corresponding nitrogen base using an oxidising agent such as hydrogen peroxide in the presence of an acid such as acetic acid, at an elevated temperature, for example around 70° C to 80°C, or alternatively by reaction with a peracid such as peracetic acid in a solvent, e.g. dichloromethane, at ambient temperature.

Salts of compounds of formula (1) may be prepared by reaction of a compound of formula (1) with an appropriate base in a suitable solvent or mixture of solvents e.g. an organic solvent such as an ether e.g. diethylether, or an alcohol, e.g. ethanol using conventional procedures.

Where it is desired to obtain a particular enantiomer of a compound of formula (1) this may be produced from a corresponding mixture of enantiomers using any suitable conventional procedure for resolving enantiomers.

Thus for example diastereomeric derivatives, e.g. salts, may be produced by reaction of a mixture of enantiomers of formula (1) e.g. a racemate, and an appropriate chiral compound, e.g. a chiral base. The diastereomers may then be separated by any convenient means, for example by crystallisation and the desired enantiomer recovered, e.g. by treatment with an acid in the instance where the diastereomer is a salt.

In another resolution process a racemate of formula (1) may be separated using chiral High Performance Liquid Chromatography. Alternatively, if desired a particular enantiomer may be obtained by using an appropriate chiral intermediate in one of the processes described above.

Chromatography, recrystallisation and other conventional separation procedures may also be used with intermediates or final products where it is desired to obtain a particular geometric isomer of the invention.

The following Examples illustrate the invention. All temperatures are in °C. The following abbreviations are used:

| | |
|---|---|
| NMM - N-methylmorpholine; | EtOAc - ethyl acetate; |
| MeOH - methanol; | BOC - butoxycarbonyl; |
| DCM - dichloromethane; | AcOH - acetic acid; |
| DIPEA - diisopropylethylamine; | EtOH - ethanol; |
| Pyr - pyridine; | Ar - aryl; |
| DMSO - dimethylsulphoxide; | iPr - isopropyl; |
| Et₂O diethylether; | Me - methyl; |
| THF - tetrahydrofuran, | DMF - N,N-dimethylformamide; |
| FMOC - 9-fluorenylmethoxycarbonyl; | |
| DBU - 1,8-diazabicyclo[5,4,0]undec-7-ene | |

All NMR's were obtained at 300MHz and 400MHz.

### INTERMEDIATE 1

### 2-Benzyl-1,3-cyclopentanedione

To a suspension of 1,3-cyclopentanedione (500mg, 5.10mmol) and anhydrous lithium iodide (750mg, 5.61 mmol) in acetonitrile (10ml) was added DBU. After 30min benzyl bromide (1.74g, 10.2mmol) was added and the reaction heated for 24h at reflux. The reaction was poured into water (40ml), extracted with EtOAc (4 x 50ml), dried (MgSO₄), the solution concentrated *in vacuo* and the residue purified by chromatography (SiO₂; EtOAc) to give the title compound as an off white solid (222mg, 23%). δH (CD₃OD) 7.20 (4H, d), 7.10 (1H, m), 3.41 (2H, s), 2.52 (4H, s). m/z (ESI, 70V) 189 (MH⁺).

### INTERMEDIATE 2

### 2-Allyl-1,3-cyclopentanedione

Prepared in a similar manner to Intermediate 1, from 1,3-cyclopentanedione and alkyl bromide, heating to reflux for 3 days to give the title compound as an refluxing for 3d with allyl bromide off-white solid (115mg, 8%). δH (CD₃OD) 5.80 (1 H, m), 5.00-4.80 (2H, 3 x m), 2.85 (2H, d), 2.48 (4H, s) m/z (ESI, 70V) 139 (MH⁺).

### INTERMEDIATE 3

### 2-Butyl-1,3-cyclopentanedione

To a suspension of cyclopent-4-ene-1,3-dione (1.22g, 12.6mmol) in acetic anhydride (4ml) were added ethyl orthobutyrate (3.74g, 25.3mmol) and anhydrous ZnCl₂. The reaction was heated at 80° for 24h, solid removed by filtration, the filtrate concentrated *in vacuo* and the residues purified by chromatography (SiO₂; 70:30 hexane/EtOAc) to give 2-(α-methoxypropyl idene)cyclopent-4-ene-1,3-dione as a brown oil. δH (CDCl₃) 6.88 (2H, s), 4.07 (3H, s), 3.00 (2H, t), 1.63 (2H, m), 1.08 (3H, t). m/z (ESI, 70V) 181 (MH⁺).
A solution of this oil (EtOH, 11ml) was subjected to catalytic (Pd/C, 200mg) hydrogenation for 24h, filtered, the solution concentrated *in vacuo,* and the residue purified by chromatography (SiO₂; EtOAc) to give the title compound as a white, waxy solid (494mg, 25%). δH (CD₃OD) 2.46 (4H, s), 2.09 (2H, t, J 7.3Hz), 1.33 (4H, m), 0.89 (3H, t, J 9.5Hz). m/z (ESI, 70V) 155 (MH⁺).

### INTERMEDIATE 4

### 2-Phenyl-1,3-Cyclonentanedione

To a solution of BF₃.OEt₂ (1.3g, 9.2mmol) and benzaldehyde diemethylacetal (1.0g, 6.6mmol) in DCM (5ml) under N₂ at -78° was added a solution of 1,2-bistrimethylsilyloxycyclobutene (1.4g, 6.1mmol) in DCM (3ml) maintaining a temperature of less than -70°. After 3h at -78C the solution was poured into saturated NaHCO₃ solution (40ml), extracted into DCM, washed (NaHCO₃ x 1, H₂O x 1), dried (MgSO₄), the solution concentrated *in vacuo* and the residue distilled (170°, 0.4mm Hg) to give 1.1g of clear liquid. To this liquid was added TFA (20ml) and the solution heated at reflux for 15 min. The solvent was removed *in vacuo,* the residue slurried in Et₂O and the off-white solids isolated by filtration and dried to give the title compound (760mg, 72%). δH (d⁶-DMSO) 7.80 (2H, d, J 8.4Hz), 7.31 (2H, t), 7.16 (1 H, t, J 7.35HZ), 2.51 (4H, s). m/z (ESI, 70V) 175 (MH⁺).

### INTERMEDIATE 5

### 2-Propyl-1,3-cyclopentanedione

To a solution of BF₃.OEt₂ (1.30g, 9.2mmol) and butyraldehyde (0.40g, 5.5mmol) in DCM (5ml) under N₂ at -78° was added a solution of 1,2-bis trimethylsilyloxycyclobutene (1.4g, 6.1 mmol) in DCM (3ml) maintaining a temperature of less than -70°. After 2h at -78° the solution was poured into saturated NaHCO₃, extracted into EtOAc, washed (brine), dried (MgSO₄) and the solution concentrated *in vacuo* to yield a clear liquid. To this liquid was added TFA (20ml) and the solution heated at reflux for 24h. The solution was concentrated *in vacuo* and the residue purified by chromatography (SiO₂, 50:50 EtOAc/hexane) to yield the title compound as an off-white solid (140mg, 18%). δH (CD₃OD) 2.46 (4H, s), 2.07 (2H, t, J 7.5Hz), 1.40 (2H, m), 0.86 (3H, t, J 7.4Hz). m/z (ESI, 70V) 141 (MH⁺).

### INTERMEDIATE 6

### 2-Isopropyl-1,3-cyclopentanedione

To a solution of TiCl₄ (1.47g, 7.77mmol) and isobutyraldehyde (559mg, 7.75mmol) in DCM (16ml) under N₂ at -78° was added a solution of 1,2-bistrimethylsilyloxycyclobutene (1.97g, 8.56mmol) in DCM (16ml) maintaining a temperature of less than -70°. After 30min the reaction was poured into water, extracted into DCM, washed (H₂O x 2), dried (MgSO₄) and the solution concentrated *in vacuo* to yield a clear oil. To this oil was added TFA (20ml) and the solution heated at reflux for 24h. The solution was concentrated *in vacuo* and the residue purified by chromatography (SiO₂; 50:50 EtOAc/hexane) to give the title compound as an off-white solid (180mg, 17%). δH (d⁶-DMSO) 2.61 (1H, sept, J 7.0Hz), 2.29 (4H, s), 1.04 (6H, d, J 7.0Hz). m/z (ESI, 70V) 141 (MH⁺).

### INTERMEDIATE 7

### 2-Isobutyl-1,3-cyclopenentanedione

To a stirring mixtue of silica gel (45g) isobutyraldehyde (375mg, 5.20) and 4-methylbenzenethiol (3.80g, 30.6mmol) in DCM (15ml) was addd 1,3-cyclopentanedione (300mg, 3.06mmol). The suspension was stirred for 24h, the solvent removed *in vacuo,* the residues dry packed onto the top of a silica column and the column run with gradient elution (SiO₂; 100:0 - 50:50 Hexane/EtOAc) to give a thiol trapped intermediate as a white solid (400mg, 47%),. To this intermediate (300mg, 1.09mmol) in MeOH (5ml) was added NaCNBH₃ (55mg, 0.8mmol), the reaction stirred for 24h and quenched with water. The solution was concentrated *in vacuo* and the residue purified by chromatography (SiO₂: 50:50, EtOAc/hexane) to give the title compound as an off-white solid (80mg, 28% overall). δH (CD₃OD) 2.43 (4H, s), 1.96 (2H, d, J 7.1 Hz), 1.77 (1H, sept, J 6.6Hz), 0.83 (6H, d, J 6.6Hz). m/z (ESI, 70V) 155 (MH⁺).

### INTERMEDIATE 8

### 2-(1-Ethylpropyl)-1,3-cyclopentanedione

Prepared in a similar manner to intermediate 6 from 2-ethylbutyraldehyde δH (CD₃OD) 2.46 (4H, s), 2.30 (1H, m), 1.67 (2H, m), 1.52 (2H, m), 0.76 (6H, t, J 7.4Hz). m/z (ESI, 70V) 169 (MH⁺).

### INTERMEDIATE 9

### 2-Cyclohexyl-1,3-cyclopentanedione

To a suspension of 1,3-cyclopentandedione (4.00g, 0.040mmol) in water (30ml) were added LiOH.H₂O (1.88g, 0.045moll) and 3-bromocyclohexene (7.18g, 0.045mmol). The solution was stirred overnight, the pH raised to 12-14 (LiOH.H₂O) and stirred for 2h. The solution was washed with Et₂O (4 x 40ml), acidified to pH 1-2 and resulting solids isolated by filtration, washed and dried to give 2-cyclohexene-1,3-cyclopentanedione. [δH (CD₃OD) 5.69 (1H, m), 5.44 (1 H, m), 3.20 (1 H, m), 2.47 (4H, s), 2.15-1.19 ~(6H, m). m/z ESI, 70V) 179 (MH⁺)]. The product was dissolved in EtOH (50ml) and reduced under an atmosphere of H₂ at RT with Pd/C (200mg). After 5h the reaction was filtered and the solvent removed to give the title compound as a white solid (890mg 12%). δH (CD₃OD) 2.44 (4H, 2), 2.39 (1H, m), 1.85-1.70 (5H, m), 1.50-1.45 (2H, m), 1.35-1.15 (3H, m). m/z (ESI, 70V) 181 (MH⁺).

### INTERMEDIATE 10

### 4-(2-(N,N-Dimethylamino)ethylen-1-yl)-3-cyanopyrridine

A solution of 4-methyl-3-cyanopyridine (prepared acccording to Ref: J. Prakt. Chem. 338, 663 (1996), 8.0g, 67.8mmol) and N,N dimethylformamide diethyl acetal (11.0g, 74.8mmol) in dry DMF (50ml) was stirred at 140° under N₂ for 2 days. An additional portion of N,N,-dimethylformamide diethyl acetal (5g) was added and stirred at 140° for 4h. The volatiles were removed *in vacuo* and the obtained dark oil partitioned between EtOAc (300ml) and water (50ml). The phases were separated and the aqueous layer re-extracted with EtOAc (3 × 100ml). The combined organic extracts were washed with brine (30ml), dried (Na₂SO₄), treated with activated charcoal, filtered and evaporated in *vacuo* to afford essentially pure title compound as a dull orange solid (10.1g, 85%). δH (CDCl₃) 8.49 (1H, s), 8.25 (1h, d, J 5.9hz), 7.29 (1H, d, J 13.2Hz), 7.09 (1 H, d, J 5.9Hz), 5.25 (1 H, d, J 13.2Hz) and 2.99 (6H, s); m/z (ES⁺, 70V) 174 (MH⁺).

### INTERMEDIATE 11

### 1-Hydroxy-2,7-naphthyridine hydrochloride salt

HCl gas was bubbled through a stirred solution of Intermediate 10 (6.2g, 3.58mmol) in glacial acetic acid (50ml) and water (0.64ml, 3.55mmol) for 1-2min. The reaction mixture was stirred in a stoppered flask at 40° for 18h. The volatiles were removed *in vacuo* affording a dark residue, which was treated with water (3 × 20ml) and re-evaporated *in vacuo.* The obtained dark semi-solid was treated with 40ml warm ethanol, ice-cooled, and the undissolved solid collected by filtration affording the title compound as a green coloured solid (5.2g, 80%) δH (DMSO-D⁶) 12.5 (1 H, br s), 9.38 (1 H, s), 8.84 (1 H, d, J 7.0Hz), 8.15 (1 H, d, J 7.0Hz), 7.89 (1 H, br dd, J 7.0, 5.0Hz) and 6.85 (1 H, d, J 7.0Hz); m/z (ES⁺, 70V), 147 (MH⁺).

### INTERMEDIATE 12

### 1 -Chloro-2,7-naphthyridine

Intermediate 11 (5.2g, 28.5mmol) was stirred with phosphorous oxychloride (75ml) at 110° for 24h. The volatiles were removed *in vacuo* affording a dark oil which was poured into an ica-bath cooled mixture of saturated aqueous NaHC0₃ (1 00ml containing 20g solid NaHC0₃) and EtOAc (100ml). After thorough mixing the phases were separated and the aqueous layer re-extracted with EtOAc (2 x 75ml). The combined organic extracts were washed with brine (15m1), dried (Na₂S0₄) and evaporated in *vacuo* to afford the title compound as a yellow solid (4.0g, 85%) δH (CDCl₃) 9.45 (1 H, s), 8.81 (1 H, d, J 5.7HZ), 8.47 (1 H, d, J 5.7Hz), 7.66 (1 H, d, J 5.7Hz) and 7.60 (1 H, d, J 5.7HZ); m/z (ES⁺, 70V) 165 and 167 (MH⁺).

### INTERMEDIATE 13

### Ethyl (S)-3-[4-(2,7-naphthyridin-1-ylamino)phenyl]-2-amino propanoate

A solution of ethyl-(*S*)-3-[4-aminophenyl]-2-[*t*-butoxycarbonylamino] propanoate (638mg, 2.07mmol) and Intermediate 12 (310mg, 1.88mmol) in ethoxyethanol (2ml) was stirred at 120° for 15 min and at 100° for 1h under nitrogen. The volatiles were removed *in vacuo* and the dark residue partitioned between EtOAc (70ml) and saturated aqueous NaHCO₃ (10ml). The phases were separated and the aqueous layer re-extracted with EtOAc (2 x 30ml). The combined organic extracts were washed with brine (10ml), dried (Na₂SO₄) and evaporated *in vacuo* to afford a dark foam. Chromatography (SiO₂; 5 to 10% MeOH/DCM) afforded a mixture of ethyl-(*S*)-3-[4-(2,7-naphthyridin-1-ylamino)phenyl]-2-[(*t*-butoxycarbonyl) amino]propanoate and some of the title compound (730mg). This mixture was treated with a solution of trifluoroacetic acid (5ml) and DCM (5ml) at room temperature for 1h. The volatiles were removed *in vacuo* and the residue partitioned between EtOAc (75ml) and saturated aqueous NaHCO₃ (20ml). The phases were separated and the aqueous layer re-extracted with EtOAc (3 x 30ml). The combined organic extracts were washed with brine (10ml), dried (Na₂SO₄) and evaporated *in vacuo* to afford an orange solid. Chromatography (silica; 10% MeOH/DCM) afforded the title compound as a straw-coloured solid (420mg, 60% over two steps). δH (CDCl₃) 10.70 (1 H, s), 10.31 (1 H, s), 9.44 (1 H, d, J 5.6Hz), 8.94 (1 H, d, J 5.6Hz), 8.55 (1H, d, J 7.3Hz), 8.54 (2H, d, J 8.5Hz), 8.46 (1 H, d, J 5.6Hz), 7.94 (2H, d, J 8.5Hz), 4.84 (2H, q, J 7.1 Hz), 4.35 (1 H, t, J 6.6Hz), 4.10 (2H, br s), 3.64 (1 H, dd, J 13.5, 6.4Hz), 3.56 (1H, dd, J 13.5, 7.0Hz) and 1.95 (3H, t, J 7.1 Hz); m/z (ES⁺, 70V) 337 (MH⁺).

### INTERMEDIATE 14

### Ethyl(S)-3-[4-(2,7-naphthyridin-1-yloxy)phenyl]-2-aminopropanoate

A mixture of *N*-(BOC)-(*S*)-tyrosine ethyl ester (1.79g, 5.80 (mmol) potassium carbonate (0.80g, 5.80mmol) and Intermediate 12 (1.0g, 6.08mmol) in dry DMF (10ml) was stirred at room temperature for 18h, and at 40° for 18h. The DMF was removed *in vacuo* and the residue partitioned between EtOAc (80ml) and 10% aqueous Na₂CO₃ (20ml). The phases were separated and the aqueous layer re-extracted with EtOAc (2 x 20ml). The combined organic extracts were washed with brine (10ml), dried (Na₂SO₄) and evaporated *in vacuo* to afford a new colourless oil. Chromatography (silica; 2.5% MeOH/DCM) afforded reasonably pure N-t-butoxycarbonyl protected title compound (1.81 g, 71%). This material was dissolved in EtOAc (40ml) and HCl gas was bubbled through the stirred solution for 1 min. then the mixture was stirred for an additional 0.5h. The volatiles were removed *in vacuo* affording a yellow solid which was partitioned between EtOAc (80ml) and saturated aqueous NaHCO₃ (20ml). The phases were separated and the aqueous layer re-extracted with EtOAc (2 x 20ml). The combined organic extracts were washed with brine (10ml), dried (Na₂SO₄) and evaporated *in vacuo.* The obtained oil was chromatographed (silica; 5% MeOH/DCM) to afford the title compound as a near colourless oil (0.87g, 62%) δH (CDCl₃) 9.77 (1H, s), 8.75 (1 H, d, J 5.8Hz), 8.10 (1 H, d, J 5.8Hz), 7.58 (1 H, d, J 5.8Hz), 7.29 (2H, d, J 8.4Hz), 7.25 (1 H, d, J 5.9Hz), 7.21 (2H, d, J 8.4Hz), 4.22 (2H, q, J 7/1 Hz), 3.80-3.70 (1 H, obscured m), 3.15 (1 H, dd, J 13.6, 5.1 Hz), 2.88 (1 H, dd, J 13.6, 8.0Hz) 1.30 (3H, t, J. 7.1 Hz) and 0.78 (2H, br s); m/z (ES⁺ 70V) 324 (MH⁺).

### INTERMEDIATE 15

### 4-Acetonyl-3-cyanopyridine

A solution of 4-methyl-3-cyanopyridine (4g, 33.9mmol) and *N*,*N-*dimethylacetamide dimethylacetyl (5.4g, 40.6mmol) in dry DMF (20ml) was stirred at 130° for 7h. The volatiles were removed *in vacuo* to afford a dark oil which solidified on standing. This material was chromatographed (silica; 50% EtOAc/ Hexane - 100% EtOAc) affording the title compound as an off-yellow solid (3.73g, 69%). δH (CDCl₃) 8.87 (1 H, s), 8.74 (1H, d, J 5.2Hz), 7.28 (1 H, d, J 5,2Hz), 4.00 (2H, s) and 2.36 (3H, s); m/z (ES⁺, 70V) 161 (MH⁺).

### INTERMEDIATE 16

### 1-Hydroxy-3-methyl-2,7-naphthyridine hydrochloride

HCl gas was bubbled through a stirred solution of Intermediate 15 (3.73g, 23.3mmol) in glacial acetic acid (40ml) for several minutes. The flask was stoppered and reaction stirred for 18h at ambient temperature. The volatiles were removed *in vacuo* affording a straw-coloured solid. This was twice treated with water (30ml portions) and re-evaporated *in vacuo* to dryness, affording the title compound (contaminated with ~25% unidentified by-product) as a dark straw coloured solid (4.1g). δH (DMSO-d⁶) 12.46 (1H. br s), 9.32 (1H,s), 8.71 (1H, d, J 6.5Hz), 7.98 (1 H, d, J 6.5Hz), 6.67 (1 H,s) and 2.38 (3H, s); m/z (ES⁺, 70V) 161 (MH⁺). Used without further purification.

### INTERMEDIATE 17

### 1-Chloro-3-methyl-2,7-naphthyridine

Intermediate 16 (4.1g) was treated with phosphorus oxychloride (50ml) at 130° for 3h, affording a dark solution. The volatiles were removed in *vacuo* and the obtained dark oil extracted with Et₂O (100ml). Saturated aqueous NaHCO₃ (ice cold; containing 10g additional solid NaHCO₃) was poured (with CARE!) onto the crude product with swirling and ice-bath cooling. After thorough shaking, addition Et₂O (80ml) was added, the mixture re-shaken, and the phases separated. The aqueous layer was re-extracted with Et_{z}O (2 x 80ml) and the combined ethereal extracts washed with brine (20ml), dried (Na₂SO₄) and evaporated *in vacuo* to afford an orange solid (3.6g). Chromatography (silica; 70% EtOAc/Hexane - 100% EtOAc) afforded a more-polar by-product (3-methyl-1H-pyrano[3,4-C]pyridin-1-one, (0.7g) and the title compound as a white solid (2.82g, 79% from intermediate 7) δH (CDCl₃) 9.66 (1H, s), 8.73 (1H, d, J 5.8hz), 7.56 (1 H, d, J 5.8Hz), 7.40 (1 H, s) and 2,69 (3H, s); m/z(ES⁺, 70V) 179 and 181 (MH⁺).

### INTERMEDIATE 18

### Ethyl (S)-3-[4-(3-methyl-2,7-naphthyridin-1-ylamino)phenyl]-2-[N-(tertbutyloxycarbonyl)amino]propanoate hydrochloride

Acetylchloride (55mg, 50µl, 0.70mmol) was added to absolute ethanol (25ml) and stirred for one minute. Intermediate 17 (2.50g, 14.0mmol) and ethyl-(*S*)-3-[4-aminophenyl]-2-{tert-butyloxycarbonyl]propanoate (4.31 g, 14.0mmol) were added and the reaction mixture stirred at 60° for 2.75h. The volatiles were removed *in vacuo* to afford a yellow-orange solid. This was treated with EtOAc (~25ml), warmed, re-cooled and the precipitate collected by filtration, with Et₂O washing, affording the title compound as yellow solid (4.96g, 73%). δH (CDCl₃) 10.44 (1h, br s), 10.33 (1H, br s), 8.60 (1H, d, J 6.5Hz), 8.00 (1H, d, J 6.5Hz), 7.85 (2H, d, J 8.5Hz), 7.28 (1H, d, J 8.0Hz), 7.23 (2H, d, J 8.5Hz), 7.16 (1H,s), 4.19-4.01 (1H, m), 4.08 (2H, q, J 7.0Hz), 2.97 (1 H, dd, J 13.8, 5.4 Hz), 2.86 (1 H, dd, J 13.8, 10.0Hz), 2.50 (3H,s), 1.34 (9H, s) and 1.15 (3H, t, J 7.0Hz); m/z (ES⁺, 70V) 451 (MH⁺).

### INTERMEDIATE 19

### Ethyl-(S)-3-[4-(3-methyl-2,7-naphthyridin-1-ylamino)phenyl]-2-aminopropanoate

HCl gas was bubbled through a stirred solution of Intermediate 18 (4.95g, 10.2mmol) for 1-2min. After 30min stirring at ambient temperature the volatiles were removed *in vacuo* affording a yellow powder. This was treatd with saturated aqueous NaHCO₃ (30ml) then extracted with EtOAc (100ml, and 3 x 50ml). The combined organic extracts were washed with brine (10ml), dried (Na₂SO₄) and evaporated *in vacuo* affording the title compound as a yellow solid (3.56, 100%). δH (CDCl₃) 9.25 (1H, s), 8.50 (1 H, d, J 5.6Hz), 7.66 (2H, d, J 8.4Hz), 7.35 (1H d, J 5.6Hz), 7.34 (1 H, masked s), 7.14 (2H, d, J 8.4Hz), 6.81 (1 H, s), 4.12 (2H, q, J 7.2Hz), 3.65 91 H, dd, J 7.8, 5.2Hz), 3.02 (1H, dd, J 13.7, 5.2Hz), 2.80 (1H, dd, J 13.7, 7.8Hz), 2.48 (3H, s), 1.56 (2H, br s) and 1.21 (3H, t, J 7.2Hz); m/z (ES⁺, 70V) 351 (MH⁺).

### INTERMEDIATE 20

### Ethyl (S)-3-[4-(3-methyl-2,7-naphthyridin-1yloxy)phenyl]-2-(N-t-butyloxycarbonylamono)-propanoate

A mixture of *N*-*t*butyloxycarbonyl-(*S*)-tyrosine ethyl ester (14.5g, 46.9mmol), caesium carbonate (14.05g,43.1mmol) and Intermediate 17 (7.0g, 39.2mmol) in dry DMF (60ml) was stirred at room temperature for 48h. The reaction was diluted with Et₂O (150ml) and filtered off. The filtrate was evaporated under high vacuum and the residue was chromatographed (SiO₂; 40% - 60% EtOAc/Hexane) which afforded the title compound as a viscous, straw-coloured oil (16.2g, 77%) δH (CDCl₃) 9.56 (1H, s), 8.58 (1H, d, J 5.8Hz), 7.39 (1 H, d, J 5.8Hz), 7.15-7.10 (4H, m), 7.00 (1H, s), 4.99-4.91 (1H,m), 4.54-4.46 (1H, m), 4.09 (2H, q, J 7.1Hz), 3.10-2.99 (2H,m), 2.36 (3H, s), 1.34 (9H, s) and 1.15 (3H, t, J 7.1 Hz); m/z (ES⁺, 70V) 452 (MH⁺).

### INTERMEDIATE 21

### Ethyl (S)-3-[4-(3-methyl)-2,7-naphthyridin-1-yloxy)phenyl]-2-aminopropanoate

HCl gas was bubbled through a stirred solution of Intermediate 20 (16g) in EtOAc (300ml) until a persistent fine white precipitate formed (~2minutes). After stirring for 0.5h the volatiles were removed *in vacuo.* The obtained solid was partitioned between EtOAc (250ml) and saturated aqueous NaHCO₃ (80ml plus 5g solid NaHCO₃). The phases were separated and the aqueous layer re-extracted with EtOAc (5 x 50ml). The combined organic extracts were washed with brine (10ml), dried (Na₂SO₄) and evaporated *in vacuo* to afford an oil Chromatography (SiO₂; 100% EtOAC - 10% EtOH/EtOAc) afforded the title compound as a viscous oil (11.1 g, 89%). δH (CDCl₃) 9.71 (1H,s), 8.70 (1H, d, J 5.Hz), 7.50 (1H, d, J 5.8Hz), 7.31-7.28 (4H,m), 7.11 (1H, s), 4.23 (2H, q, J 7.1Hz), 3.79-3.72 (1 H, m), 3.14 (1 H, dd, J 14.1, 5.4Hz), 2.94 (1 H, dd, J 14.1, 7.8Hz), 2.47 (3H, s), 1.75-1.50 (2H, br s) and 1.30 (3H, t, J 7.1 Hz); m/z (ES⁺, 70V) 352 (MH⁺).

### INTERMEDIATE 22

### Methyl (2S)-2-[(tert-butoxycarbonyl)amino]-3-(4-{[trifluoromethylsulfonyl]oxy}phenyl)propanoate

Triflic anhydride (5.05ml, 30mmol) was added to a mixture of N-BOC tyrosine methyl ester (7.38g, 25mmol) and pyridine (10ml, 125mmol) in DCM (40ml) at 0°. After 45min at 0° water (80ml) and DCM (100ml) were added. The organic phase was washed with NaOH aq. (0.5M, 60ml), water (60ml), citric acid (10%, 2 x 80ml) and water (60ml), dried (Na₂SO₄) and concentrated *in vacuo* to give the title compound as a yellow oil which solidified on standing (10.6g). δH (CDCl₃) 7.26-7.18 (4H, m), 5.05 (1 H, v br d), 4.59 (1 H, v br q), 3.70 (3H, s), 3.16 (1 H, dd, J 13.7, 5.7Hz), 3.02 (1 H, dd, J 13.8, 6.5Hz), 1.40 (9H, s); m/z (ES⁺, 70V) 450 (M⁺+ Na).

### INTERMEDIATE 23

### Methyl (2S)-[(tert-butoxycarbonyl)amino]-3-(4-[2',6'-dimethoxy] biphenylyl)propanoate

A mixture of the Intermediate 22 (4.27g, 10mmol), 2,6-dimethoxybenzene boronic acid (4.55g, 25mmol), potassium carbonate (6.9g, 50mmol) tetrakis(triphenylphosphine)palladium(O) (2.31 g) in DME (45ml) and water (5ml) was heated at 80° overnight. The mixture was diluted with EtOAc, washed with dilute HCl, NaHCO₃ (aq.), water and brine, dried (Na₂SO₄) and concentrated *in vacuo.* Column chromatography (SiO₂; EtOAc/hexane, 20:80 - 30:70) gave the title compound (2.27g). δH (DMSO-d⁶) 7.33 (1H. d, J 8.2Hz), 7.27 (1 H, t, J 8.3Hz), 7.20 (2H, d, J 8.1 Hz), 7.10 (2H, d, J 8.0Hz), 6.71 (2H, d, J 8.4Hz), 4.2 (1 H, m), 3.63 (9H, s), 3.01 (1 H, dd, J 13.9, 4.5Hz), 2.84 (1 H, dd, J 13.7, 10.3Hz), 1.34 (9H, s); m/z (ES⁺, 70V) 438 (M⁺ + Na).

### INTERMEDIATE 24

### Methyl (2S)-2-amino-3-(4-[2',6'-dimethoxy]biphenylyl)propionate hydochloride

Anhydrous HCl was bubbled through a solution of Intermediate 23 (1.30g, 3.13mmol) in EtOAc (30ml) for a few seconds. The mixture was stirred at room temperature for 1h. Some solvent was removed *in vacuo* until material began to precipitate. The precipitate was filtered off and dried to give the title compound as pale yellow crystals (888mg, 81%). δH (DMSO-d⁶) 8.7 (2H, br s), 7.28 (1H, t, J 8.4Hz), 7.21 (2H, d, J 8.4Hz), 7.17 (2H, d, J 8.3Hz), 6.73 (2H, d, J 8.4Hz), 4.30 (1H, t, J 6.6Hz), 3.69 (3H, s), 3.64 (6H, s), 3.18 (1 H, dd, J 14.1, 6.2Hz), 3.10 (1 H, dd, J 14.1, 7.1 Hz); m/z (ES⁺, 70V) 316 (MH⁺).

### INTERMEDIATE 25

### Ethyl N-(diphenylmethylene)-2-amino-3-(5-benzenesulphonyloxypyrid-2-yl)propionate

A solution of ethyl *N*-(diphenylmethylene)glycinate (1.71g, 6.40mmol) in dry THF (10ml) was added to a stirrred solution of LDA (2M in heptane/TNF/ethytbenzene, 320ml, 6.40mmol) in dry THF 910ml) at -70° under nitrogen. After stirring at this temperture for 0.75h, a solution of 5-benzenesulphonyloxy-2-bromomethylpyridine (2.00g, 6.01 mmol); [prepared as described by Myers et al, J.O.C. 61, 813 (1996)] in dry THF (10ml) was added. The reaction mixture was stirred at -70° for 1h then at room temperature for 18h. The reaction was quenched with water 910ml) then partitioned between EtOAc (70ml) and bring (30ml). The phases were separated and the aqueous phase re-extracted with EtOAc (2 x 40ml). The combined organic extracts were washed with brine (10ml), dried (Na₂SO₄) and evaporated *in vacuo* to afford the crude product as a dark oil. Chromatography (silica: 60-75% Et₂O/Hexane) afforded the title compound as a tan-coloured solid (2.25g, 72%). δH (CDCl₃) 8.02 (1 H, d, J 2.8Hz), 7.72 (2H, d, J 8.0hz), 7.59 (1H, t, J 8.0hz), 7.50 (2H, dd, J 8.4Hz), 7.40-7.27 (8H, m), 7.19 (1H. dd, J 8.5, 2.8Hz), 7.11 (1 H, d, J 8.5Hz), 6.67 (2H, br d, J 8.0Hz), 4.50 (1H, dd, J 9.0, 4.6Hz), 4.25-4.10 (2H, m), 3.50-3.33 (2H, m) and 1.24 (3H, t, J 7.2Hz), m/Z (ES⁺, 70V) 515 (MH⁺).

### INTERMEDIATE 26

### Ethyl 2-amino-3-(5-benzenesulphonyloxypyrid-2-yl)propionate

A solution of Intermediate 25 (1.9g, 3.7mmol) in 10% aqueous HCl (5ml) and ethanol (120ml) was stirred at room temperature for 1.5h. Most of the solvent was removed *in vacuo* and the residue partitioned between half-saturated aqueous NaHCO₃ (50ml) and EtOAc (80ml). The phases were separated and the aqueous layer re-extracted with EtOAc (4 x 40ml). The combined organic extracts were dried (Na₂SO₄) and evaporated *in vacuo.* The obtained yellow oil was chromatographed (silica; EtOAc) to afford the title compound as a coourless oil (1.15g, 78%). δH (CDCl₃) 8.04 (1H, d, J 2.8Hz), 7.80 (2H, d, J. 8.OHz), 7.65 (1H, t, J 8 0Hz), 7.51 (2H, t, J 8.0Hz), 7.31 (1 H, dd, J 8.5, 2.8Hz), 7.12 (1 H, d, J. 8.5Hz), 4.10 (2H, q, J 7.1 Hz), 3.86 (1 H, dd, J. 7.9, 4.9Hz), 3.19 (1 H, dd, J. 14.4, 4.9Hz), 2.99 (1 H, dd, J 14.4, 7.9Hz), 1.66 (2H, br s) and 1.17 (3H, t, J 7.1Hz). m/z (ES⁺, 70V) 351 (MH⁺).

### INTERMEDIATE 27

### Ethyl-2-(N-t-butyloxycarbonylamino)-3-(5-hydroxypyrid-2-yl)propionate

A solution of Intermediate 26 (3.50g, 10mmol) and LiOH/H₂O (920mg, 22mmol) in dioxan (20ml) and water (30ml) was stirred at room temperature for 5h. The volatiles were removed *in vavuo* and the residue treated with ethanol (50ml). HCl gas was bubbled through the mixture for a few minutes and the reaction mixture heated at 50° overnight. The volatiles were removed *in vacuo* and the residue treated with a mixture of di-*tert*-butyl dicarbonate (1.53g, 7.0mmol) and NaHCO₃ (3.36g, 40mmol) in THF (10ml) and water (20ml). After stirring at room temperature for 2h, additional di-*tert*-butyldicarbonate (150mg, 0.7mmol) was added, the reaction mixture stirred for a further 2h. The organic volatiles were removed *in vacuo* and aqueous residue was extracted with EtOAc (2 x 75ml). The combined organic extracts were washed with brine (5ml), dried (MgSO₄) and evaporated *in vacuo.* The crude product was chromatographed (silica, 50% EtOAc/hexane) to afford the title compound as a white solid (1.81 g, 58%), δ H (CDCl₃; 8:3 ratio of rotamers) 8.14 (0.73H, br s), 8.01 (0.27H, br s), 7.18-7.08 (1 H, m), 7.08-6.90 (1 H, m), 6.24 (0.27H, br dm J 8.5Hz), 5.77 (0.73H, br d J 8.5Hz), 4.64 (0.73H, m), 4.43 (0.27H, m), 4.17 (2H, q, J 7.1 Hz), 3.27-3.14 (2H, m), 1.41 (9H, s and 1.23 (3H, t, J 7.1 Hz). m/z (ES⁺, 70V) 311 (MH⁺).

### INTERMEDIATE 28

### Ethyl-2-(N-t-butyloxycarbonylamino)-3-[5-(3-methyl-2,7-naphthyridin-1-yloxy)-pyrid-2-yl]propionate

A mixture of intermediate 27 (1.814g, 5.85mmol), Intermediate 17 (0.87g, 4.87mmol) and Cs₂CO₃ (1.73g, 5.31 mmol) in dry DMF (12ml) was stirred at 40° overnight. The inorganics were removed by filtration with DCM washed and the filtrate evaporated *in vacuo* to afford a dark oil. Chromatography (silica; 50% EtOAc/Hexane- 100% EtOAc) afforded the title compound as a white foam (1.69g, 77%). δH (CDCl₃) 9.71 (1H, s), 8.72 (1 H, d, J 5.8Hz), 8.56 (1H, d, J 2.7Hz), 7.62 (1H, dd, J 8.4, 2.7Hz), 7.53 (1 H, dd, J 5.8, 0.8Hz), 7.26 (1 H, d, J 8.4Hz), 7.15 (1 H, s), 5.86 (1 H, br d, J 9.2Hz), 4.71 (1H, m), 4.81 (2H, m), 3.45-3.29 (2H, m), 1.46 (9H, s) and 1.27 (3H, t, J 7.2Hz); m/z (ES⁺, 70V) 453 (MH⁺).

### INTERMEDIATE 29

### Ethyl-2-amino-3-[5-(3-methyl-2,7-naphthyridin-1-yloxy)pyrid-2-yl]propionate

Intermediate 28 was dissolved in EtOAc and HCl gas bubbled through for 10min. Solution neutralised with aqueous NaHCO₃ and extracted into EtOAc to quantitatively give the title compound used crude without purification.

### INTERMEDIATE 30

### 1,1-Dioxo-tetrahydrothiophen-3-one

Tetrahydrothiophen-3-one (2g, 19.6mmol) in CH₂Cl₂ (100ml) at 0° was treated with 3-chloroperoxybenzoic acid (9g) in CH₂Cl₂ (10ml). After 3h the reaction was complete and the solid removed by filtration and the filtrate concentrated *in vacuo.* The title compound was purified by chromatography (SiO₂; EtOAc/hexane 4:1) to give the title compound as a white solid (340mg; 2.5mmol; 13%). δH (CDCl₃) 3.72 (2H, s), 3.60 (2H, t, J 7.8Hz), 3.10 (2H, t, J 7.8Hz). m/z (ES⁺, 70V) 135 (MH⁺).

### EXAMPLE 1

### (2S)-Ethyl-3-{4-(3,5-dichloropyrid-4-ylcarboxamido)phenyl]-2-[(3-oxo-1-cyclopentenyl)amino]propionate

A solution of (*S*)-ethyl-3-[4-(3,5-dichloropyrid-4-yl carboxamido)phenyl]-2-aminopropionate (prepared from 3,5-dichloroisonicotinoyl chloride and N-BOC-*L*-4 aminophenylalanine ethylester) (128mg, 0.34mmol), and 1,3-cyclopentanedione (49mg, 0.50mmol) in chloroform (5ml) was treated with 4A molecular sieves (~1g) and heated to reflux for 24h. The sieves were removed by filtration, the solution concentrated *in vacuo* and the residue purified by chromatography (SiO₂, gradient elution 98:2-96:4 DCM/MeOH) to give the title compound as a white solid (120mg, 77%). δH (CD₃OD) 8.61 (2H, s), 7.59 (2H, d, J 8.5Hz), 7.25 (2H, d, J 8.5Hz), 4.98 (1 H, s), 4.33 (1H, t), 4.16 (2H, q, J 7.1 Hz), 3.29-3.03 (2H, m), 2.63 (2H, m), 2.32 (2H, t, J 5.1 Hz), 1.22 (3H, t, J 7.1 Hz). m/z (ESI, 70V) 462 (MH⁺).

In a similar manner to Example 1 were prepared Examples 2 to 4.

### EXAMPLE 2

### (2S)-Ethyl-3-[4-(3,5-dichloropyrid-4-ylcarboxamido)phenyl]-2-[(3-oxo-1-cyclohexenyl)amino]propionate

Prepared from 1,3-cyclohexanedione δH (CD₃OD) 8.62 (2H, s), 7.59 (2H, d, J 8.5Hz), 7.25 (2H, d, J 8.5Hz), 5.04 (1 H, s), 4.35 (1 H, t), 4.14 (2H, q, J 7.1 Hz), 3.21-3.03 (2H, m), 2.43 (2H, m), 2.23 (2H, t, J 6.3Hz), 1.89 (2H, m), 1.19 (3H, t, J 7.1Hz). m/z (ESI, 70V) 476 (MH⁺).

### EXAMPLE 3

### (2S)-Ethyl-3-[4-(3,5-dochloropyrid-4-ylcarboxamido)phenyl]-2-[(2-ethyl-3-oxo-1-cyclopentenyl)amino]propionate

Prepared from 2-ethyl-1,3-cyclopentanedione. Heated at reflux in 1,2-dichloro-ethane for 5 days. δH (CD₃OD) 8.62 (2H, s), 7.59 (2H, d, J 8.4Hz), 7.29 (2H, d, J 8.4Hz), 4.48 (1 H, m), 4.22 (2H, q, J 7.1Hz), 3.32 (1H, dd, J 13.7, 4.6Hz), 3.06 (1H, dd, J 13.7, 9.8Hz), 2.5-2.0 (6H, m), 1.27 (3H, t, J 7.1 Hz), 0.91 (3H, t, J 7.4Hz). m/z. (ESI, 70V) 490 (MH⁺).

### EXAMPLE 4

### (2S)-Ethyl-3-[4-(3,5-dichloropyrid-4-ylcarboxamido)phenyl]-2-(5,5-dimethyl-3-oxo-1-cyclohexenyl)amino]propionate

Using 5,5-dimethyl-1,3-cyclohexanedione. Heated at reflux in 1,2-dichloroethane for 5 days (SiO₂, gradient 75:25 - 100:0 EtOAc/hexane). δH (CD₃OD 8.64 (2H, s), 7.58 (2H, d, J 8.5Hz), 7.26 (2H, d, J 8.5Hz), 5.03 (1 H, s), 4.36 (1 H, m), 4.16 (2H, q, J 7.1HZ), 3.22 (1 H, m), 3.05 (1H, m), 2.29-2.11 (4H, m), 1.23 (3H, t, J 7.1Hz), 1.03 (3H, s), 0.97 (3H, s). m/z (ESI, 70V) 504 (MH⁺).

### EXAMPLE 5

### (2S)-Ethyl-3-[4-(2,6-naphthyridin-1-yl)amino)phenyl]-2-[(2-methyl-3-oxo-1-cyrclopentenyl)amino]propionate

A solution of (*S*)-ethyl-3-(2,6-naphthyrid-1-yl) amino)phenyl]-2-amino propionate (prepared from 1-chloro-2,6-naphthyridine and N-BOC-L-4-aminophenylalanine ethyl ester as described in International Patent Application WO 00/73260) (512mg, 1.52mmol) and 2-methyl-1,3-cyclopentanedione (179mg, 1.60mmol) in 1,2-dichloroethane (20ml) was treated with 4A molecular sieves (0.5g) and heated at 90°C for 3 days. The solution was filtered, concentrated in in *vacuo* and the residue purified by chromatography (SiO₂, 98.2: 96.4 DCM/MeOH) to give the title compound as a yellow solid (388mg, 59%). δH (CD₃OD) 9.13 (1 H, s), 8.56 (1 H, d, J 6.0Hz), 8.22 (1 H, dt, J 6.0, 0.9Hz), 8.08 (1 H, d, J 5.8Hz), 7.66 (2H, d, J 8.6Hz),7.26 (2H, d, J 8.6Hz), 7.24 (1 H, d, J 5.9Hz), 4.50 (1 H, m), 4.24 (2H, q, J 7.1Hz), 3.30 (1 H, m), 3.03 (1 H, dd, J 13.7, 9.7Hz), 2.44 (1 H, m), 2.30-2.05 (3H, m), 1.60 (3H, s), 1.29 (3H, t, J 7.1 Hz). m/z (ESI, 70V) 431 (MH⁺.

In a similar manner to Example 5 from (*S*)-ethyl-3-[4-[(2,6-naphthyridin-1-yl)amino]phenyl]-2-aminopropionate and the appropriate dione were prepared Examples 6-16.

### EXAMPLE 6

### (2S)-Ethyl-3-[4-(2,6-naphthyridin-1-yl)amino)phenyl]-2-[(2-ethyl-3-oxo-1-cyclopentenyl)amino]propionate

Prepared from 2-ethyl-1,3-cyclopentanedione. δH (CD₃OD) 9.13 (1 H, s), 8.57 (1H, d, J 5.9Hz), 8.22 (1H, d, J 6.0Hz), 8.08 (1H, d, J 5.8Hz), 7.67 (22H, d, J 8.5Hz), 7.26 (2H, d, J 8.5Hz), 7.25 (1 H, d, J 5.0Hz), 4.48 (1 H, m) 4.25 (2H, q, J 7.1 Hz) 3.31 (1H, m) 3.05 (1h, m), 2.48 (1H, m), 2.25-2.05 (5H, m), 1.30 (3H, t, J 7.1 Hz), 0.93 (3H, t, J 6.5Hz). m/z. (ESI, 70V) 445 (MH⁺).

### EXAMPLE 7

### (2S)-Ethyl-3-[4-(2,6-naphthyridin-1-yl)amino)phenyl]-2-[(2-allyl-3-oxo-1-cyclohexenyl)amino]propionate

Prepared from 2-allyl-1,3-cyclohexanedione. δH (CDl₃) 9.16 (1H, s), 8.62 (1H, d, J 5.9Hz), 8.20 (1 H, d, J 5.6Hz), 7.88 (1 H, d, J 5.8Hz), 7.73 (2H, d, J 8.3Hz), 7.18 (1H, d, J 5.8Hz), 7.12 (2H, d, J 8.3Hz), 5.68 (1H, m), 4.98 (2H, m), 4.36 (1H, m), 4.23 (2H, q, J 7.1 Hz), 3.25-2.95 (4H, m), 2.40-1.70 (6H, 3xm), 1.30 (3H, t, J 7.1 Hz). m/z (ESI, 70V) 471 (MH⁺).

### EXAMPLE 8

### (2S)-Ethyl-3-[4-(2,6-naphthyridin-1-yl)amino)phenyl]-2-[(5-phenyl-3-oxo-1-cyclohexenyl)amino]propionate

Prepared from 5-phenyl-1,3-cyclohexanedione. δH (CDl₃) 9.19 (1H, s), 8.70 (1 H, 2xd J 5.8Hz), 8.16-8.15 (1 H, 2x d, .J 5.1 Hz), 8.01 (1 H, br), 7.66-7.61 (2H, 2 x d, J 8.4Hz), 7.35-7.05 (9H, m), 5.20 (1 H, br), 4.24-4.22 (2H, 2 x q, J 7.1 Hz), 3.34 (1H, m), 3.25 (1 H, m), 3.05 (1 H, m), 2.80-2.40 (4H, m), 1.32-1.30 (3H, 2 x t J 7.1Hz). m/z (ESI, 70V) 507 (MH⁺).

### EXAMPLE 9

### (2S)-Ethyl-3-[4-(2,6-naphthyridin-1-yl)amino)phenyl]-2-[(5-propyl-3-oxo-1-cyclohexenynamino]propionate

Prepared from 5-propyl-1,3-cyclohexanedione 4Å sieves replaced with Na₂SO₄ (~1g) and acetic acid (1 drop). δ H (CD₃OD 9.13 (1 H, s), 8.57 (1 H, d, J 6.0Hz), 8.23 (1 H, d, J 6.0Hz), 8.09 (1 H, d, J 5.8Hz), 7.68 (2H, d, J 8.4Hz), 7.24 (3H, m), 4.33 (1 H, m), 4.16 (2H, m), 3.18 (1 H, m), 3.05 (1 H, m), 2.60-1.90 (3H, m), 1.35 (4H, br), 1.23-1.22 (3H, 2 x t, J 7.1 Hz), 0.91 (3H, t). m/z (ESI, 70V) 473 (MH⁺).

### EXAMPLE 10

### (2S)-Ethyl-3-[4-(2,6-naphthyridin-1-yl)amino)phenyl]-2-[(2-benzyl-3-oxo-1-cyclopentenyl)amino]propionate

Prepared from Intermediate 1. δ H (CD₃OD 9.15 (1 H, s), 8.59 (1 H, d, J 6.0Hz), 8.24 (1 H, d, J 6.0Hz), 8.12 (1 H, d, J 5.8Hz), 7.62 (2H, d, J 8.4Hz), 7.30-7.00 (8H, m), 4.48 (1 H, m), 4.21 (2H, q, J 7.0HZ), 3.52 (2H, m), 3.25 (1H, m), 2.95 (1 H, m), 2.53 (1 H, m), 2.30-2.05 (3H, m), 1.27 (3H, t, J 7.0Hz). m/z (ESI, 70V) 507 (MH⁺).

### EXAMPLE 11

### (2S)-Ethyl-3-[4-(2,6-naphthyridin-1-yl)amino)phenyl]-2-[(2-allyl)-3-oxo-1-cyclopentenyl)amino]propionate

Prepared from Intermediate 2. δH (CD₃OD) 9.14 (1 H, s), 8.58 (1 H, d, J 6.0Hz), 8.23 (1 H, d, J 5.9Hz), 8.09 (1 H, d, J 5.8Hz), 7.67 (2H, d, J 8.5Hz), 7.25 (1 H, d, J 5.9Hz), 7.23 (2H, d, J 8.5Hz), 5.74 (1 H, m), 5.00 (2H, m), 4.52 (1 H, m), 4.24 (2H, q, J 7.1Hz), 3.30 (1 H, m), 3.02 (1 H, dd, J. 13.8, 9.4Hz), 2.89 (2H, d, J 5.9Hz), 2.52 (1 H, m), 2.30-2.05 (3H, m), 1.29 (3H, t, J 7.1 Hz). m/z (ESI, 70V) 457 (MH⁺).

### EXAMPLE 12

### (2S)-Ethyl-3-[4-(2,6-naphthyridin-1-yl)amino)phenyl]-2-[(2-butyl-3-oxo-1-cyclopentenyl)amino]propionate

Prepared from Intermediate 3 4Å sieves replaced with Na₂SO₄ (~1g) and acetic acid (1 drop). δH (CD₃OD) 9.14 (1 H, s), 8.57 (1 H, d, J 6.0Hz), 8.23 (1 H, d, J 6.0Hz), 8.09 (1 H, d, J 5.8Hz), 7.68 (2H, d, J 8.5Hz), 7.26 (2H, d, J 8.5Hz), 7.25 (1 H,d), 4.50 (1 H, dd), 4.25(2H, q, J 7.1 Hz), 3.31 (1 H, m), 3.05 (1H, dd), 2.45 (1 H, m), 2.25-2.05 (5H, m), 1.30 (7H, m) 0.89 (3H, t). m/z (ESI, 70V) 473 (MH⁺).

### EXAMPLE 13

### (2S)-Ethyl-3-[4-(2,6-naphthyridin-1-yl)amino)phenyl]-2-[(2-phenyl-3-oxo-1-cyclopentenyl)amino]propionate

Prepared from Intermediate 4. δH (CD₃OD) 9.14 (1H, d, J 0.9Hz), 8.58 (1H, d, J 6.0Hz), 8.24 (1H, d, J 6.0Hz), 8.11 (1 H, d, J 5.8Hz), 7.68 (2H, m), 7.39 (2H, m), 7.26 (5H, m), 4.52 (1 H, m), 4.24 (2H, q), 3.30 (1 H, m), 3.07 (1 H, m), 2.60 (1 H, m), 2.45-2.20 (3H, m), 1.30 (3H, t). m/z (ESI, 70V) 493 (MH⁺).

### EXAMPLE 14

### (2S)-Ethyl-3-[4-(2,6-naphthyridin-1-yl)amino)phenyl]-2-[(2-propyl-3 oxo-1-cyclopentenyl)amino]propionate

Prepared from Intermediate 5. δH (CD₃OD) 9.10 (1 H, d, J 0.8Hz), 8.55 (1 H, d, J 6.0Hz), 8.20 (1 H, d, J 6.OHz), 8.07 (1 H, d, J 5.8Hz), 7.66 (2H, d, J 8.5Hz), 7.24 (2H, d, J 8.5Hz), 7.21 (1 H, d), 4.47 (1 H, dd, J 9.8, 4.6Hz), 4.24 (2H, 1, J 7.1HZ), 3.29 (1 H, m), 3.04 (1H, dd, J 13.8, 9.8Hz), 2.45 (1 H, m), 2.25-2.07 (5H, m), 1.36 (2H, m, J 7.5Hz), 1.29 (3H, t, J 7.1 Hz), 0.87 (3H, t, J 7.3Hz). m/z (ESI, 70V), 459 (MH⁺).

### EXAMPLE 15

### (2S)-Ethyl-3-[4-(2,6-naphthyridin-1-yl)amino)phenyl]-2-[(2-isopropyl-3-oxo-1-cyclopentenyl)amino]propionate

Prepared from Intermediate 6 4Å sieves replaced with Na₂SO₄ (~1g) and acetic acid (1 drop) (240mg, 41%). δH (CD₃OD) 9.13 (1H, br), 8.55 (1 H, br), 8.20 (1 H, d), 8.09 (1H, d, J 5.8Hz), 7.68 (2H, d, J 8.6Hz), 7.24 (3H, d), 4.47 (1 H, dd, J 9.3, 4.6Hz), 4.25 (2H, q, J 7.1 Hz), 3.30 (1 H, m), 3.07 (1 H, dd, J 13.7, 9.3Hz), 2.70 (1 H, sept, J 7.1 Hz), 2.40 (1H, m), 2.25-2.05 (3H, m), 1.30 (3H, t, J 7.1HZ), 1.16 (3H, d, J 7.1Hz), 1.15 (3H, d, J 7.1Hz). m/z (ESI, 70V) 459 (MH⁺).

### EXAMPLE 16

### (2S)-Ethyl-3-[4-(2,6-napthyridin-1-yl)amino)phenyl]-2-[(2-isobutyl-3-oxo-1-cyclopentenyl)amino]propionate

Prepared from Intermediate 7, 4Å sieves replaced with Na₂SO₄ sieves (~1g) and acetic acid (1 drop) (50mg, 20%). δ H (CD₃OD) 9.12 (1H, d, J 0.8Hz), 8.57 (1 H, d, J 6.0Hz), 8.22 (1 H, d, J 6.0Hz), 8.08 (1H, d, J 5.8Hz), 7.67 (2H, d, J 8.5Hz), 7.24 (3H, d), 4.50 (1 H, dd, J 9.6, 4.5Hz), 4.24 (2H, q, J 7.1Hz), 3.30 (1 H, m), 3.05 (1H, dd, J 13.8, 9.7Hz), 2.43 (1H, m), 2.25-2.05 (3H, m), 1.98 (2H, d, J 14.6Hz), 1.74 (1 H, sept), 1.29 (3H, t, J 7.1 Hz), 0.84 (3H, d, J 6.6Hz), 0.83 (3H, d, J 6.6Hz). m/z (ESI, 70V) 473 (MH⁺).

### EXAMPLE 17

### (2S)-Ethyl-3-[4-(3-ethyl-isoquinolin-1-ylamino)phenyl]-2-[(2-ethyl-3-oxo-1-cyclopentenyl)amino]propionate

A solution of (*S*)-ethyl-3-[4-(3-ethyl-isoquinol-1-yl amino)phenyl]-2-aminopropionate [prepared from 1-chloro,3-ethyl-isoquinoline (from reaction of o-toluic acid with propionitrile and subsequent treatment with phosphorous oxychloride) and *N*-BOC-*L*-4-aminophenylalanine ethylester] (750mg, 2.07mmol) and 2-ethyl-1,3-cyclopentanedione (273mg, 2.17mmol) in 1,2-dichloroethane (10ml) was treated with 4A sieves (~1g) and heated at 90° for 3 days. The solution was filtered, concentrated *in vacuo* and the residue purified by chromatorgaphy (SiO₂, 100:1 DCM/MeOH) to give the title compound as a brown solid (511mg, 52%). δH (d⁶-DMSO) 9.05 (1 H, s), 8.46 (1 H, d, J 7.0Hz), 7.91 (2H, d, J 8.6Hz), 7.72 (1 H, d, J 7.0Hz), 7.64 (1 H, t, J 7.0Hz), 7.51 (1 H, t, J 7.0Hz), 7.23 (2H, d, J 8.6Hz), 6.99 (1 H, s), 4.32 (1 H, m), 4.15 (2H, q, J 7.1Hz), 3.15 (1 H, dd, J 13.5, 3.5Hz), 3.01 (1 H, dd, J 13.5, 9.6Hz), 2.69 (2H, 1, J 7.5Hz), 2.29 (1 H, m), 2.18 (1H. m), 2.06 (4H, m), 1.28 (3H, t, J 7.5Hz), 0.91 (3H, t, J 7.4Hz). m/z (ESI, 70V) 472 (MH⁺).

### EXAMPLE 18

### (2S)-3-[4-(3,5-Dichloropyrid-4-yl carboxamido)phenyl]-2-[(3-oxo-1-cyclopentenyl)amino]propionic acid

A solution of Example 1 (170mg, 0.37mmol) in THF (2ml) and water (2ml) was treated with LiOH.H₂O (23mg, 0.55mmol) and stirred for 30 mins. The mixture was concentrated *in vacuo* and purified by chromatography (SiO₂, DCM:MeOH:AcOH:H₂O 200:20:3:2) to give the title compound as a white solid (134mg, 84%). δH (d⁶-DMSO) 10.72 (1 H, br s), 8.65 (2H, s), 7.55 (1 H, br s), 7.42 (2H, d, J 8.4Hz), 7.12 (2H, d, J 8.4Hz), 4.64 (1 H, s), 3.92 (1 H, br s), 2.94-2.81 (2H, m), 2.34 (2H, m), 1.97 (2H, t, J 5.5Hz). m/z (ESI, 70V) 434 (MH⁺).

In a similar manner to Example 18 were prepared Examples 19-34:

### EXAMPLE 19

### (2S)-3-[4-(3,5-Dichloropyrid-4-yl carboxamido)phenyl]-2-[(3-oxo-1-cyclohexenyl)amino]propionic acid

Prepared from Example 2. δH (d⁶-DMSO) 10.95 (1 H, br s), 8.90 (2H, s), 7.64 (2H, d, J 8.3Hz), 7.31 (2H, d, J 8.3Hz), 7.0 (1 H, br s), 4.90 (1 H, s), 3.97 (1 H, br s), 3.15 (1 H, dd, J 13.6, 5.5Hz), 3.06 (1 H, dd, J 13.6, 6.8Hz), 2.43-2.17 (4H, m), 1.89 (2H, m). m/z (ESI, 70V) 448 (MH⁺).

### EXAMPLE 20

### (2S)-3-[4-(3,5-Dichloropyrid-4-yl carboxamido)phenyl]-2-[(2-ethlyl-3-oxo-1-cyrclopentenyl)amino]propionic acid

Prepared from Example 3. δH (d⁶-DMSO) 10.86 (1H, s), 8.79 (2H, s), 7.55 (2H, d, J 8.3Hz), 7.31 (2H, d, J 8.3Hz), 7.08 (1 H, d, J 9.1 Hz), 4.26 (1H, m), 3.17 (1H, dd, J 13.5, 4.2Hz), 3.01 (1H, dd, J 13.5, 9.9Hz), 2.31 (1H, dd, J 13.1, 5.0Hz), 2.10-1.90 (5H, m), 0.81 (3H, t, J 7.4Hz). m/z (ESI, 70V) 462 (MH⁺).

### EXAMPLE 21

### (2S)-3-[4-(3,5-Dichloropyrid-4-ylcarboxamido)phenyl]-2-[(5,5-dimethyl-3-oxo-1cyclohexenyl)amino] proponic acid

Prepared from Example 4. δH (d⁶-DMSO) 10.48 (1H, s), 8.70 (2H, s), 7.55 (2H, d, J 7.7Hz), 7.26 (2H, d, J 7.7Hz), 4.87 (1 H, s), 4.12 (1 H, s), 3.13 (1 H, dd, J 13.9, 5.8Hz), 3.01 (1H, dd, J 13.9, 7.6Hz), 2.21 (2H, m), 1.97 (2H, s), 0.99 (3H, s), 0.96 (3H, s). m/z (ESI, 70V) 476 (MH⁺).

### EXAMPLE 22

### (2S)-3-[4-(2,6-Naphthyridin-1-yl)amino)phenyl]-2-[(2-methyl-3-oxo-1-cyclopentenyl)amino]propionic acid

Prepared from Example 5. δH (d⁶-DMSO) 9.28 (1 H, s), 9.21 (1 H, s), 8.66 (1H, d, J 5.9Hz), 8.40 (1H, dd, J 6.0, 0.8Hz), 8.13 (1H, d, J 5.7Hz), 7.71 (2H, d, J 8.5Hz), 7.25 (1H, d, J 5.7Hz), 7.15 (2H, d, J 8.5Hz), 6.56 (1H, d, J 8.1 Hz), 3.78 (1 H, m), 3.11 (1H, d), 2.85 (1H, dd, J 13.3, 8.0Hz), 2.31 (1H, m), 2.00-1.75 (3H, m), 1.44 (3H, s). m/z (ESI, 70V) 403 (MH⁺).

### EXAMPLE 23

### (2S)-3-[4-(2,6-Naphthyridin-1-yl)amino)phenyl]-2-[(2-ethyl-3-oxo-1-cyclopentenyl)amino]propionic acid

Prepared from Example 6. δH (d⁶-DMSO) 9.30 (1H, s), 9.22 (1 H, s), 8.67 (1H, d), 8.39 (1 H, d), 8.14 (1 H, d), 7.78 (2H, m), 7.28 (3H, m), 7.10 (1 H, d), 4.28 (1H, m), 3.18 (1 H, dd, J 13.6, 4.3Hz), 3.01 (1 H, dd, J 13.6, 10.0Hz), 2.40-1.90 (6H, m), 0.83 (3H, t, J 7.4Hz). m/z (ESI, 70V) 417 (MH⁺).

### EXAMPLE 24

### (2S)3-[4-(2,6-Naphthyridin-1-yl)amino)phenyl]-2-[(2-allyl-3-oxo-1-cyclohexenyl)amino]propionic acid

Prepared from Example 7. δH (d⁶-DMSO) 9.31 (1 H, s), 9.23 (1H, s), 8.68 (1H, d, J 5.9Hz), 8.41 (1 H, d, J 5.8Hz), 8.14 (1 H, d, J 5.7Hz), 7.80 (2H, m), 7.28 (1 H, d, J 5.7Hz), 7.18 (2H, d, J 8.5Hz), 5.77 (1 H, d, J 8.7Hz), 5.57 (1H, m), 4.93 (1H, dd, J 17.2, 1.8Hz), 4.85 (1H, dd, J 10.0, 1.8Hz), 4.42 (1H, br), 3.11 (1 H, dd, J 13.7, 4.7Hz), 3.07 (1 H, dd, J 13.7, 8.0Hz), 2.95 (2H, d, J 5.6Hz), 2.31 (1 H, m), 2.14 (1 H, m), 2.06 (2H, m), 1.69 (2H, m). m/z (ESI, 70V) 443 (MH⁺).

### EXAMPLE 25

### (2S)-3-[4-(2,6-Naphthyridin-1-yl)amino)phenyl]-2-[(5-phenyl-3-oxo-1-cyclohexenyl)amino]propionic acid

Prepared from Example 8. δH (d⁶-DMSO) 9.33 (1 H, s), 9.23 (1 H, s), 8.69 (1 H, d, J 5.8Hz), 8.41 (1H, d, J 5.8Hz), 8.15 (1 H, d, J 5.6Hz), 7.32-7.20 (9H, m), 4.89 (1 H, s), 4.10 (1 H, m), 3.20 (2H, m), 3.09 (1 H, dd, J 13.7, 5.2Hz), 2.99 (1 H, m), 2.72-2.23 (4H, m). m/z (ESI, 70V) 479 (MH⁺).

### EXAMPLE 26

### (2S)-3-[4-(2,6-Naphthyridin-1-yl) amino)phenyl]-2-[(5-propyl-3-oxo-1-cyclohexenyl)amino]propionic acid

Prepared from Example 9. δH (d⁶-DMSO) 9.40 (1H, d, J 0.7Hz), 9.30 (1 H, s), 8.86 (1 H, d, J 5.9 Hz), 8.56 (1 H, d, J 5.9Hz), 8.35 (1 H, d, J 5.7Hz), 7.99 (2H, d, J 8.3Hz), 7.43 (3H, m), 6.95 (1 H, d, J 7.4Hz), 4.31 (1 H, m), 3.31 (1 H, m), 3.16 (1 H, m), 2.64 (1 H, dt, J 15.5, 3.7Hz), 2.37-2.00 (4H, m), 1.51 (4H, m), 1.08 (3H, dt, J 6.8. 1.9Hz). m/z (ESI, 70V) 445 (MH⁺).

### EXAMPLE 27

### (2S)-3-[4-(2,6-Naphthyridin-1-yl)amino)phenyl]-2-[(2-benzyl-3-oxo-1-cyclopentenyl)amino]propionic acid

Prepared from Example 10. δH (d⁶-DMSO) 9.30 (1 H, s), 9.23 (1 H, s), 8.68 (1H, d, J. 5.9Hz), 8.41 (1H, d, J 5.9Hz), 8.16 (1H, d, J 5.7Hz), 7.76 (2H, d), 7.29 (1 H, d, J 5.8Hz), 7.23-7.10 (6H, m), 7.09 (1 H, m), 4.17 (1H, br), 3.39 (2H, m), 3.16 (1 H, br d), 2.94 (1H, br d), 2.39 (1 H, dd, J 16.3, 7.0Hz), 2.15-1.92 (3H, m). m/z (ESI, 70V) 479 (MH⁺).

### EXAMPLE 28

### (2S)-3-[4-(2,6-Naphthyridin-1-yl)amino)phenyl]-2-[(2-allyl-3-oxo-1-cyclopentenyl)amino]propionic acid

Prepared from Example 11. δH (d⁶-DMSO) 9.29 (1 H, s), 9.22 (1 H, s), 8.67 (1 H, d, J 5.9Hz), 8.39 (1 H, d, J 5.9Hz), 8.13 (1 H, d, J 5.7Hz), 7.76 (2H, m), 7.27 (1 H, d, J 5.7Hz), 7.22 (2H, d, J 8.5Hz), 6.93 (1H, br), 5.69 (1 H, m), 4.93 (1 H, d, J 16.8Hz), 4.86 (1 H, dd, J 10.0, 2.0Hz), 4.22 (1 H, br), 3.14 (1 H, dd, J 13.8, 4.3Hz), 2.96 (1 H, m), 2.78 (2H, d, J 4.3Hz), 2.36 (1 H, dd, J 16.8, 7.1 Hz), 2.10-1.90 (3H, m). m/z (ESI, 70V) 429 (MH⁺).

### EXAMPLE 29

### (2S)-3-[4-(2,6-Naphthyridin-1-yl)amino)phenyl]-2-[(2-butyl-3-oxo-1-cyclopentenyl)amino]propionic acid

Prepared from Example 12. δH (d⁶-DMSO) 9.29 (1 H, s), 9.22 (1H, s), 8.68 (1H, d, J 5.9Hz), 8.40 (1H, dd, J 5.9, 1.0Hz), 8.14 (1 H, dd, J 5.7, 1.3HZ), 7.79 (2H, d, J 8.5Hz), 7.26 (3H, m), 6.97 (1H, br), 4.25 (1H, m), 3.17 (1H, dd, J 13.6, 3.9Hz), 3.00 (1H, dd, J 13.0, 10.0Hz), 2.32 (1 H, m), 2.20-1.95 (5H, m), 1.21 (4H, m), 0.85 (3H, t, J 6.9Hz). m/z (ESI, 70V) 445 (MH⁺).

### EXAMPLE 30

### (2S)-3-[4-(2,6-Naphthyridin-1-yl)amino)phenyl]-2-[(2-phenyl-3-oxo-1-cyclopentenyl)amino]propionic acid

Prepared from Example 13. δH (d⁶-DMSO) 9.30 (1 H, br), 9.23 (1 H,d, J 0.6Hz), 8.68 (1H, d, J 5.9Hz), 8.39 (1H, d, J 5.9Hz), 8.13 (1 H, d, J 5.8Hz), 7.80 (2H, d, J 8.5Hz), 7.38-7.18 (8H, m), 6.70 (1 H, d, J 8.9Hz), 4.49 (1 H, m), 3.17 (1 H, m), 3.10 (1 H, m), 2.59 (1 H, m), 2.43 (1 H, m), 2.25 (1 H, m). m/z (ESI, 70V) 465 (MH⁺).

### EXAMPLE 31

### (2S)-3-[4(2,6-Naphthyridin-1-yl)amino)phenyl]-2-[(2-propyl-3-oxo-1-cyclopentenyl)amino]propionic acid

Prepared from Example 14. δH (d⁶-DMSO) 9.29 (1 H, s), 9.22 (1 H, s), 8.68 (1 H, d, J 5.9Hz), 8.39 (1 H, dd, J 5.9, 0.9Hz), 8.14 (1 H, dd, J 5.6, 1.3Hz), 7.78 (2H, m), 7.27 (3H, m), 7.05 (1 H, d, J 9.4Hz), 4.28 (1H, m), 3.18 (1 H, dd, J 13.6, 4.3Hz), 2.97 (1 H, dd, J 13.6, 9.9Hz), 2.33 (1 H, m), 2.15-2.05 (5H, m), 1.26 (2H, hex, J 7.1 Hz), 0.82 (3H, t, J 7.3Hz). m/z (ESI, 70V) 431 (MH⁺).

### EXAMPLE 32

### (2S)-3-[4-(2,6-Naphthyridin-1-yl)amino)phenyl]-2-[(2-ispropyl-3-oxo-1-cyclopentenyl)amino]propionic acid

Prepared from Example 15. δH (d⁶-DMSO) 9.30 (1 H, s), 9.23 (1 H, s), 8.68 (1 H, br), 8.40 (1 H, d, J 5.7Hz), 8.14 (1 H, d, J 5.7Hz), 7.79 (2H, m), 7.25 (3H, m), 6.79 (1 H, d, J 9.4Hz), 4.29 (1H, m), 3.17 (1H, dd, J 13.6, 4.4Hz), 3.04 (1H, dd, J 13.5, 9.6Hz), 2.69 (1H, sept, J 7.0Hz), 2.27 (1H, m), 2.05-1.85 (3H, m), 1.09 (3H, d, J 7.1Hz), 1.06 (3H, d, J 7.1Hz). m/z (ESI, 70V) 431 (MH⁺).

### EXAMPLE 33

### (2S)-3-[4-(2,6-Naphthyridin-1-yl)amino)phenyl]-2-[(2-isobutyl-3-oxo-1-cyclopentenyl)amino]propionic acid

Prepared from Example 16. δH (d⁶-DMSO) 9.29 (1 H, s), 9.22 (1 H, s), 8.67 (1 H, d, J 5.9Hz), 8.40 (1 H, d, J 5.9Hz), 8.13 (1 H, d, J 5.7Hz), 7.78 (2H, d, J 8.5Hz), 7.24 (3H, m), 6.89 (1 H, d, J 9.2Hz), 4.22 (1 H, m), 3.17 (1 H, dd, J 13.6, 4.2Hz), 2.99 (1H, dd, J 13.4, 9.6Hz), 2.33 (1H, m), 2.07-1.88 (5H, m), 1.67 (1 H, sept, J 6.6Hz), 0.78 (3H, d, J 6.6Hz), 0.77 (3H, d, J 6.6Hz). m/z (ESI, 70V) 445 (MH⁺).

### EXAMPLE 34

### (2S)-3-[4-(3-Ethylisoquinolin-1-ylamino)phenyl]-2-[(2-ethyl-3-oxo-1-cyclopentenyl)amino]propionic acid

Prepared from Example 17. δH (d⁶-DMSO) 9.04 (1 H. s), 8.46 (1H, d, J 7.1 Hz), 7.87 (2H, d, J 8.5Hz), 7.71 (1 H, d, J 7.1Hz), 7.64 (1 H, t, J 7.1Hz), 7.51 (1H, t, J 7.1 Hz), 7.23 (2H, d, J 8.6Hz), 7.07 (1 H, d, J 9.2Hz), 6.98 (1H, s), 4.28 (1 H, m), 3.16 (1H, dd, J 13.6, 4.3Hz), 3.00 (1 H, dd, J 13.6, 9.9Hz), 2.69 (2H, q, J 7.5Hz), 2.33 (1 H, m), 2.06 (5H, m), 1.28 (3H, t, J 7.5 Hz), 0.84 (3H, t, J 7.3Hz). m/z (ESI, 70V) 444 (MH⁺).

### EXAMPLE 35

### (S)-Ethyl-3-[4-[(2,7-Naphthyridin-1-yl)oxy]phenyl]-2-[(2-propyl-3-oxo-1-cyclopentenyl)amino]propionate

A solution of Intermediate 21 (355mg, 1.01mmol) and Intermediate 5 (156mg, 1.11 mmol) in nitromethane (5ml) with Na₂SO₄ (~0.5g) and acetic acid (2 drops) was heated for 2 days at 90°. The solution was filtered, filtrate concentrated *in vacuo* and residue purified by silica chromatography (SiO₂;0.4% MeOH in DCM) to give the title compound as an off white solid (222mg 46%). δH (DMSO) 9.43 (1 H, s), 8.56 (1H, d, J 5.7Hz), 7.59 (1 H, d, J 5.8Hz), 7.23 (1 H, s), 7.20 (2H, d, J 8.3Hz), 7.06 (2H, d, J 8.4Hz), 7.05 (1 H, d), 4.28 (1 H, m), 3.99 (2H, q, J 7.1 Hz), 3.07 (1 H, dd, J 13.6, 5.2Hz), 2.94 (1 H, dd, J 13.5, 9.8Hz), 2.19 (3H, s), 2.20-1.84 (6H, m), 1.09 2H, m), 1.03 (3H, t, J 7.1hz), 0.64 (3H, t, J 4.7Hz). m/z (ESI, 70V) 474 (MH⁺).

In a similar manner to Example 35 were prepared Examples 36 to 48

### EXAMPLE 36

### (S)-Ethyl-3-[(4-(2,6-naphthyridin-1-yl)amino]phenyl]-2-[(1-ethylpropyl]-3-oxo-1-cyclopentenyl)amino]propionate

Prepared from (*S*)-ethyl-3-[4-[(2,6-naphthyrid-1-yl)amino]phenyl]-2-amino-propionate and Intermediate 8. δH (DMSO-d⁶) 9.30-9.20 (2H, m), 8.70 (1H, m), 8.40 (1 H, br), 8.10 (1 H, br m) 7.80 (2H, m), 7.20 (3H, m), 4.50 (1H, m), 4.10 (2H, m), 3.20-3.00 (2H, m), 2.30-2.00 (5H, m), 1.60 (2H, m), 1.40 (2H, m), 1.20 (3H, m), 0.80 (6H, t, J 7.5Hz). m/z (ESI. 70V) 487 (MH⁺).

### EXAMPLE 37

### (S)-Ethyl-3-[(4-[3-methyl-2,7-naphthyridin-1-yl)oxy]phenyl-2-[(2-isobutyl-3-oxo-1-cyclopentenyl)amino]propionate

Prepared from Intermediates 21 and 7. δH (DMSO-d⁶) 9.60 (1H, s), 8.73 (1H, d, J 5.7Hz), 7.76 (1 H, d, J 5.8Hz), 7.39 (2H, d), 7.37 (1 H, s), 7.22 (2H, d), 7.13 (1H, d), 4.47 (1 H, m), 4.15 (2H, q, J 7.1Hz), 3.24 (1 H, dd), 3.09 (1H, dd, J 13.7, 10.0Hz), 2.35 (1H, m), 2.35 (3H, s), 2.20-2.00 (3H, m), 1.91 (2H, d), 1.68 (1 H, seq t), 1.20 (3H, t, J 7.1 Hz), 0.78 (3H, d, J 6.6HZ), 0.78 (3H, d, J 6.6Hz). m/z (ESI, 70V) 488 (MH⁺).

### EXAMPLE 38

### (S)-Ethyl-3-[(4-[2,7-naphthyridin-1-yl)oxy]phenyl]-2-[(2-propyl-3-oxo-1-cycllopentenyl)amino]propionate

Prepared from Intermediates 14 and 5. δH (DMSO-d⁶) 9.71 (1H, s), 8.83 (1 H, d, J 5.7HZ), 8.13 (1H. d, J 5.8Hz), 7.91 (1H, d), 7.56 (1 H, d, J 5.9Hz), 7.41 (2H, d, J 8.5HZ), 7.24 (3H, d), 4.46 (1 H, m), 4.18 (2H, q, J 7.1 Hz), 3.25 (1 H, dd, J 13.6, 5.0Hz), 3.11 (1H,dd, J 13.5, 9.8Hz), 2.34 (1 H, m), 2.17-2.00 (5H, m), 1. 27 (2H,m), 1.23 (3H, t, J 7.1Hz), 0.82 (3H, t, J 7.3Hz). m/z (ESI, 70V) 460 (MH⁺).

### EXAMPLE 39

### (S)-Ethyl-3-[4-(2,7-naphthyridin-1-yl)oxy]phenyl]-2-[(2-isobutyl-3-oxo-1 -cyclopentenyl)amino]propionate

Prepared from Intermediates 14 and 7. δH (DMSO-d⁶) 9.74 (1H, s), 8.87 (1 H, d, J 5.7Hz), 8.17 (1 H, d, J 5.8Hz), 7.95 (2H, d, J 5.8Hz), 7.59 (1 H, d, J 5.8Hz), 7.44 (2H, d, J 8.5Hz), 7.27 (2H, d, J 8.5Hz), 7.19 (1H, d, J 9.6Hz), 4.52 (1 H, m), 4.21 (2H, q, J 7.1 Hz), 3.30 (1 H, dd, J 13.6, 5.0Hz), 3.15 (1 H, dd, J 13.6, 9.9Hz), 2.56 (1H, m), 2.39-2.04 (3H, m), 1.97 (2H, d), 1.73 (1 H, sept J 6.7Hz), 1.26 (2H, t, J 7.1Hz). 0.83 (3H, d, J 6.6HZ), 0.82 (3H, d, J 6.6Hz). m/z (ESI, 70V) 474 (MH⁺).

### EXAMPLE 40

### (S)-Ethyl-3-[(4-(2,7-naphthyridin-1-yl)amino]phenyl]-2-[(2-isobutyl-3-oxo-1-cyclopentenyl)amino]propionate

Prepared from Intermediates 13 and 7. δH (DMSO-d⁶) 9.84 (1H, s), 8.67 (1 H, d, J 5.6HZ), 8.17 (1H, d), 7.78 (2H, m), 7.70 (1H, d, J 5.6HZ), 7.27 (2H, d, J 8.5HZ), 7.14 (1 H, d, J 5.7Hz), 7.09 (1 H, d, J 9.4Hz), 4.42 (1 H, m), 4.16 (2H, q, J 7.1Hz), 3.18 (1H. m), 4.16 (2H, q, J 7.1Hz), 3.18 (1H. dd, J 13.6, 4.9Hz), 3.05 (1 H, dd, J 13.6, 9.7Hz), 2.36 (1 H, m), 2.17-1.97 (5H, m), 1.69 (1 H, m), 1.22 (3H, t, J 7.1 HZ), 0.80 (6H, d, J 6.6Hz). m/z (ESI, 70V), 473 (MH⁺).

### EXAMPLE 41

### (S)-Ethyl-3-[(4-(3-methyl-2,7-naphthyridin-1-yl)amino]phenyl]-2-[(2-isobutyl-3-oxo-1-cyclopentenyl)amino]propionate

Prepared from Intermediates 18 and 7 as an orange solid. δH (DMSO-d⁶) 8.57 (1 H, d), 7.87 (2H, d, J 8.3Hz), 7.57 (1H, d), 7.25 (2H, d, J 8.4Hz), 7.14 (1 H, d), 6.97 (1H,s), 4.45 (1 H, m), 4.17 (2H, q, J 7.4Hz), 3.17 (1 H, dd, J 14.2, 4.9Hz), 3.05 (1 H, dd, J 14.2, 10.2Hz), 2.44 (3H, s), 2.37 (1 H, m), 2.16 (1 H, m), 2.05 (2H, m), 1.92 (2H, d), 1.70 (1 H, m), 1.21 (3H, t), 0.79 (6H, d); m/z (ESI, 70V) 487 (MH⁺).

### EXAMPLE 42

### (S)-Methyl-3-(4-[2',6'-dimethoxy]biphenylyl)-2-[(2-isobutyl-3-oxo-1-cyclopentenyl)amino]propionate

Prepared from the free amino of Intermediate 24 and Intermediate 7. δH (DMSO-d⁶) 7.26 (3H, m), 7.14 (2H, d, J 8.2Hz), 6.75 (2H, d, J, 8.4HZ), 6.68 (1 H, d, J 9.5Hz), 4.48 (1H, m), 3.73 (1 H, s), 3.65 (1 H, s), 3.25 (1 H, dd, J 13.7, 5.0Hz), 3.10 (1 H, dd, J 13.7, 9.3Hz), 2.35 (1 H, m), 2.13-1.99 (3H,m), 1.95 (2H, d, J 7.2Hz), 1.77 (1 H, seq, J 6.81 Hz), 0.82 (3H, d, J 6.7Hz), 0.81 (3H, d, J 6.6HZ). m/z (ESI, 70V) 452 (MH⁺).

### EXAMPLE 43

### Ethyl-3-[4-(3,5-dichloropyrid-4-ylcarboxamido)phenyl]-3-[(2-ethyl-3-oxo-1-cyclopentenyl)amino]propionate

Prepared from Ethyl-3-[4-({3,5-dichloropyrid-4-yl}carboxamido) phenyl]-3-aminopropionate and 2-ethyl-cyclopentane-1,3-dione. δH (DMSO-d⁶), 8.81 (2H, s), 7.64 (2H, d), 7.51 (1H, d), 7.45 (2H, d), 5.00 (1H, m), 4.10 (2H, q, J 7.1 Hz), 3.05 (1 H, dd), 2.84 (1 H, dd), 2.64 (1 H, m), 2.31 (1 H, m), 2.09 (4H, m), 1.17 (3H, t, J 7.09 Hz), 0.86 (3H, t, J 7.3 Hz). m/z (ES1, 70V) 491 (MH⁺).

### EXAMPLE 44

### Ethyl-3-[4-({3,5-dichloropyrid-4-yl}carboxamido)phenyl]-3-[(2-propyl-3-oxo-1-cyclopentenyl)amino]propionate

Prepared from ethyl-3-[4-({3,5-dichloropyrid-4-yl}carboxamido)phenyl]-3-aminopropionate and Intermediate 5. δH (DMSO-d⁶), 8.89 (2H, s), 7.66 (2H, d), 7.47 (2H, d), 5.03 (1 H, m), 4.11 (2H, q, J 7.3Hz), 3.08 (1 H, dd), 2.88 (1 H, dd), 2.73 (1 H, m), 2.35 (1 H, m), 2.09 (4H, m), 1.35 (2H, quin, J 7.13, 4.73Hz), 1.16 (3H, t, J 7.1 Hz), 0.88 (3H, t, J 7.2Hz). m/z (ES+, 70V) 505 (MH⁺).

### EXAMPLE 45

### Ethyl-3-[4-({3,5-dichloropyrid-4-yl}carboxamido)phenyl]-3-[(2-isobutyl-3-oxo-1-cyclopentenyl)amino]propionate

Prepared from ethyl-3-[4-({3,5-dichloropyrid-4-yl}carboxamido)phenyl]-3-aminopropionate (prepared according to the methods of International Patent Application WO 00/32575) and Intermediate 7. δH (DMSO-d⁶), 8.83 (2H, s), 7.65 (2H, d), 7.46 (2H, d), 7.40 (1 H, d), 5.01 (1 H, m), 4.12 (2H, q, J 7.14Hz), 2.98 (1 H, m), 2.81 (2H, m); 2.33 (1 H, m), 2.17 (2H, m), 1.99 (2H, m),1.78 (1H, m), 1.17 (3H, t, J 7.1Hz), 0.85 (6H, d, J 6.55Hz). m/z (ES+, 70V) 519 (MH⁺).

### EXAMPLE 46

### Methyl-3-[4-({3,5-dichloropyrid-4-yl}carboxamido)phenyl]-3-[(2-butyl-3-oxo-1-cyclopentenyl)amino]propionate

Prepared from methyl-3-[4-({3,5-dichloropyrid-4-yl}carboxamido)phenyl]-3-amino propionate (prepared according to the methods in International Patent Application WO 00/18759) and Intermediate 3. δH (DMSO-d⁶) 8.80 (2H, s), 7.60 (2H, d), 7.42 (2H, d), 7.38 (1 H, d), 4.98 (1H, m), 3.62 (3H, s), 3.05 (1 H, dd), 2.87 (1 H, dd), 2.38-1.95 (6H, m), 1.21 (4H, m), 0.42 (3H, t). m/z. (ESI, 70V) 504 (MH⁺).

### EXAMPLE 47

### Methyl-3-[4-(3,5-dichloropyrid-4-ylcarboxamido)phenyl]-3-[(2-phenyl-3-oxo-1-cyclopentenyl)amino]propionate

Prepared from Methyl-3-[4-(3,5-dichloropyrid-4-yl carboxamido)phenyl]-3-aminopropionate and intermediate 4. δH (CD₃OD) 8.66 (2H, s), 7.73-7.17 (9H, 3 x m), 5.22 (1H, br), 3.77 (3H, s), 3.10-2.90 (2H, m), 2.57-2.45 (4H, m). m/z (ESI, 70V) 523 (MH⁺).

### EXAMPLE 48

### Ethyl-3-[4-(3{,5-dichloropyrid-4-yl}carboxamido)phenyl-3-[(5-propyl-3-oxo-1-cyclohexenyl)amino]propionate

Prepared from ethyl-3-[4-({3,5-dichloropyrid-4-yl}carboxamido)phenyl]-3-amino-propionate and 5-propyl-1,3-cyclohexanedione. δH (DMSO-d⁶), 8.80 (2H, s), 7.56 (2H, d), 7.46 (1 H, d), 7.36 (2H, d), 4.71 (1 H, m), 4.08 (2H, q, J 7.4Hz), 2.86 (1 H, m), 2.74 (1 H, m), 2.38 (1 H, m), 2.09 (2H, m), 1.81 (2H, m), 1.32 (4H, m), 1.18 (3H, t), 0.88 (3H, t). m/z (ES⁺, 70V) 519 (MH⁺).

In a similar manner to Example 18 were prepared Examples 49 - 62.

### EXAMPLE 49

### (S)-3-[4[(3-Methyl-2,7-naphthyridinyl)oxy]phenyl]-2-[(2-propyl-3-oxo-1-cyclopentenyl)amino]propionic acid

Prepared from Example 35. δH (DMSO-d⁶) 9.34 (1 H, s), 9.27 (1 H, s), 8.72 (1H, d. J 5.8Hz), 8.45 (1 H, d, J 5.9Hz), 8.18 (1H, d, J 5.7HZ), 7.80 (2H, d, J. 8.4HZ), 7.32 (1H, d, J 5.7Hz), 7.24 (2H, d, J 8.4Hz), 6.67 (1 H, d, J 8.8Hz), 4.15 (1 H, br), 3.17 (1H, dd, J 13.5, 4.1 Hz), 3.01 (1H, dd, J. 13.2, 8.5Hz), 2.38 (1 H, m), 2.21 (1H, m), 2.20-1.90 (3H, m), 1.75-1.60 (2H, m), 1.60-1.40 (2H, m), 0.78 (3H, t, J 7.1 Hz). 0.77 (3H, t, J 7.4Hz); m/z (ESI, 70V) 459 **(MH⁺).**

### EXAMPLE 50

### (S)-3-[4-[(2,6-Naphthyridinyl)amino]phenyl]-2-[(2-(1-ethypropyl)-3-oxo-1-cyclopentenyl)amino]propionic acid

Prepared from Example 36. δH (DMSO-d⁶) 9.82 (1 H, s), 8.75 (1 H, d, J 5.7Hz), 7.98 (1 H, d, J 5.8Hz), 7.43 (3H, d), 7.30 (1 H, d), 4.56 (1 H, m), 3.47 (1H, dd, J 13.6, 4.3HZ), 3.29 (1H, dd, J 13.6, 10.2Hz), 2.58 (3H, s), 2.55 (1 H, m), 2.30-2.20 (5H, m), 1.47 (2H, m), 1.02 (3H, t, J 7.2Hz). m/z (ESI, 70V) 446 (MH⁺).

### EXAMPLE 51

### (S)-3-[(4-[3-Methyl-2,7-naphthyridinyl]oxy)phenyl]-2-[(2-isobutyl-3-oxo-1-cyclopentenyl)amino]propionic acid

Prepared from Example 37. δH (DMSO-d⁶) 9.61 (1H, s), 8.74 (1H, d, J 5.7Hz), 7.77 (1 H, d, J 5.7Hz), 7.39 (2H, d, J 8.3Hz), 7.37 (1 H, s), 7.22 (2H, d, J 8.3Hz), 7.06 (1 H, d, J 9.7Hz), 4.38 (1 H, m), 3.26 (1 H, dd ???), 3.09 (1H, dd, J 13.4, 10.3Hz), 2.33 (3H, s), 2.37 (1H, m), 2.10-1.85 (5H, m), 1.68 (1 H, m), 0.78 (6H, m). m/z (ESI, 70V) 460 (MH⁺).

### EXAMPLE 52

### (S)-3-[(4-(2,7-naphthyridin-1-yl)oxy)phenyl]-2-[(2-propyl-3-oxo-1-cyclopentenyl)amino]propionic acid

Prepared from Example 38. δH (DMSO-d⁶) 9.71 (1H,s), 9.83 (1H, d, J 5.7HZ), 8.14 (1 H, d, J 5.8Hz), 7.91 (1 H, d, J 5.8Hz), 7.55 (1 H, d, J 5.8Hz), 7.41 (2H, d, J 8.Hz), 7.23 (2H, d, J 8.5Hz), 7.05 (1 H, d), 4.36 (1 H, m), 3.26 (1H, dd), 3.08 (1 H, dd, J 13.5, 19.2Hz), 2.33 (1H, m), 2.01 (5H, m), 1.27 (2H,m), 0.82 3H, t, J 7.3Hz). m/z (ESI, 70V) 432 (MH⁺).

### EXAMPLE 53

### (S)-3-[(4-(2,7-Naphthyridin-1-yl)oxy)phenyl]-2-[(2-isobutyl-3-oxo-1-cyclopentenyl)amino]propionic acid

Prepared from Example 39. δH (DMSO-d⁶) 9.70 (1H, s), 8.83 (1H, d, J 5.7Hz), 8.13 (1 H, d, J 5.8Hz), 7.90 (1 H, d, J 5.7Hz), 7.55 (1 H, d, J 5.8Hz), 7.40 (2H, d, J 8.4HZ), 7.22 (2H, d, J 5.8Hz), 7.05 (1H. d), 4.38 (1H, m), 3.27 (1H, m) 3.08 (1H, dd, J 13.5,10.1 Hz), 2.36 (1H, m), 2.06 (3H, m), 1.91 (2H,d), 1.69 (1 H, m), 0.79 (3H, d, J 6.6Hz), 0.78 (3H, d, J 6.6Hz). m/z (ESI, 70V) 446 (MH⁺).

### EXAMPLE 54

### (S)-3-[4-[(2,7-Naphthyridin-1yl)amino]phenyl]-2-[(2-osobutyl-3-oxo-1-cyclopentenyl)amino]propionic acid

Prepared from Example 40. δH (DMSO-d⁶) 9.83 (1H,s), 9.52 (1H, s), 8.66 (1 H, d, J 5.6Hz), 8.16 (1 H, d, J 5.7HZ), 7.73 (2H, d, J 8.4Hz), 7.69 (1 H, d, J 5.6Hz), 7.22 (2H, d, J 8.4Hz), 7.13 (1 H, d, J 5.Hz), 4.13 (1H, br), 3.15 (1 H, dd, J 13.6, 3.7Hz), 2.97 (1H, dd, J 13.2, 9.2Hz), 2.36 (1H, m), 2.10-1.88 (5H, m), 1.69 (1H, m), 0.80 (3H, d, J 6.3Hz), 0.79 (3H, d, J 6.4Hz). m/z (ESI,70V) 445 (MH⁺).

### EXAMPLE 55

### (S)-3-[4-[(3-Methyl-2,7-naphthyridin-1-yl)amino]phenyl]-2-[(2-isobutyl-3-oxo-1-cyclopentenyl)amino]propionic acid

Prepared from Example 41. δH (DMSO-d⁶) 8.55 (1 H, d), 7.86 (2H, d, J 8.2Hz), 7.56 (1 H, d), 7.24 (2H, d, J 8.3Hz), 7.15 (1 H, d), 6.96 (1 H, s), 4.43 (1 H, m), 3.17 (1H, dd, J 14.1, 5.8Hz), 3.04 (1 H, dd, J 14.1, 10.1 Hz), 2.43 (3H, s), 2.36 (1H, m), 2.15 (1 H, m), 1.91 (2H, d), 1.71 (1 H, m), 1.22 (3H, t), 0.78 (6H, d). m/z (ESI, 70V) 459 (MH⁺).

### EXAMPLE 56

### (S)-3-[4-(-2,'6'-Dimethoxy)biphenyl]-2-[(2-isobutyl-3-oxo-1-cyclopentenyl)amino]propionic acid

Prepared from Example 42. δH (DMSO-d⁶) 7.27 (3H, m), 7.09 (3H, d), 6.72 (2H, d, J 8.4Hz), 4.36 (1 H, m), 3.64 (6H, s), 3.24 (1H, dd, J 13.6, 4.2HZ), 3.03 (1 H, dd, J 13.6, 10.2Hz), 2.32 (1 H, m), 2.06 (1 H, m), 1.97-1.91 (4H, m), 1.72 (1H, m), 0.79 (6H, d, J 6.5Hz). m/z (ESI, 70V) 438 (MH⁺).

### EXAMPLE 57

### 3-[4-[(3,5-Dichloropyrid-4 yl)carboxamido]phenyl]-3-[(2-ethyl-3-oxo-1-cyclopentenyl)amino]propionic acid

Prepared from Example 43. δH NMR (DMSO-d⁶), 8.79 (2H, s), 7.62 (2H, d), 7.49 (1 H, d), 7.42 (2H, d), 4.97 (1H, m), 3.03 (1H. dd), 2.82 (1H, dd), 2.62 (1 H, m), 2.29 (1 H, m), 2.07 (4H, m), 0.85 (3H, t, J 7.3Hz). m/Z (ES+, 70V) 463 (MH⁺).

### EXAMPLE 58

### 3-[4-[(3,5-Dichloropyrid-4-yl)carboxamido]phenyl]-3-[(2-propyl-3-oxo-1-cyclopentenyl)amino]propionic acid

Prepared from Example 44. δH NMR (DMSO-d⁶), 8.85 (2H, s), 7.63 (2H, d), 7.45 (2H, d), 4.98 (1H, m), 3.04 (1 H, dd), 2.85 (1 H, dd), 2.71 (1H, m), 2.31 (1 H, m), 2.05 (4H, m), 1.33 (2H, quin, J 7.13, 4.73Hz), 0.89 (3H, t, J 7.2Hz). m/z (ES+, 70V) 477 (MH⁺).

### EXAMPLE 59

### 3-[4-[(3,5-Dichloropyrid-4-yl)carboxamido]phenyl]-3-[(isobutyl-3-oxo-1-cyclopentenyl)amino]propionic acid

Prepared from Example 45. δH NMR (DMSO-d⁶), 8.81 (2H, s), 7.63 (2H, d), 7.44 (2H, d), 7.38 (1 H, d), 4.97 (1 H, m), 2.96 (1 H, m), 2.79 (2H, m), 2.31 (1H, m), 2.15 (2H, m), 1.96 (2H, m), 1.75 (1H. m), 0.82 (6H, d, J 6.54Hz). m/z (ES+, 70V) 491 (MH⁺).

### EXAMPLE 60

### 3-[4-[(3,5-Dichloropyrid-4-yl)carboxamido]phenyl]-3-[(2-butyl-3-oxo-1-cyclopentenyl)amino]propionic acid

Prepared from Example 46. δH (DMSO-d⁶) 10.89 (1 H, s), 8.76 (2H, s), 7.58 (2H, d, J 8.5Hz), 7.38 (3H, d), 4.90 (1 H, m), 2.88 (1 H, m), 2.71 (1 H, dd, J 15.9, 4.9Hz), 2.63 (1 H, m), 2.21 (1 H, m), 2.10-2.00 (4H, m), 1.20 (4H, br), 0.82 (3H, t, J 6.9Hz). m/z (ESI, 70V) 489 (MH⁺).

### EXAMPLE 61

### 3-[4-[(3,5-Dichloropyrid-4-yl)carboxamido]phenyl]1-3[(2-phenyl-3-oxo-1-cyclopentenyl)amino]propionic acid

Prepared from Example 47. δ H (DMSO-d⁶) 11.00 (1 H, s), 8.85 (1 H, s), 7.98 (1 H, br), 7.70 (2H, d, J 8.0Hz), 7.52 (2H, d, J 8.0Hz), 7.41 (4H, m), 7.27 (1 H, m), 5.12 (1 H, br), 3.08 (1 H, m), 2.88 (1 H, m), 2.73 (1 H, m), 2.49 (1 H, m), 2.32 (2H, m). m/z (ESI, 70V) 510 (MH⁺).

### EXAMPLE 62

### 3-[4-[(3,5-Dichloropyrid-4-yl)carboxamido]phenyl]-3-[(5-propyl-3-oxo-1-cyclohexenyl)amino]propionic acid

Prepared from Example 48. δH NMR (DMSO-d⁶), 8.80 (2H, s), 7.60 (2H, d), 7.53 (1H, d), 7.35 (2H, d), 4.71 (1H, m), 2.81 (1 H, m), 2.68 (1H, m), 2.40 (1 H, m), 2.10 (2H, m), 1.81 (2H, m), 1.29 (4H, m), 0.88 (3H, t). m/z (ES+, 70V) 491 (MH⁺).

In a similar manner to Example 35 were prepared Examples 63-66:

### EXAMPLE 63

### Ethyl-3-[5-[(3-methyl-2,7-naphthyridin-1-yl)oxy]-pyridin-2-yl]-2-[(2-propyl-3-oxo-1-cyclopentenyl)amino]propionate

Prepared from intermediates 29 and 5 δH (CDCl₃) 9.72 (1H, s), 8.73 (1H, d, J 5.8Hz), 8.59 (1H, d, J 2.6Hz), 7.65 (1H, dd, J 8.4, 2.7Hz), 7.54 (1 H, d, J 5.8Hz), 7.26 (1H, d, J 8.4HZ), 7.17 (1H,s), 6.51 (1 H, d, J 9.3Hz), 4.67-4.61 (1H, symm. m), 4.20 (2H, q, J 7.1 Hz), 3.45-3.37 (2H, m), 2.45 (3H, s), 2.52-2.31 (4H, m), 2.14 (2H, qd, J 14.6, 7.5Hz), 1.46 (2h, hextet, J 7.4Hz), 1.23 (3H, t, J 7.1 Hz) and 0.92 (3H, t, J 7.3Hz); m/z (ES⁺, 70V) 475 (MH⁺).

### EXAMPLE 64

### Ethyl-3-[5-[(3-methyl-2,7-naphthyridin-1-yl)oxy]-pyridin-2-yl]-2-[(2-isobutyl-3-oxo-1-cyclopentenyl)amino]propionate

Prepared from Intermediates 29 and 7. δH (CDCl₃) 9.63 (1 H, s), 8.64 (1 H, d, J 5.8Hz), 8.51 (1 H, d, J 2.7Hz), 7.56 (1 H, dd, J 8.4, 2.7Hz), 7.45 (1 H, dd, J 5.8, 0.7Hz), 7.17 (1 H, d, J 8.4Hz), 7.08 (1H,s), 6.49 (1 H, d, J 9.2Hz), 4.59-4.52 (1 H, m), 4.11 (2H, q, J 7.1HZ), 3.34-3.32 (2H, m), 2.45-2.27 (4H, m), 2.37 (3H, s), 1.95 (2H, qd, J 13.9, 7.2HZ), 1.75 (1H, m), 1.14 (3H, t, J 7.1 Hz), 0.82 (3H, d, J 6.2Hz) and 0.80 (3H, d, J 6.2HZ), m/Z (ES⁺, 70V) 489 (MH⁺).

### EXAMPLE 65

### Ethyl-3-[5-[(3-methyl-2,7-naphthyridin-1-yl)oxy]-pyridin-2-yl]-2-[(2-ethyl-3-oxo-1-cyclopentenyl)amino]propionate

Prepared from Intermediate 29 and 2-ethyl-1,3-cyclopentanedione. δH (CDCl₃) 9.63 (1 H, s), 8.64 (1 H, d, J 5.8Hz), 8.51 (1H, d, J 2.6Hz), 7.56 (1H. dd, J 8.4, 2.7Hz), 7.46 (1 H, dd, J 5.8, 0.8Hz), 7.18 (1 H, d, J.8.4Hz), 7.08 (1 H, s), 6.34 (1 H, d, J 9.3Hz), 4.59-4.52 (1 H, m), 4.12 (2H, q, J 7.1 Hz), 3.39-3.29 (2H, m), 2.37 (3H, s), 2.38-2.25 (4H, m), 2.17-2.03 (2H, m), 1.16 (3H, t, J 7.1 Hz) and 0.93 (3H, t, J 7.5Hz); m/z (ES⁺, 70V) 461 (MH⁺).

### EXAMPLE 66

### (S)-Ethyl-3-[4-[(3,5-dichloropyrid-4-yl)carboxamide]phenyl]-2-[(-2-isobutyl-3-oxo-1-cyclopentenyl)amino]propionate

Prepared from (S)-ethyl-3-[4-[(3,5-dichloropyrid-4-yl)carboxamido]phenyl]-2-aminopropionate and Intermediate 7. δH (MeOD) 8.55 (2H, s), 7.50 (2H, d, J 8.5Hz), 7.19 (2H, d, J 8.5hz), 4.41 (1 H, dd, J 9.8, 4.7Hz), 4.16 (1H, q, J 7.2Hz), 4.13 (1 H, q, J 7.2Hz), 3.23 (1H, dd, J 13.9, 4.6Hz), 2.97 (1H, dd, J 13.9, 9.9Hz), 2.38 (1H, m), 2.18 (3H, m), 1.88 (2H, d, J 7.3Hz), 1.58 (1 H, septet, J 6.9Hz), 1.19 (3H, J 7.2Hz), 0.72 (6H, dd, J 6.9, 1.5HZ); m/z (ES⁺, 70V) 518 (MH⁺).

To a similar manner to Example 18 were prepared Examples 67 - 70.

### EXAMPLE 67

### 3-[5-[(3-Methyl-2,7-naphthyridin-1-yl)oxy]-pyridin-2-yl]-2-[(2-propyl-3-oxo-1-cyclopentenyl)amino]propionic acid

Prepared from Example 63. δH (DMSO-d⁶) 9.65 (1 H, s), 8.76 (1 H, d, J 5.8Hz), 8.55 (1H, d, J 2.7Hz), 7.79 (1 H, d, J 5.8Hz), 7.77 (1 H, dd, J 8.4, 2.7Hz), 7.43 (1 H, d, J 8.4Hz), 7.41 (1 H, s), 7.22 (1 H, d, J 9.6Hz), 4.65 (1 H, narrow, m), 3.40 (1 H, dd, J 14.1, 4.5Hz), 3.31 91 H, dd, J 14.1, 9.Hz), 2.43-2.37 (1 H, m), 2.38 (3H, s), 2.27-2.22 (1 H, m), 2.09-2.05 (2H, m), 2.04-1.97 (2H, m), 1.24 (2H, hextet, J. 7.4Hz), and 0.79 (3H, t, J. 7.3Hz); m/z (ES⁺, 70V) 447 (MH⁺).

### EXAMPLE 68

### 3-[5-[(3-Methyl-2,7-naphthyridin-1-yl)oxy]-pyrridin-2-yl]-2-[(2-isobutyl-3-oxo-1-cyclopentenyl)amino]propionic acid

Prepared from Example 64. δH (DMSO-d⁶) 9.65 (1H, s), 8.76 (1H, d, J 5.7Hz), 8.57 (1 H, d, J 2.7Hz), 7.79 (1 H, d, J 5.8hz), 7.74 (1 H, dd, J 8.4, 2.7Hz), 7.41 (1 H, s), 7.41 (1 H, d, J 8.4Hz), 7.11 (1 h, d, J 9.3HZ), 4.60 (1H, m), 3.40 (1 H, m), 3.30 91 H, m), 2.45-2.40 (1 H, m), 2.37 (3H, s), 2.27-2.22 (1 H, m), 2.15-2.00 (2H, m), 1.95-1.82 (2H, m), 1.65 (1 H, m), 0.77 (3H, d, J. 6.6Hz), and 0.72 (3H, d, J 6.6Hz), m/z (ES⁺, 70V) 461 (MH⁺).

### EXAMPLE 69

### 3-[5-[(3-Methyl-2,7-naphthyridin-1-yl)oxy]-pyridin-2-yl]-2-[(2-ethyl-3-oxo-1-cyclopentenyl)amino]propionic acid

Prepared from Example 65. δH (DMSO-d⁶) 9.65 (1H, s), 8.76 (1H, d, J 5.7HZ), 8.58 (1 H, d, J 2.7Hz), 7.80-7.76 (2H, overlapping signals), 7.44 (1 H,d, J 8.5Hz), 7.41 (1 H, s), 7.24 (1H, d, J 9.5Hz), 4.65 (1 H, m), 3.40 (1 H, dd, J 14.1, 4.5Hz), 3.31 (1 H, dd, J 14.1, 9.5Hz), 2.43-2.39 (1 H, m) 2.38 (3H, s), 2.26-2.20 (1 H, m), 2.08-2.00 (4H, m) and 0.82 (3H, t, J 7.4Hz); m/z (ES⁺, 70V) 433 (MH⁺).

### EXAMPLE 70

### (S)-3-[4-[(3,5-Dichloropyrid-4-yl)carboxamido)phenyl]-2-[(2-isobutyl-3-oxo-1-cyclopentenyl)amino]propionic acid

Prepared from Example 66. δH (MeOD) 8.67 (2H,s), 7.59 (2H, d, J 8.5Hz), 7.31 (2H, d, J 8.5Hz), 4.46 (1 H, dd, J 9.8, 4.2Hz), 3.37 (1H, dd, J 13.9, 10.0Hz), 2.50 (1H dd, J 14.2, 4.9Hz), 2.22 (3H, m), 1.99 (2H, d, J 5.2Hz), 1.69 (1 H, septet, J 6.6Hz), 0.82 (6H, d, J 6.6Hz). m/z (ES⁺, 70V) 491 (MH⁺).

### EXAMPLE 71

### (S)-Ethyl-3-[4-{(3-methyl-2,7-naphthyridin-1-yl)amino}phenyl]-2-[(2-cyclohexyl-3-oxo-1-cyclopentenyl)amino]propionate

Prepared from the Intermediate 9 and the Intermediate 19 in a similar manner to Example 35. δH (DMSO-d⁶) 9-77 (1H, s), 9.47 (1H, s), 8.59 (1H, d, J 6.0Hz), 7.88 (2H, d, J 4.0Hz). 7.85 (1H, d, J 5.0Hz), 7.84 (2H, d, J 3.0Hz), 7.57 (1H. s), 7.24 (1H. d, J 8.0Hz), 4.40 (1H. m), 4.18 (2H, q, J 7.0Hz), 3.19 (1H, dd, J 14.0, 5.0Hz), 3.08 (1H, dd, J 14.0, 9.0Hz), 2.44 (3H, s), 2.35 (1H, m), 2.33 (1H. m), 2.29 (1H. m), 1.83 (3H, s), 1.80 (2H, m), 9.70 (5H, m), 1.63 (5H, m), 1.30 (3H, m). m/z (ES⁺, 70V) 512 (MH⁺).

### EXAMPLE 72

### (S)-3-[4-{(3-Methyl-2,7-naphthyridin-1-yl)amino}phenyl]-2-[(2-cyclohexyl-3-oxo-1-cyclopentenyl)amino]propionic acid

Prepared from Example 71 in a similar manner to Example 18. δH (DMSO-d⁶) 9.75 (1H. s), 8.58 (1H, d, J 6.0Hx). 7.87 (2H, d, J 8.0Hz), 7.54 (1H, d, J 6.0Hz), 7.25 (2H, d, J 8.0Hz), 6.96 (1H, s), 6.31 (1 H, br s), (4.27 (1H, m), 3-21 (1H, dd, J 14.0, 4.0Hz), 3.09 (1H, dd, J 14.0, 8.0Hz), 2.47 (3H, s), 2.39 (2H, m), 2.35 (3H, s), 2.08 (2H, m), 1.75 (5H, m), 1,39 (5H, m). m/z (ES⁺, 70V) 485 (MH⁺).

The following assays can be used to demonstrate the potency and selectivity of the compounds according to the invention. In each of these assays an IC₅₀ value was determined for each test compound and represents the concentration of compound necessary to achieve 50% inhibition of cell adhesion where 100% = adhesion assessed in the absence of the test compound and 0% = absorbance in wells that did not receive cells.

### α₄β₁ Integrin-dependent Jurkat cell adhesion to VCAM-Ig

96 well NUNC plates were coated with F(ab)₂ fragment goat anti-human IgG Fcγ-specific antibody [Jackson Immuno Research 109-006-098: 100 µl at 2 µg/ml in 0.1M NaHCO₃, pH 8.4], overnight at 4°. The plates were washed (3x) in phosphate-buffered saline (PBS) and then blocked for 1h in PBS/1% BSA at room temperature on a rocking platform. After washing (3x in PBS) 9 ng/ml of purified 2d VCAM-Ig diluted in PBS/1% BSA was added and the plates left for 60 minutes at room temperature on a rocking platform. The plates were washed (3x in PBS) and the assay then performed at 37° for 30 min in a total volume of 200 µl containing 2.5 x 10⁵ Jurkat cells in the presence or absence of titrated test compounds.

Each plate was washed (2x) with medium and the adherent cells were fixed with 100 µl methanol for 10 minutes followed by another wash. 100 µl 0.25% Rose Bengal (Sigma R4507) in PBS was added for 5 minutes at room temperature and the plates washed (3x) in PBS. 100 µl 50% (v/v) ethanol in PBS was added and the plates left for 60min after which the absorbance (570nm) was measured.

### α₄β₇ Integrin-depend JY cell adhesion to MAdCAM-Ig

This assay was performed in the same manner as the α4β1 assay except that MAdCAM-lg (150ng/ml) was used in place of 2d VCAM-lg and a sub-line of the β-lympho blastoid cell-line JY was used in place of Jurkat cells. The IC₅₀ value for each test compound was determined as described in the α₄β₁ integrin assay.

### α₅β₁- Integrin-dependent K562 cell adhesion to fibronectin

96 well tissue culture plates were coated with human plasma fibronectin (Sigma F0895) at 5µg/ml in phosphate-buffered saline (PBS) for 2 hr at 37°C. The plates were washed (3x in PBS) and then blocked for 1h in 100µl PBS/1% BSA at room temperature on a rocking platform. The blocked plates were washed (3x in PBS) and the assay then performed at 37°C in a total volume of 200µl containing 2.5x 10⁵ K562 cells, phorbol-12-myristate-13-acetate at 10ng/ml and in the presence or absence of titrated test compounds. Incubation time was 30 minutes. Each plate was fixed and stained as described in the α₄β₁ assay above.

### αₘβ₂-dependent human polymorphonuclear neutrophils adhesion to plastic

96 well tissue culture plates were coated with RPMI 1640/10% FCS for 2h at 37°C. 2 x 10⁵ freshly isolated human venous polymorphonuclear neutrophils (PMN) were added to the wells in a total volume of 200µl in the presence of 10ng/ml phorbol-12-myristate-13-acetate, and in the presence or absence of test compounds, and incubated for 20m in at 37°C followed by 30min at room temperature. The plates were washed in medium and 100µl 0.1% (w/v) HMB (hexadecyl trimethyl ammonium bromide, Sigma H5882) in 0.05M potassium phosphate buffer, pH 6.0 added to each well. The plates were then left on a rocker at room temperature for 60 min. Endogenous peroxidase activity was then assessed using tetramethyl benzidine (TMB) as follows: PMN lysate samples mixed with 0.22% H₂O₂ (Sigma) and 50µg/ml TMB (Boehringer Mannheim) in 0.1M sodium acetate/citrate buffer, pH 6.0 and absorbance measured at 630nm.

### αIIb/β₃ -dependent human platelet aggregation

Human platelet aggregation was assessed using impedance aggregation on the Chronolog Whole Blood Lumiaggregometer. Human platelet-rich plasma (PRP) was obtained by spinning fresh human venous blood anticoagulated with 0.38% (v/v) tri-sodium citrate at 220xg for 10 min and diluted to a cell density of 6 x 10⁸/ml in autologous plasma. Cuvettes contained equal volumes of PRP and filtered Tyrode's buffer (g/liter: NaCl 8.0; MgCl₂.H₂O 0.427; CaCl₂ 0.2; KCI 0.2; D-glucose 1.0; NaHCO₃ 1.0; NaHPO₄.2H₂O 0.065). Aggregation was monitored following addition of 2.5µM ADP (Sigma) in the presence or absence of inhibitors.

In the above assays the preferred compounds of the invention in which R¹ is an α₄ integrin binding group, such as the compounds of the Examples generally have IC₅₀ values in the α₄β₁ and α₄β₇ assays of 1 µM and below. In the other assays featuring α integrins of other subgroups the same compounds had IC₅₀ values of 50µM and above thus demonstrating the potency and selectivity of their action against α₄ integrins.

## Claims

1. A compound according to formula (1): 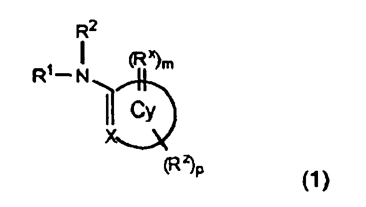 wherein
R¹ is a group Ar¹L²Ar²Alk- in which Ar¹ is an optionally substituted aromatic or heteroaromatic group, L² is a covalent bond or a linker atom or group L^{2a} or a linker -(Alk^{a})L^{2a}-, where Alk^{a} is an aliphatic or heteroaliphatic chain, and L^{2a} is a linker atom or group selected from -O- or -S- atoms or -C(O)-, -C(O)O-, -OC(O)-, -C(S)-, -S(O)-, -S(O)₂-, -N(R⁸)- [where R⁸ is a hydrogen atom or a straight or branched C₁₋₆alkyl group], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)-, or -N(R⁸)SO₂N(R⁸)-, Ar² is an optionally substituted arylene or heteroarylene group and Alk is a chain -CH₂-CH(R)-,in which R is a carboxylic acid (-CO₂H) or a -CO₂Alk⁷ group;
Alk⁷ is a straight or branched C₁₋₈alkyl group, or a C₂₋₈alkenyl, C₂₋₈alkynyl, C₃₋₈cycloalkyl, C₃₋₈cycloalkylC₁₋₈alkyl, C₃₋₈heterocycloalkylC₁₋₆alkyl, C₁₋₆alkyloxyC₁₋₆alkyl, C₁₋₆alkylthioC₁₋₆alkyl, C₁₋₆alkylsulfinylC₁₋₆alkyl, C₁₋₆alkylsulfonylC₁₋₆alkyl, C₃₋₈cycloalkyloxyC₁₋₆alkyl, C₃₋₈cycloalkylthioC₁₋₆alkyl, C₃₋₈cycloalkylsulfinylC₁₋₆alkyl, C₃₋₈cycloalkylsulfonylC₁₋₆alkyl, C₁₋₆alkyloxycarbonylC₁₋₆alkyl, C₁₋₆alkyloxycarbonylC₁₋₆alkenyl, C₁₋₆alkyloxycarbonyloxyC₁₋₆alkyl, C₁₋₆alkyloxycarbonyloxyC₁₋₆alkenyl, C₃₋₈cycloalkyloxycarbonyloxyC₁₋₆alkyl, N-di-C₁₋₈alkylaminoC₁₋₈alkyl, N-C₆₋₁₂aryl-N-C₁₋₆alkylaminoC₁₋₆alkyl, N-di-C₁₋₈alkylcarbamoylC₁₋₈alkyl, C₆₋₁₀arylC₁₋₆alkyl, C₆₋₁₀aryl, C₆₋₁₀aryloxyC₁₋₈alkyl, C₆₋₁₂arylthioC₁₋₈alkyl, C₆₋₁₂arylsulfinylC₁₋₈alkyl, C₆₋₁₂arylsulfonylC₁₋₈alkyl, C₁₋₈alkanoyloxyC₁₋₈alkyl, C₄₋₈imidoC₁₋₈alkyl, C₆₋₁₂aroyloxyC₁₋₈alkyl, or a triglyceride group;
R² is a hydrogen atom;
the ring Cy is an unsaturated cycloaliphatic or heterocycloaliphatic ring containing X, in which X is a N atom or a C(R^{w}) group;
R^{w} is a group R^{z};
R^{x} which may be present on any available carbon atom of the ring Cy is a oxo (=O) group;
m is the integer 1;
R^{z} which may be present on any available carbon or nitrogen atom of the ring Cy is selected from a halogen atom or -(Alk⁴)ᵥL¹(Alk¹)ₙR³ in which Alk⁴ is a straight or branched C₁₋₃alkylene chain, v is zero or the integer 1, L¹ is a covalent bond or a linker atom or group as defined for L^{2a}, n is zero or the integer 1, Alk¹ is an optionally substituted aliphatic chain and R³ is a hydrogen atom or a -CN, -NO₂ or an optionally substituted heteroaliphatic, cycloaliphatic, heterocycloaliphatic, polycycloaliphatic, heteropolycycloaliphatic, aromatic or hetero-aromatic group;
p is zero or the integer 1, 2, 3 or 4;
provided that Cy is not a cyclobutenedione group; and the compound of formula (1) is not dimedonyl-(S)-tyrosine-O-benzyl methyl ester;
and the salts, solvates, hydrates and N-oxides thereof.

2. A compound according to Claim 1 in which R is a carboxylic acid (-CO₂H) group.

3. A compound according to Claim 1 in which R is an esterified carboxyl group of formula -CO₂Alk⁷.

4. A compound according to any one of Claims 1 to 3 in which Ar² is an optionally substituted phenylene group.

5. A compound according to any one of Claims 1 to 4 in which Ar¹ is an optionally substituted phenyl or five-, six- or ten-membered heteroaromatic group.

6. A compound according to Claim 5 in which Ar¹ is an optionally substituted pyridyl, pyrimidinyl, naphthyridinyl, quinolinyl or isoquinolinyl group.

7. A compound according to Claim 1 in which Cy is an optionally substituted cyclopentenyl, cyclohexenyl, cycloheptenyl, dihydropyrimidinyl, dihydropyridinyl or imidazolinyl group.

8. A compound according to any one of Claims 1 to 6 in which X is a N atom.

9. A compound according to Claim 8 in which Cy is an optionally substituted 2-aminopyridin-4-one [where amino refers to the group - N(R²)-], 4-aminopyrimidin-2-one, 2-aminopyridin-4-one, 6-amino-pyridin-2-one or 2-aminoimiadozolin-4-one ring.

10. A compound according to any one of Claim 1 to 6 in which X is a C(R^{w}) group.

11. A compound according to Claim 10 in which Cy is an optionally substituted 3-amino-2-cyclopenten-1-one [where amino refers to the group -N(R²)-] or 3-amino-2-cyclohexen-1-one ring.

12. A compound according to Claim 11 in which p is zero or the integer 1 or 2.

13. A compound according to any one of Claims 1 to 12 in which v in the group R^{z} is zero, Alk¹ is an aliphatic chain and R³ is a hydrogen atom.

14. A compound according to Claim 13 in which -Alk¹R³ is an optionally substituted C₁₋₆alkyl, allyl, (-CH₂CH=CH₂) or propargyl (-CH₂CCH) group.

15. A compound according to Claim 14 in which L¹ is a covalent bond.

16. A compound which is:
(*S*)-3-[4-[(2,6-Naphthyridinyl)amino]phenyl]-2-[(2-(1-ethylpropyl)-3-oxo-1-cyclopentenyl)amino]propionic acid;
(*S*)-3-[4-[(3-Methyl-2,7-naphthyridinyl)oxy]phenyl]-2-[(2-propyl-3-oxo-1-cyclopentenyl)amino]propionic acid;
(*S*)-3-[(4-[3-Methyl-2,7-naphthyridinyl]oxy)phenyl]-2-[(2-isobutyl-3-oxo-1-cyclopentenyl)amino]propionic acid;
(*S*)-3-[(4-(2,7-Naphthyridin-1-yl)oxy)phenyl]-2-[(2-isobutyl-3-oxo-1-cyclopentenyl)amino]propionic acid;
(*S*)-3-[4-[(3,5-Dichloropyrid-4-yl)carboxamido)phenyl]-2-[(2-isobutyl-3-oxo-1-cyclopentenyl)amino]propionic acid;
(*S*)-3-[4-[(3-Methyl-2,7-naphthyridin-1-yl)amino]phenyl]-2-[(2-isobutyl-3-oxo-1-cyclopentenyl)amino]propionic acid;
(*S*)-3-[4-(-2',6'-Dimethoxy)biphenyl]-2-[(2-isobutyl-3-oxo-1-cyclopentenyl)amino]propionic acid;
(2*S*)-3-[4-(2,6-Naphthyridin-1-y()amino)phenylj-2-[(2-isobutyl-3-oxo-1-cyclopentenyl)amino]propionate;
(2*S*)-3-[4-(2,6-Naphthyridin-1-yl)amino)phenyl]-2-[(2-isopropyl-3-oxo-1-cyclopentenyl)amino]propionic acid;
(2*S*)-3-[4-(2,6-Naphthyridin-1-yl)amino)phenyl]-2-[(2-allyl-3-oxo-1-cyclopentenyl)amino]propionate;
(2*S*)-3-[4-(2,6-Naphthyridin-1-yl)amino)phenyl]-2-[(2-propyl-3-oxo-1-cyclopentenyl)amino]propionate;
and the salts, solvates, hydrates and N-oxides and carboxylic acid ester, particularly methyl, ethyl, propyl and i-propyl esters thereof.

17. A pharmaceutical composition comprising a compound according to Claim 1 together with one or more pharmaceutically acceptable carriers, excipients or diluents.

## Patentansprüche

1. Verbindung gemäß Formel (1): 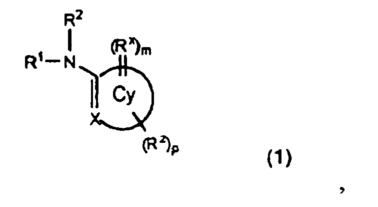 worin
R¹ eine Gruppe Ar¹L²Ar²Alk- ist, worin Ar¹ eine gegebenenfalls substituierte aromatische oder heteroaromatische Gruppe ist, L² eine kovalente Bindung oder ein Linkeratom oder -gruppe L^{2a} oder ein Linker -(Alk^{a})L^{2a}- ist, wo Alk^{a} eine aliphatische oder heteroaliphatische Kette ist, und L^{2a} ein Linkeratom oder -gruppe ist, ausgewählt aus -O- oder -S-Atomen oder -C(O)-, -C(O)O-, -OC(O)-, -C(S)-, -S(O)-, -S(O)₂-, -N(R⁸)- [wo R⁸ ein Wasserstoffatom oder eine gerade oder verzweigte C₁₋₆-Alkylgruppe ist], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R⁸)-, -N(R⁸)N(R⁸)-, -N(R⁸)CON(R⁸)-, -N(R⁸)CSN(R⁸)- oder -N(R⁸)SO₂N(R⁸)-, Ar² eine gegebenenfalls substituierte Arylen- oder Heteroarylengruppe ist, und Alk eine Kette -CH₂-CH(R)- ist, worin R eine Carbonsäure (-CO₂H) oder eine -CO₂Alk⁷-Gruppe ist;
Alk⁷ eine gerade oder verzweigte C₁₋₈-Alkylgruppe oder eine C₂₋₈-Alkenyl-, C₂₋₈-Alkinyl-, C₃₋₈-Cycloalkyl-, C₃₋₈-Cycloalkyl-C₁₋₈-alkyl-, C₃₋₈-Heterocycloalkyl-C₁₋₆-alkyl-, C₁₋₆-Alkyloxy-C₁₋₆-alkyl-, C₁₋₆-Alkylthio-C₁₋₆-alkyl-, C₁₋₆-Alkylsulfinyl-C₁₋₆-alkyl-, C₁₋₆-Alkylsulfonyl-C₁₋₆-alkyl-, C₃₋₈-Cycloalkyloxy-C₁₋₆-alkyl-, C₃₋₈-Cycloalkylthio-C₁₋₆-alkyl-, C₃₋₈-Cycloalkylsulfinyl-C₁₋₆-alkyl-, C₃₋₈-Cycloalkylsulfonyl-C₁₋₆-alkyl-, C₁₋₆-Alkyloxycarbonyl-C₁₋₆-alkyl-, C₁₋₆-Alkyloxycarbonyl-C₁₋₆-alkenyl-, C₁₋₆-Alkyloxycarbonyloxy-C₁₋₆-alkyl-, C₁₋₆-Alkyloxycarbonyloxy-C₁₋₆-alkenyl-, C₃₋₈-Cycloalkyloxycarbonyloxy-C₁₋₆-alkyl-, N-Di-C₁₋₈-alkylamino-C₁₋₈-alkyl-, N-C₆₋₁₂-Aryl-N-C₁₋₆-alkylamino-C₁₋₆-alkyl-, N-Di-C₁₋₈₋alkylcarbamoyl-C₁₋₈-alkyl-, C₆₋₁₀-Aryl-C₁₋₆-alkyl-, C₆₋₁₀-Aryl-, C₆₋₁₀-Aryloxy-C₁₋₈-alkyl-, C₆₋₁₂-Arylthio-C₁₋₈-alkyl-, C₆₋₁₂-Arylsulfinyl-C₁₋₈-alkyl-, C₆₋₁₂-Arylsulfonyl-C₁₋₈-alkyl-, C₁₋₈-Alkanoyloxy-C₁₋₈-alkyl-, C₄₋₈-Imido-C₁₋₈-alkyl-, C₆₋₁₂-Aroyloxy-C₁₋₈-alkyl- oder eine Triglyceridgruppe ist;
R² ein Wasserstoffatom ist;
der Ring Cy ein ungesättigter cycloaliphatischer oder heterocycloaliphatischer Ring ist, enthaltend X, wobei X ein N-Atom oder eine C(R^{w})-Gruppe ist;
R^{w} eine Gruppe R^{z} ist;
R^{x}, das an jedem verfügbaren Kohlenstoffatom des Rings Cy vorliegen kann, eine OxoGruppe (=O-Gruppe) ist;
m die ganze Zahl 1 ist;
R^{z}, das an jedem verfügbaren Kohlenstoffatom oder Stickstoffatom des Rings Cy vorliegen kann, ausgewählt ist aus einem Halogenatom oder -(Alk⁴)ᵥL¹(Alk¹)ₙR³ worin Alk⁴ eine gerade oder verzweigte C₁₋₃-Alkylenkette ist, v null oder die ganze Zahl 1 ist, L¹ eine kovalente Bindung oder ein Linkeratom oder -gruppe, wie für L^{2a} definiert, ist, n null oder die ganze Zahl 1 ist, Alk¹ eine gegebenenfalls substituierte aliphatische Kette ist und R³ ein Wasserstoffatom oder eine -CN-, -NO₂- oder eine gegebenenfalls substituierte heteroaliphatische, cycloaliphatische, heterocycloaliphatische, polycycloaliphatische, heteropolycycloaliphatische, aromatische oder heteroaromatische Gruppe ist;
p null oder die ganze Zahl 1, 2, 3 oder 4 ist;
mit der Maßgabe, daß Cy keine Cyclobutendiongruppe ist; und die Verbindung der Formel (1) nicht Dimedonyl-(S)-tyrosin-O-benzylmethylester ist;
und die Salze, Solvate, Hydrate und N-Oxide davon.

2. Verbindung nach Anspruch 1, wobei R eine Carbonsäure-Gruppe (-CO₂H-Gruppe) ist.

3. Verbindung nach Anspruch 1, wobei R eine veresterte Carboxylgruppe der Formel -CO_{z}Alk⁷ ist.

4. Verbindung nach einem der Ansprüche 1 bis 3, wobei Ar² eine gegebenenfalls substituierte Phenylengruppe ist.

5. Verbindung nach einem der Ansprüche 1 bis 4, wobei Ar¹ eine gegebenenfalls substituierte Phenyl- oder fünf-, sechs- oder zehngliedrige heteroaromatische Gruppe ist.

6. Verbindung nach Anspruch 5, wobei Ar¹ eine gegebenenfalls substituierte Pyridyl-, Pyrimidinyl-, Naphthyridinyl-, Chinolinyl- oder Isochinolinylgruppe ist.

7. Verbindung nach Anspruch 1, wobei Cy eine gegebenenfalls substituierte Cyclopentenyl-, Cyclohexenyl-, Cycloheptenyl-, Dihydropyrimidinyl-, Dihydropyridinyl- oder Imidazolinylgruppe ist.

8. Verbindung nach einem der Ansprüche 1 bis 6, wobei X ein N-Atom ist.

9. Verbindung nach Anspruch 8, wobei Cy ein gegebenenfalls substituierter 2-Aminopyridin-4-on- [wo sich Amino auf die Gruppe -N(R²)- bezieht], 4-Aminopyrimidin-2-on-, 2-Aminopyridin-4-on-, 6-Aminopyridin-2-on- oder 2-Aminoimidazolin-4-on-Ring ist.

10. Verbindung nach einem der Ansprüche 1 bis 6, wobei X eine C(R^{w})-Gruppe ist.

11. Verbindung nach Anspruch 10, wobei Cy ein gegebenenfalls substituierter 3-Amino-2-cyclopenten-1-on- [wo sich Amino auf die Gruppe -N-(R²)- bezieht] oder 3-Amino-2-cyclohexen-1-on-Ring ist.

12. Verbindung nach Anspruch 11, wobei p null oder die ganze Zahl 1 oder 2 ist.

13. Verbindung nach einem der Ansprüche 1 bis 12, wobei v in der Gruppe R^{z} null ist, Alk¹ eine aliphatische Kette ist und R³ ein Wasserstoffatom ist.

14. Verbindung nach Anspruch 13, wobei -Alk¹R³ eine gegebenenfalls substituierte C₁₋₆-Alkyl-, Allyl-, (-CH₂CH=CH₂)- oder Propargyl-Gruppe (-CH₂CCH-Gruppe) ist.

15. Verbindung nach Anspruch 14, wobei L¹ eine kovalente Bindung ist.

16. Verbindung, die:
(S)-3-[4-[(2,6-Naphthyridinyl)amino]phenyl]-2-[(2-(1-ethylpropyl)-3-oxo-1-cyclopentenyl)-amino]propionsäure;
(S)-3-[4-[(3-Methyl-2,7-naphthyridinyl)oxy]phenyl]-2-[(2-propyl-3-oxo-1-cyclopentenyl)-amino]propionsäure;
(S)-3-[(4-[3-Methyl-2,7-naphthyridinyl]oxy)phenyl]-2-[(2-isobutyl-3-oxo-1-cyclopentenyl)-amino]propionsäure;
(S)-3-[(4-(2,7-Naphthyridin-1-yl)oxy)phenyl]-2-[(2-isobutyl-3-oxo-1-cyclopentenyl)amino]-propionsäure;
(S)-3-[4-[(3,5-Dichlorpyrid-4-yl)carboxamido)phenyl]-2-[(2-isobutyl-3-oxo-1-cyclopentenyl)amino]propionsäure;
(S)-3-[4-[(3-Methyl-2,7-naphthyridin-1-yl)amino]phenyl]-2-[(2-isobutyl-3-oxo-1-cyclopentenyl)amino]propionsäure;
(S)-3-[4-(2',6'-Dimethoxy)biphenyl]-2-[(2-isobutyl-3-oxo-1-cyclopentenyl)amino]propionsäure;
(2S)-3-[4-(2,6-Naphthyridin-1-yl)amino)phenyl]-2-[(2-isobutyl-3-oxo-1-cyclopentenyl)-amino]propionat;
(2S)-3-[4-(2,6-Naphthyridin-1-yl)amino)phenyl]-2-[(2-isopropyl-3-oxo-1-cyclopentenyl)-amino]propionsäure;
(2S)-3-[4-(2,6-Naphthyridin-1-yl)amino)phenyl]-2-[(2-allyl-3-oxo-1-cyclopentenyl)amino]-propionat;
(2S)-3-[4-(2,6-Naphthyridin-1-yl)amino)phenyl]-2-[(2-propyl-3-oxo-1-cyclopentenyl)amino]-propionat;
und die Salze, Solvate, Hydrate und N-Oxide und Carbonsäureester, insbesondere Methyl-, Ethyl-, Propyl- und i-Propylester, davon ist.

17. Pharmazeutische Zusammensetzung, umfassend eine Verbindung nach Anspruch 1 zusammen mit einem oder mehreren pharmazeutisch akzeptablen Trägem, Hilfsstoffen oder Verdünnungsmitteln.

## Revendications

1. Composé de formule (1) : 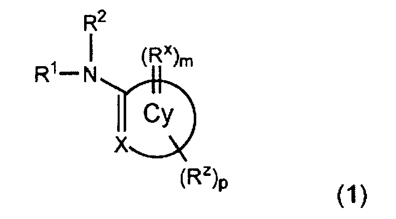 dans laquelle
R¹ est un groupe Ar¹L²Ar²Alk- dans lequel Ar¹ est un groupe aromatique ou hétéroaromatique éventuellement substitué, L² est une liaison covalente ou un atome ou un groupe de liaison L^{2a} ou une liaison -(Alk^{a}) L^{2a-}, dans laquelle Alk^{a} est une chaîne aliphatique ou hétéroaliphatique, et L^{2a} est un atome ou un groupe de liaison choisi parmi des atomes de -0- ou de -S- ou -C (O) -, -C(O)O-, -OC(O)-, -C(S)-, -S(O)-, -S(O)₂, -N(R⁸)-[dans laquelle R⁸ est un atome d'hydrogène ou un groupe alcoyle linéaire ou ramifié ayant de 1 à 6 atomes de carbone], -CON(R⁸)-, -OC(O)N(R⁸)-, -CSN(R⁸)-, -N(R⁸)CO-, -N(R⁸)C(O)O-, -N(R⁸)CS-, -S(O)₂N(R⁸)-, -N(R⁸)S(O)₂-, -N(R⁸)O-, -ON(R⁸), -N(R⁸)N(R⁸)-, -N(R⁸)CON(R⁸)-, -N (R⁸) CSN (R⁸) - ou --N (R⁸) SO₂N (R⁸) -, Ar² est un groupe arylène ou hétéroarylène éventuellement substitué et Alk est une chaîne -CH₂-CH(R)- dans laquelle R est un acide carboxylique (-CO₂H) ou un groupe -CO₂Alk⁷ ;
Alk⁷ est un groupe alcoyle linéaire ou ramifié ayant de 1 à 8 atomes de carbone ou un groupe alcényle ayant de 2 à 8 atomes de carbone, alcynyle ayant de 2 à 8 atomes de carbone, cycloalcoyle ayant de 3 à 8 atomes de carbone, cycloalcoylalcoyle ayant de 3 à 8 atomes de carbone dans la partie cycloalcoyle et de 1 à 8 atomes de carbone dans la partie alcoyle, hétérocycloalcoylalcoyle ayant de 3 à 8 atomes de carbone dans la partie hétérocycloalcoyle et de 1 à 6 atomes de carbone dans la partie alcoyle, alcoyloxyalcoyle ayant de 1 à 6 atomes de carbone dans la partie alcoyloxy et de 1 à 6 atomes de carbone dans la partie alcoyle, alcoylthioalcoyle ayant de 1 à 6 atomes de carbone dans la partie alcoylthio et de 1 à 6 atomes de carbone dans la partie alcoyle, alcoylsulfinylalcoyle ayant de 1 à 6 atomes de carbone dans la partie alcoylsulfinyle et de 1 à 6 atomes de carbone dans la partie alcoyle, alcoylsulfonylalcoyle ayant de 1 à 6 atomes de carbone dans la partie alcoylsulfonyle et de 1 à 6 atomes de carbone dans la partie alcoyle, cycloalcoyloxyalcoyle ayant de 3 à 8 atomes de carbone dans la partie cycloalcoyloxy et de 1 à 6 atomes de carbone dans la partie alcoyle, cycloalcoylthioalcoyle ayant de 3 à 8 atomes de carbone dans la partie cycloalcoylthio et de 1 à 6 atomes de carbone dans la partie alcoyle, cycloalcoylsulfinylalcoyle ayant de 3 à 8 atomes de carbone dans la partie cycloalcoylsulfinyle et de 1 à 6 atomes de carbone dans la partie alcoyle, cycloalcoylsulfonylalcoyle ayant de 3 à 8 atomes de carbone dans la partie cycloalcoylsulfonyle et de 1 à 6 atomes de carbone dans la partie alcoyle, alcoyloxycarbonylalcoyle ayant de 1 à 6 atomes de carbone dans les parties alcoyle, alcoyloxycarbonylalcényle ayant de 1 à 6 atomes de carbone dans la partie alcoyle et de 1 à 6 atomes de carbone dans la partie alcényle, alcoyloxycarbonyloxyalcoyle ayant de 1 à 6 atomes de carbone dans les parties alcoyle, alcoyloxycarbonyloxyalcényle ayant de 1 à 6 atomes de carbone dans la partie alcoyloxy et de 1 à 6 atomes de carbone dans la partie alcényle, cycloalcoyloxycarbonyloxyalcoyle ayant de 3 à 8 atomes de carbone dans la partie cycloalcoyloxy et de 1 à 6 atomes de carbone dans la partie alcoyle, N-di-alcoylaminoalcoyle ayant de 1 à 8 atomes de carbone dans les parties alcoyle, N-aryl-N-alcoylaminoalcoyle ayant de 6 à 12 atomes de carbone dans la partie aryle, de 1 à 6 atomes de carbone dans la partie alcoylamino et de 1 à 6 atomes de carbone dans la partie alcoyle, N-di-alcoylcarbamoylalcoyle ayant de 1 à 8 atomes de carbone dans les parties alcoyle, arylalcoyle ayant de 6 à 10 atomes de carbone dans la partie aryle et de 1 à 6 atomes de carbone dans la partie alcoyle, aryle ayant de 6 à 10 atomes de carbone, aryloxyalcoyle ayant de 6 à 10 atomes de carbone dans la partie aryloxy et de 1 à 8 atomes de carbone dans la partie alcoyle, arylthioalcoyle ayant de 6 à 12 atomes de carbone dans la partie arylthio et de 1 à 8 atomes de carbone dans la partie alcoyle, arylsulfinylalcoyle ayant de 6 à 12 atomes de carbone dans la partie arylsulfinyle et de 1 à 8 atomes de carbone dans la partie alcoyle, arylsulfonylalcoyle ayant de 6 à 12 atomes de carbone dans la partie arylsulfonyle et de 1 à 8 atomes de carbone dans la partie alcoyle, alcanoyloxyalcoyle ayant de 1 à 8 atomes de carbone dans la partie alcanoyloxy et dans la partie alcoyle, imidoalcoyle ayant de 4 à 8 atomes de carbone dans la partie imido et de 1 à 8 atomes de carbone dans la partie alcoyle, aroyloxyalcoyle ayant de 6 à 12 atomes de carbone dans la partie aroyloxy et de 1 à 8 atomes de carbone dans la partie alcoyle, ou un groupe triglycéride ;
R² est un atome d'hydrogène ;
le cycle Cy est un cycle cycloaliphatique ou hétérocycloaliphatique insaturé contenant X, dans lequel X est un atome de N ou un groupe C(R^{w}) ;
R^{w} est un groupe R^{z} ;
R^{x}, qui peut être présent sur n'importe quel atome de carbone disponible du cycle Cy, est un groupe oxo (=O) ;
m est le nombre entier 1 ;
R^{z}, qui peut être présent sur n'importe quel atome de carbone ou d'azote disponible du cycle Cy, est choisi parmi un atome d'halogène ou (Alk⁴)ᵥL¹(Alk¹) ₙR³, dans laquelle Alk⁴ est une chaîne alcoylène linéaire ou ramifiée ayant de 1 à 3 atomes de carbone, v est zéro ou le nombre entier 1, L¹ est une liaison covalente ou un atome ou un groupe de liaison tel que défini pour L^{2a}, n est zéro ou le nombre entier 1, Alk¹ est une chaîne aliphatique éventuellement substituée et R³ est un atome d'hydrogène ou un -CN, -NO₂ ou un groupe hétéroaliphatique cycloaliphatique, hétérocycloaliphatique, polycycloaliphatique, hétéropolycycloaliphatique, aromatique ou hétéroaromatique, éventuellement substitué ;
p est zéro ou le nombre entier 1, 2, 3 ou 4 ;
pourvu que Cy ne soit pas un groupe cyclobutènedione; et le composé de formule (1) ne soit pas l'ester méthylique de dimédonyl-(S)-tyrosine-O-benzyle ;
et ses sels, produits de solvatation, produits d'hydratation et N-oxydes.

2. Composé suivant la revendication 1, dans lequel R est un groupe acide carboxylique (-CO₂H).

3. Composé suivant la revendication 1, dans lequel R est un groupe carboxyle estérifié de formule -CO₂Alk⁷.

4. Composé suivant l'une quelconque des revendications 1 à 3, dans lequel Ar² est un groupe phénylène éventuellement substitué.

5. Composé suivant l'une quelconque des revendications 1 à 4, dans lequel Ar¹ est un groupe phényle ou un groupe hétéroaromatique à cinq, six ou dix chaînons éventuellement substitué.

6. Composé suivant la revendication 5, dans lequel Ar¹ est un groupe pyridyle, pyrimidinyle, naphtyridinyle, quinolinyle ou isoquinolinyle éventuellement substitué.

7. Composé suivant la revendication 1, dans lequel Cy est un groupe cyclopentényle, cyclohexényle, cycloheptényle, dihydropyrimidinyle, dihydropyridinyle ou imidazolinyle éventuellement substitué.

8. Composé suivant l'une quelconque des revendications 1 à 6, dans lequel X est un atome de N.

9. Composé suivant la revendication 8, dans lequel Cy est un cycle 2-aminopyridine-4-one [amino se référant au groupe -N(R²)-], 4-aminopyrimidine-2-one, 2-aminopyridine-4-one, 6-aminopyridine-2-one ou 2-aminoimiadozoline-4-one éventuellement substitué.

10. Composé suivant l'une quelconque des revendications 1 à 6, dans lequel X est un groupe C(R^{w}).

11. Composé suivant la revendication 10, dans lequel Cy est un cycle 3-amino-2-cyclopentène-1-one [dans lequel amino se réfère au groupe -N (R²)-], ou 3-amino-2-cyclohexène-1-one éventuellement substitué.

12. Composé suivant la revendication 11, dans lequel p est zéro ou le nombre entier 1 ou 2.

13. Composé suivant l'une quelconque des revendications 1 à 12, dans lequel v dans le groupe R^{z} est zéro, Alk¹ est un chaîne aliphatique et R³ est un atome d'hydrogène.

14. Composé suivant la revendication 13, dans lequel -Alk¹R³ est un groupe alcoyle ayant de 1 à 6 atomes de carbone, allyle (-CH₂CH=CH₂) ou propargyle (-CH₂CCH) éventuellement substitué.

15. Composé suivant la revendication 14, dans lequel L¹ est une liaison covalente.

16. Composé qui est :
l'acide (S)-3-[4-[(2,6-naphtyridinyl)amino]-phényl]-2-[(2-(1-éthylpropyl)-3-oxo-1-cyclopentényl)amino]propionique ;
l'acide (S)-3-[4-[(3-méthyl-2,7-naphtyridinyl)-oxy]phényl]-2-[(2-propyl-3-oxo-1-cyclopentényl)amino]propionique ;
l'acide (S)-3-[(4-[3-méthyl-2,7-naphtyridinyl]-oxy)phényl]-2-[(2-isobutyl-3-oxo-1-cyclopentényl)amino]propionique ;
l'acide (S)-3-[(4-(2,7-naphtyridine-1-yl)oxy)-phényl]-2-[(2-isobutyl-3-oxo-1-cyclopentényl)amino]propionique ;
l'acide (S)-3-[4-[(3,5-dichloropyrid-4-yl)carboxamido)phényl]-2-[(2-isobutyl-3-oxo-1-cyclopentényl)amino]propionique ;
l'acide 3-(S)-3-[4-[(3-méthyl-2,7-naphtyridine-1-yl)amino]phényl]-2-[(2-isobutyl-3-oxo-1-cyclopentényl)-amino]propionique ;
l'acide (S)-3-[4-(-2',6'-diméthoxy)biphényl]-2-[(2-isobutyl-3-oxo-1-cyclopentényl)amino]propionique ;
le (2S)-3-[4-(2,6-naphtyridine-1-yl)amino)phényl]-2-[(2-isobutyl-3-oxo-1-cyclopentényl)-amino]propionate ;
l'acide (2S)-3-[4-(2,6-naphtyridine-1-yl)amino)phényl]-2-[(2-isopropyl-3-oxo-1-cyclopentényl)-amino]propionique ;
le (2S)-3-[4-(2,6-naphtyridine-1-yl)amino)phényl]-2-[(2-allyl-3-oxo-1-cyclopentényl)-amino]propionate ;
le (2S)-3-[4-(2,6-naphtyridine-1-yl)amino)phényl]-2-[(2-propyl-3-oxo-1-cyclopentényl)-amino]propionate ;
et leurs sels, produits de solvatation, produits d'hydratation et N-oxydes et ester d'acide carboxylique, en particulier leurs esters méthyliques, éthyliques, propyliques et iso-propyliques.

17. Composition pharmaceutique comprenant un composé suivant la revendication 1, ensemble avec un ou plusieurs véhicules, excipients ou diluants acceptables pharmaceutiquement.
